# EUROPEAN PATENT APPLICATION

(11) **EP 4 032 900 A1**
(43) Date of publication of application: **27.07.2022**
(21) Application number: 21152693.4
(22) Date of filing: 21.01.2021
(51) Int. Cl.: C07K 14/37, C07K 14/38, C12N 15/52, C12N 9/02, C12P 7/22

(54) **NEW ENZYMES AND METHODS FOR VANILLIC ACID-RELATED METABOLIC PATHWAYS**

(71) Applicant: Koninklijke Nederlandse Akademie van Wetenschappen, 3584 CT Utrecht (NL)
(72) Inventor: LUBBERS, Ronnie Johannes Maria, 3453 NZ Utrecht (NL); DE VRIES, Ronald Peter, 3723 XT Bilthoven (NL)
(74) Representative: Nederlandsch Octrooibureau

(57) **Abstract**

The present invention relates to enzymes from a cluster related to metabolism of 1-substituted 4-hydroxy-3-methoxybenzene, and to host cells comprising these enzymes, and to methods for using the enzymes. The enzymes are useful for the production of vanillin, vanillic acid, and methoxyhydroquinone.

## Description

### Field of the invention

The present invention relates to the field of industrial microbiology and biotechnology; specifically to the production of compounds of vanillic acid-related metabolic pathways such as a 1-substituted 4-hydroxy-3-methoxybenzene.

### Background of the invention

Vanillin is one of the world's most important food flavour compounds and is used in the food, beverage, cosmetic and pharmaceutical industries. Vanillin and derivatives can also be used in the production of polymers. Currently, less than 1% of the produced vanillin is derived from natural sources, while the majority of vanillin is obtained through chemical synthesis of phenol, eugenol and guaiacol. Vanillin obtained through chemical synthesis is considered "artificial" based on European and United states regulations and the use of chemical vanillin in food and pharmaceutical industries is restricted by multiple food safety control agencies worldwide. Therefore, there is still a need for alternatives such as biotechnological production of vanillin for use in these industries. Several vanillin metabolic pathways are known in microorganisms. Vanillin is toxic in low concentrations for many microorganisms and therefore the ability to degrade vanillin is essential for them. Bacterial systems using ferulic acid or eugenol as precursor have been described (Kaur and Chakraborty 2013). In bacteria, vanillin is converted to vanillic acid which is catalysed by vanillin dehydrogenase (Vdh), followed by the decarboxylation to guaiacol by vanillic acid decarboxylase (Chow et al. 1999). Alternatively, vanillic acid can also be demethylated to protocatechuic acid by vanillate-o-demethylase oxidoreductase (Rosini et al. 2016). Another conversion that has been observed is the hydroxylation of vanillic acid to methoxyhydroquinone by vanillate hydroxylase (Vhy), however this conversion appears to be uncommon for bacteria (El-Mansi and Anderson 2004).

The above vanillic acid conversions have been also observed in filamentous fungi, however the conversion towards methoxyhydroquinone appears to be more common (Guiraud et al. 1992). The fungal genes encoding these enzymes are unknown but in *Sporotichum pulverulentum* and *Phanerochaete chrysosporium,* a Vhy has been characterized (Yajima et al. 1979; Busswell and Eriksson 1988). In *Aspergillus niger,* coniferyl alcohol is converted to ferulic acid which is then converted to vanillic acid and further to methoxyhydroquinone (Guiraud et al. 1992; Baqueiro-Peña et al. 2010; Lubbers et al. 2020). Vanillin is considered to be an intermediate between the conversion of ferulic acid and vanillic acid, however it was not detected. In fungi, only one method to produce vanillin has been described (Lesage-Meessen et al. 1996). In this method, *Aspergillus niger* is used to convert ferulic acid to vanillic acid, which is further converted by *Pycnoporus cinnabarinus* to vanillin.

Industrial production of biochemical compounds using bacteria is commonly known to present unique challenges, such as low tolerance to industrially-relevant process conditions (for example low pH values), expensive nutrient requirements and susceptibility to phage infections. The economic viability of biotechnological production of compounds of vanillic acid-related metabolic pathways such as 1-substituted 4-hydroxy-3-methoxybenzenes can additionally benefit from improving obtained product yields and/or productivities of the cells and/or processes. Therefore, there is still a need forthe provision of improved cells forthe production of these compounds. There is still a need for the provision of improved methods of biological production of these compounds. Further, there is a need for production methods that reduce energy requirements, that reduce feedstock requirements, that allow a broader choice of feedstock, and that result in increased yield or purity of produced compounds.

### Summary of the invention

The inventors have surprisingly found new enzymes that have utility in metabolic pathways related to 4-hydroxy-3-methoxybenzenes. It was also found that disruption of one or more of these enzymes can lead to the useful production of intermediate compounds. Thus, the present invention relates to a cell capable of expressing functional enzymes from a cluster related to metabolism of 1-substituted 4-hydroxy-3-methoxybenzenes, and to methods for using the cell and/or enzymes. In a first aspect the invention provides a host cell capable of expressing at least one of a functional enzyme with vanillin dehydrogenase (Vdh) activity and a functional enzyme with vanillate hydroxylase (Vhy) activity, wherein the host cell has reduced or no expression of a functional enzyme with methoxyhydroquinone dioxygenase (Mhd) activity, preferably 1,2-dioxygenase activity. In some embodiments of this aspect the host cell is provided, wherein the host cell is a microbial cell, preferably a fungal cell, more preferably a filamentous fungal cell. In further embodiments of this aspect the host cell is provided, wherein the enzyme with Vdh activity is heterologous, preferably VdhA from *Aspergillus niger,* and/or the enzyme with Vhy activity is heterologous, preferably VhyA from *Aspergillus niger,* and/or the enzyme with Mhd activity is heterologous, preferably MhdA from *Aspergillus niger.* In some embodiments of this aspect the host cell is provided, wherein the enzyme with Vdh activity has at least 60%, preferably at least 80%, more preferably at least 90%, most preferably 100% sequence identity with SEQ ID NO: 1, or is encoded by a nucleotide sequence which has at least 60%, preferably at least 80%, more preferably at least 90%, most preferably 100% sequence identity with SEQ ID NO : 2. In some embodiments of this aspect the host cell is provided, wherein the enzyme with Vhy activity has at least 60%, preferably at least 80%, more preferably at least 90%, most preferably 100% sequence identity with SEQ ID NO: 3, or is encoded by a nucleotide sequence which has at least 60%, preferably at least 80%, more preferably at least 90%, most preferably 100% sequence identity with SEQ ID NO : 4. In some embodiments of this aspect the host cell is provided, wherein the enzyme with Mhd activity has at least 60%, preferably at least 80%, more preferably at least 90%, most preferably 100% sequence identity with SEQ ID NO: 5, or is encoded by a nucleotide sequence which has at least 60%, preferably at least 80%, more preferably at least 90%, most preferably 100% sequence identity with SEQ ID NO : 6. In further embodiments of this aspect the host cell is provided, wherein the nucleotide sequence encoding at least one of the enzymes with Vdh, Vhy or Mhd activity is codon optimized. In preferred embodiments of this aspect the host cell is provided, wherein the cell is capable of expressing a functional enzyme with vanillin dehydrogenase (Vdh) activity and has reduced or no expression of a functional enzyme with vanillate hydroxylase (Vhy) activity, and has reduced or no expression of a functional enzyme with methoxyhydroquinone 1,2-dioxygenase (Mhd) activity. In other preferred embodiments of this aspect the host cell is provided, wherein the cell is capable of expressing a functional enzyme with vanillin dehydrogenase (Vdh) activity and is capable of expressing a functional enzyme with vanillate hydroxylase (Vhy) activity and has reduced or no expression of a functional enzyme with methoxyhydroquinone 1,2-dioxygenase (Mhd) activity. In other preferred embodiments of this aspect the host cell is provided, wherein the cell is capable of producing at least 1.0 mM of a 1-substituted 4-Hydroxy-3-methoxybenzene, preferably selected from vanillin, vanillic acid, and methoxyhydroquinone, more preferably selected from vanillic acid and methoxyhydroquinone.

In a second aspect the invention provides a method for the production of a 1-substituted 4-hydroxy-3-methoxybenzene, comprising the steps of culturing a cell according to the first aspect under conditions conducive to the production of a 1-substituted 4-hydroxy-3-methoxybenzene, and, optionally, isolating and/or purifying the 1-substituted 4-hydroxy-3-methoxybenzene from the cell and/or the culture medium. In some embodiments of this aspect the method is provided, wherein the method is for the production of vanillic acid, and the cell is a cell capable of expressing a functional enzyme with vanillin dehydrogenase (Vdh) activity and has reduced or no expression of a functional enzyme with vanillate hydroxylase (Vhy) activity and has reduced or no expression of a functional enzyme with methoxyhydroquinone 1,2-dioxygenase (Mhd) activity. In some embodiments of this aspect the method is provided, wherein the method is for the production of methoxyhydroquinone, and wherein the cell is a cell capable of expressing a functional enzyme with vanillin dehydrogenase (Vdh) activity and is capable of expressing a functional enzyme with vanillate hydroxylase (Vhy) activity and has reduced or no expression of a functional enzyme with methoxyhydroquinone 1,2-dioxygenase (MhdA) activity.

In a third aspect, the invention provides an expression vector comprising a nucleic acid construct comprising a nucleotide sequence encoding a functional enzyme with vanillin dehydrogenase (Vdh) activity, and/or a functional enzyme with vanillate hydroxylase (Vhy) activity, and/or a functional enzyme with methoxyhydroquinone 1,2-dioxygenase (Mhd) activity, and/or a functional enzyme with 4-oxo-monomethyl esterase (Ome) activity. In a preferred embodiment, the invention provides an expression vector comprising a nucleic acid construct comprising a nucleotide sequence that has at least 60%, 65%, 70%, 75%, 80%, 85%, 90%, 95%, 96%, 97%, 98%, 99%, or 100% sequence identity with SEQ ID NO: 6 or 12, or wherein the expression vector consists of a polynucleotide that has at least 60%, 65%, 70%, 75%, 80%, 85%, 90%, 95%, 96%, 97%, 98%, 99%, or 100% sequence identity with SEQ ID NO: 9.

In a fourth aspect, the invention provides a polypeptide product expressed from the expression vector according to the third aspect.

In a fifth aspect, the invention provides the use of a functional enzyme with methoxyhydroquinone 1,2-dioxygenase (Mhd) activity as specified in the first aspect for the production of 4-hydroxy-6-methoxy-6-oxohexa-2,4-dienoic acid. Also provided is the use of a functional enzyme with 4-oxo-monomethyl esterase (Ome) activity for the production of 3-oxo-adipic acid.

### Brief description of the figures

Figure 1 - Schematic representation of reactions catalysed by Vdh, Vhy, Mhd, and Ome and involved reactants and products.
Figure 2A - Enzymatic activity of purified VdhA, VhyA, and MhdA on their corresponding substrates.
All enzymatic reactions were incubated for 1h at 30 °C. Analysis of substrate conversion was performed by HPLC.
Figure 2B - Chromatograph of MhdA incubated with methoxyhydroquinone.
Figure 2C - Chromatograph (total ion count) of the ethyl acetate extracted reaction mixture of methoxyhydroquinone incubated with MhdA.
Figure 2D - Mass spectra of the peak at retention time 3.78 min at negative and positive ionization mode. The product was identified as 4-hydroxy-6-methoxy-6-oxohexa-2,4-dienoic acid (tautomer shown in figure 1).
Figure 3 - Growth profile of A. *niger* deletion mutants *ΔvdhA, ΔvhyA, ΔmhdA, ΔOmeA,* and the reference strain. Growth was examined on selected aromatic compounds as sole carbon source. Agar plates were incubated at 30 °C for 10 days.

### Description of the invention

The inventors have surprisingly found new enzymes that have utility in metabolic pathways related to 4-hydroxy-3-methoxybenzenes. It was also found that disruption of one or more of these enzymes can lead to the useful production of intermediate compounds.

Accordingly, in a first aspect, the invention provides a host cell capable of expressing at least one of a functional enzyme with vanillin dehydrogenase (Vdh) activity and a functional enzyme with vanillate hydroxylase (Vhy) activity, wherein the host cell has reduced or no expression of a functional enzyme with methoxyhydroquinone 1,2-dioxygenase (Mhd) activity.

In some embodiments, this aspect provides a host cell which has reduced or no expression of at least one of a functional enzyme with vanillin dehydrogenase (Vdh) activity and a functional enzyme with vanillate hydroxylase (Vhy) activity, wherein the host cell has reduced or no expression of a functional enzyme with methoxyhydroquinone 1,2-dioxygenase (Mhd) activity.

In some embodiments, this aspect provides a host cell which has reduced or no expression of a functional enzyme with vanillin dehydrogenase (Vdh) activity and a functional enzyme with vanillate hydroxylase (Vhy) activity, wherein the host cell has reduced or no expression of a functional enzyme with methoxyhydroquinone 1,2-dioxygenase (Mhd) activity.

In some embodiments, this aspect provides a host cell which has reduced or no expression of a functional enzyme with vanillin dehydrogenase (Vdh) activity, wherein the host cell is capable of expressing a functional enzyme with vanillate hydroxylase (Vhy) activity, wherein the host cell is capable of expressing a functional enzyme with methoxyhydroquinone 1,2-dioxygenase (Mhd) activity.

In some embodiments, this aspect provides a host cell which has reduced or no expression of a functional enzyme with vanillin dehydrogenase (Vdh) activity, wherein the host cell is capable of expressing a functional enzyme with vanillate hydroxylase (Vhy) activity, wherein the host cell has reduced or no expression of a functional enzyme with methoxyhydroquinone 1,2-dioxygenase (Mhd) activity.

In some embodiments, this aspect provides a host cell which has reduced or no expression of a functional enzyme with vanillin dehydrogenase (Vdh) activity and a functional enzyme with vanillate hydroxylase (Vhy) activity, wherein the host cell is capable of expressing a functional enzyme with methoxyhydroquinone 1,2-dioxygenase (Mhd) activity.

In some embodiments, this aspect provides a host cell capable of expressing a functional enzyme with vanillin dehydrogenase (Vdh) activity which has reduced or no expression of a functional enzyme with vanillate hydroxylase (Vhy) activity, wherein the host cell is capable of expressing a functional enzyme with methoxyhydroquinone 1,2-dioxygenase (Mhd) activity.

In preferred embodiments, this aspect provides a host cell capable of expressing a functional enzyme with vanillin dehydrogenase (Vdh) activity which has reduced or no expression of a functional enzyme with vanillate hydroxylase (Vhy) activity, and has reduced or no expression of a functional enzyme with methoxyhydroquinone 1,2-dioxygenase (Mhd) activity.

In other preferred embodiments, this aspect provides a host cell capable of expressing a functional enzyme with vanillin dehydrogenase (Vdh) activity and a functional enzyme with vanillate hydroxylase (Vhy) activity, and which has reduced or no expression of a functional enzyme with methoxyhydroquinone 1,2-dioxygenase (Mhd) activity.

Throughout this disclosure, the terms "host cell" and "cell" are used interchangeably and can be interpreted to encompass any cell capable of expressing at least one of the functional enzymes according to the invention. The terms may also be interpreted to encompass any cell that has reduced or no expression of at least one of the functional enzymes according to the invention as compared to a corresponding cell that has not been modified in that way. Host cells that can be used according to the invention are well-known in the art.

Suitable host cells that can be used according to the invention are well-known in the art. Suitable host cells can be mammalian, insect, plant, or microbial cells. Examples of mammalian cells include Chinese hamster ovary (CHO) cells, COS cells, 293 cells, PerC6^{™} cells and hybridomas. Examples of insect cells include Sf9 and Sf21 cells and derivatives thereof. In some embodiments, the host cell is a microbial cell. The microbial host cell can be prokaryotic or eukaryotic. A microbial prokaryote is a unicellular organism that lacks a membrane-bound nucleus, mitochondria, and other membrane-bound organelles. Examples of microbial prokaryotes include bacteria and archaea, of which bacteria are preferred. Bacterial host cells include both Gram-negative and Gram-positive organisms and can be selected from suitable groups known in the art such as the group of *Absidia, Achromobacter, Acinetobacter, Aeribacillus, Aneurinibacillus, Agrobacterium, Aeromonas, Alcaligenes, Arthrobacter, Arzoarcus, Azomonas, Azospirillum, Azotobacter, Bacillus, Beijerinckia, Bradyrhizobium, Brevibacills, Burkholderia, Byssochlamys, Citrobacter, Clostridium, Comamonas, Cupriavidus, Corynebacterium, Deinococcus, Escherichia, Enterobacter, Flavobacterium, Fusobacterium, Gossypium, Klebsiella, Lactobacillus, Listeria, Megasphaera, Micrococcus, Mycobacterium, Norcadia, Porphyromonas, Propionibacterium, Pseudomonas, Ralstonia, Rhizobium, Rhodopseudomonas, Rhodospirillum, Rodococcus, Roseburia, Shewanella, Streptomycetes, Xanthomonas, Xylella, Yersinia, Treponema, Vibrio, Streptococcus, Lactococcus, Zymomonas, Staphylococcus, Salmonella, Sphingomonas, Sphingobium, Novosphingobium, Brucella* and *Microscilla.* Preferably, the bacterial cell is selected from a species from the group consisting of *Aeribacillus pallidus, Aneurinibacillus terranovensis, Bacillus subtilis, Bacillus amyloliquefaciens, Bacillus coagulans, B. Irribbensis, Bacillus licheniformis, B. puntis, Bacillus megaterium, Bacillus halodurans, Bacillus pumilus, Brevibacillus thermoruber, Brevibacillus panacihumi, Cupriavidus basilensis, D. Iruznetsovii, D. thermophila, G. Iraustophilus, Gluconobacter oxydans, Caulobacter crescentus CB 15, Methylobacterium extorquens, Rhodobacter sphaeroides, Pelotomaculum thermopropionicum, Pseudomonas zeaxanthinifaciens, Pseudomonas putida, Paracoccus denitrificans, Escherichia coli, Corynebacterium glutamicum, Staphylococcus carnosus, Streptomyces lividans, Sinorhizobium melioti, Sphingobium sp., Novosphingobium sp., Sphingomonas henshuiensis,* and *Rhizobium radiobacter.* Within the species *Pseudomonas putida,* strains S12 and KT2440 are preferred. Within the species *Sphingobium sp.,* strains SYK-6 and 66-54 are preferred. Within the species *Sphingomonas hengshuiensis,* strain WHSC-8 is preferred. Within the species *Novosphingobium sp.,* strain AAP93 is preferred. A microbial eukaryote is a unicellular or multicellular organism which has a nucleus enclosed within a nuclear envelope and contains membrane-bound organelles such as mitochondria and a Golgi apparatus. Examples of suitable microbial eukaryotes are protozoa, algae and fungi. In preferred embodiments, the host cell is a fungal cell. Particularly when compared to bacteria, fungi, have many attractive features for industrial fermentation processes, including e.g. their high tolerance to acids, ethanol and other harmful compounds, their high osmo-tolerance, their high fermentative capacity and for some yeasts their capability of anaerobic growth. Fungal host cells can be selected from suitable groups known in the art such as yeasts of the group *Saccharomyces, Kluyveromyces, Candida, Pichia, Schizosaccharomyces, Hansenula, Kloeckera, Schwanniomyces, Yarrowia, Cryptococcus, Debaromyces, Saccharomycecopsis, Saccharomycodes, Wickerhamia, Debayomyces, Hanseniaspora, Ogataea, Kuraishia, Komagataella, Metschnikowia, Williopsis, Nakazawaea, Torulaspora, Bullera, Rhodotorula, Sporobolomyces* and filamentous fungi. Within yeasts, the species *Kluyveromyces lactis, Saccharomyces cerevisiae, Hansenula polymorpha* (also known as *Ogataea henricii*), *Yarrowia lipolytica, Candida tropicalis* and *Pichia pastoris* (also known as *Komagataella pastoris*) are preferred. In more preferred embodiments, the host cell is a filamentous fungal cell. A filamentous fungal cell according to the invention includes all members of the subdivisions Eumycota and Oomycota (preferably as defined by Hawksworth et al., In, Ainsworth and Bisby's Dictionary of The Fungi, 8th edition, CAB International, University Press, Cambridge, UK, 1995). Filamentous fungi are characterized by a mycelial wall composed of chitin, cellulose, glucan, chitosan, mannan, and other complex polysaccharides. Vegetative growth is by hyphal elongation and carbon catabolism is obligatorily aerobic. Filamentous fungal host cells can be selected from suitable groups known in the art such as *Acremonium, Agaricus, Aspergillus, Aureobasidium, Chrysosporium, Coprinus, Cryptococcus, Filibasidium, Fusarium, Humicola, Magnaporthe, Mucor, Myceliophthora, Neocallinastix, Neurospora, Paecilomyces, Penicillium, Piromyces, Panerochaete, Pleurotus, Schizophyllum, Talaromyces, Thermoascus, Thielavia, Tolypocladium, Ustilago and Trichoderma.* Within filamentous fungi, the species *Aspergillus niger, Aspergillus nidulans, Aspergillus fumigatus, Aspergillus oryzae,, Aspergillus vadensis, Penicillium chrysogenum, Penicillium citrinum, Penicillium rubens, Penicillium oxalicum, Penicillium subrubescens, Rasamsonia emersonii, Talaromyces emersonii, Acremonium chrysogenum, Trichoderma reesei, Aspergillus sojae,* and *Chrysosporium lucknowense* are preferred. Several strains belonging to the these groups are readily accessible to the public in a number of well-known collections, such as the American Type Culture Collection (ATCC), Deutsche Sammlung von Mikroorganismen and Zellkulturen GmbH (DSM), Centraalbureau Voor Schimmelcultures (CBS), and Agricultural Research Service Patent Culture Collection, Northern Regional Research Center (NRRL). Examples of such strains include *Aspergillus niger* CBS 513.88, N593, CBS 120.49, N402, ATCC 1015 *Aspergillus oryzae* ATCC 20423, IFO 4177, ATCC 1011, ATCC 9576, ATCC 14488-14491, ATCC 11601, ATCC12892, *Aspergillus vadensis* CBS 113365, CBS 102787, IMI 142717, IBT 24658, CBS 113226, *Penicillium chrysogenum* CBS 455.95, *Penicillium citrinum* ATCC 38065, *Penicillium chrysogenum* P2, Wisconsin 54-1255, *Penicillium subrubescens* CBS 132785, FBCC 1632, *Talaromyces emersonii* CBS 393.64, *Acremonium chrysogenum* ATCC 36225 or ATCC 48272, *Trichoderma reesei* ATCC 26921 or ATCC56765 or ATCC 26921, *Aspergillus sojae* ATCC11906, *Chrysosporium lucknowense* ATCC44006 and derivatives thereof. In even more preferred embodiments, the host cell is a cell selected from the group of *Aspergillus.* In most preferred embodiments, the host cell is an *Aspergillus niger* cell, preferably *Aspergillus niger* strain 120.49 or a derivative thereof.

As used herein, a derivative of a host cell has its conventional meaning used in the art, and refers to an engineered version of that host cell, or to a host cell that has otherwise been mutated as compared to the named host cell.

In an embodiment the invention provides:
A host cell capable of producing at least one of:
   i) a functional enzyme with vanillin dehydrogenase (Vdh) activity; and
   ii) a functional enzyme with vanillate hydroxylase (Vhy) activity;
wherein the host cell has reduced or no expression of a functional enzyme with methoxyhydroquinone 1,2-dioxygenase (Mhd) activity and wherein said host cell is a microbial cell, preferably a fungal cell, more preferably a filamentous fungal cell, even more preferably an *Aspergillus* cell, most preferably an *Aspergillus niger* cell.

Throughout this disclosure, the term "expression" by a host cell can be interpreted to encompass any step starting from transcription of a gene into an mRNA transcript, leading to a polypeptide such as an enzyme. An enzyme is a polypeptide that can catalyse a reaction. An enzyme that shows activity in catalysing its associated reaction is a functional enzyme. A functional enzyme can be present within the cell or secreted into the growth medium. Accordingly, in the context of the invention, expression of a functional enzyme by the host cell can be considered to include any step involved in the production of said enzyme including, but not limited to, transcription, post-transcriptional modification, translation, post-translational modification, and secretion. Any of these steps may naturally occur in the host cell or may be introduced or modified via means of genetic engineering or modification as discussed below.

Expression of a functional enzyme by a host cell also includes embodiments wherein any of the abovementioned steps involved in the production of said enzyme are increased as compared to a corresponding cell that has not been genetically modified, resulting in increased levels of enzyme transcript, enzyme polypeptide, and/or enzyme activity compared to said non-modified cell. In embodiments wherein the levels of enzyme transcript, enzyme polypeptide, and/or enzyme activity in the host cell are increased compared to a corresponding non-modified cell, said increase is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, more preferably at least 30%, even more preferably at least 40%, most preferably at least 50%.

Reduced expression of a functional enzyme by a host cell refers to embodiments wherein any of the abovementioned steps involved in the production of said enzyme are reduced as compared to a corresponding cell that has not been genetically modified in this way, resulting in decreased levels of enzyme transcript, enzyme polypeptide, and/or enzyme activity compared to said non-modified cell. Preferably, the levels of enzyme transcript, enzyme polypeptide, and/or enzyme activity in the host cell are decreased by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, more preferably at least 30%, even more preferably at least 40%, most preferably at least 50%, as compared to a corresponding non-modified cell.

The differences in levels of enzyme transcript, enzyme polypeptide and/or enzyme activity between cells can be measured by the skilled person using standard techniques in the art, as described in handbooks such as Sambrook and Russel, Molecular Cloning: A Laboratory Manual, 3rd edition, Cold Spring Harbor Laboratory, Cold Spring Harbor Laboratory Press, NY, 20011, Ausubel F. et al, eds., Current protocols in molecular biology, Green Publishing and Wiley Interscience, NY, 2003, and Bisswanger H, Practical Enzymology, 2nd edition, Wiley-VCH Verlag GmbH & Co. KgaA, 2012. Examples of methods of assessing differences in enzyme transcript levels include quantitative PCR and RNA sequencing. Examples of methods of assessing differences in enzyme polypeptide levels include SDS-PAGE and quantitative proteomics. Examples of methods of assessing differences in enzyme activity levels include enzymatic assays performed on whole cells and/or cell lysates.

No or lack of expression of a functional enzyme refers to embodiments wherein the activity of the functional enzyme is not detectable, for instance because it has been eliminated, which can be the result of a disruption of any of the abovementioned steps involved in the production of said enzyme by the host cell. This means that embodiments wherein the enzyme transcript and/or enzyme polypeptide are still present should not be excluded, as long as the activity of the functional enzyme is no longer detectable, for instance because it is eliminated. As an example, an enzyme might require a post-translational modification in order to be converted to a functional enzyme and the host cell might lack the capability of performing this post-translational modification; said cell will have no expression of a functional enzyme even though the enzyme polypeptide is present.

In the context of this invention, a cell capable of expressing a functional enzyme is a cell that comprises the genetic information required for expressing said enzyme, preferably a nucleic acid encoding said enzyme. This does not mean that the enzyme is necessarily expressed. As an example, a cell may comprise a nucleic acid encoding an enzyme wherein the nucleic acid is under the control of a promoter that responds to a particular induction; the promoter is not necessarily induced and thus the enzyme is not necessarily expressed, whereas the cell is in fact capable of such expression. In preferred embodiments, a cell capable of expressing a functional enzyme is a cell that expresses said functional enzyme. Optionally, it is a cell that a skilled person can routinely induce to commence such expression. A skilled person understands that in this context the expression of the enzyme indicates production of the enzyme, such as via expression of the coding nucleotide sequence. Expression is further defined elsewhere herein.

In the context of the invention, a host cell that has reduced or no expression of a functional enzyme may or may not comprise the genetic information required for expressing said enzyme. The skilled person is aware of methods that result in decreased expression of genes without altering the gene sequence per se. As an example, RNA interference can be used (for a review see Wilson and Doudna, 2013: Ann Rev Biophys 42:217-239). In another example, the host cell may have reduced or no expression of a transcription factor that is necessary for the expression of the gene encoding for the relevant enzyme as compared to a corresponding cell that has not been modified in this way. In another example, the gene encoding for the enzyme polypeptide is deleted or inactivated.

A functional enzyme with vanillin dehydrogenase activity is also known as vanillin dehydrogenase, Vdh, or aromatic aldehyde dehydrogenase. It is an enzyme (EC 1.2.1.67) which is able to catalyze the conversion of vanillin to vanillic acid through NAD-dependent oxidation. Vanillin is also known as 4-hydroxy-3-methoxybenzaldehyde, vanillic aldehyde, or methyl vanillin. Vanillic acid is also known as 4-hydroxy-3-methoxybenzoic acid, vanillate, or 4-hydroxy-m-anisic acid. In preferred embodiments, the enzyme with Vdh activity is VdhA from *Aspergillus niger* (SEQ ID NO: 1).

A functional enzyme with vanillate hydroxylase activity, is also known as vanillin hydroxylase or Vhy. It is an enzyme that is able to catalyze the conversion of vanillic acid to methoxyhydroquinone utilizing O₂, FAD and NAD(P)H. This reaction has been characterized in the art, see e.g. Buswell and Eriksson, 1988: Methods Enzymol 161:274-281. Methoxyhydroquinone is also known as 2-methoxyhydroquinone, 2-methoxybenzene-1,4-diol, o-methoxyhydroquinone, or 1,4-dihydroxy-2-methoxybenzene. In preferred embodiments, the enzyme with Vhy activity is VhyA from *Aspergillus niger* (SEQ ID NO: 3).

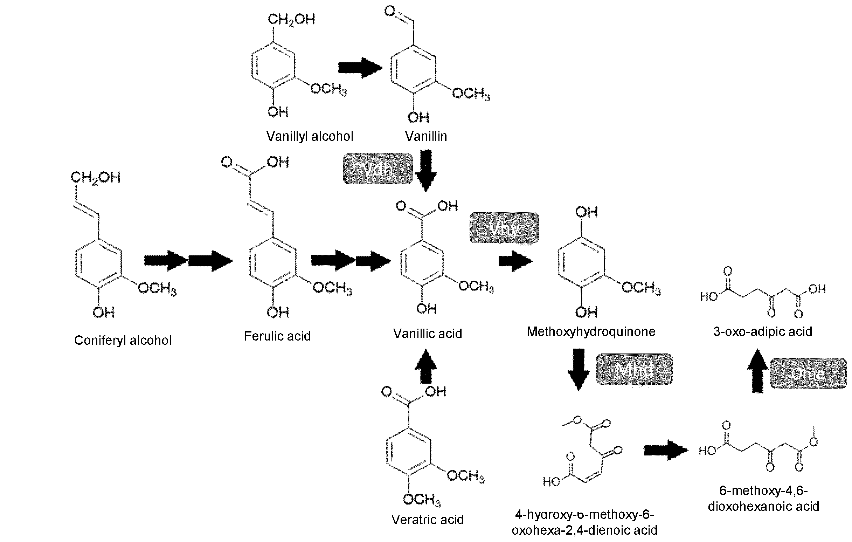

A functional enzyme with methoxyhydroquinone 1,2-dioxygenase (Mhd) activity is also known as methoxyhydroquinone1,2-dioxygenase or Mhd. It is an enzyme able to catalyze the conversion of methoxyhydroquinone to 4-hydroxy-6-methoxy-6-oxohexa-2,4-dienoic acid by cleaving the ring of methoxyhydroquinone. 4-hydroxy-6-methoxy-6-oxohexa-2,4-dienoic acid readily tautomerises to 4-oxo-6-methoxy-6-oxohex-2-enoic acid. In preferred embodiments, the enzyme with Mhd activity is MhdA from *Aspergillus niger* (SEQ ID NO: 5). An exemplary schematic representation of the above enzymatic reactions and involved compounds is given in Figure 1.

A functional enzyme with 4-oxo-monomethyl esterase (Ome) activity is also known as 6-methoxy-4,6-dioxohexanoic acid esterase (Mdh). It is an enzyme able to catalyze the conversion of 4-oxoadipate methyl ester (also known as 6-methoxy-4,6-dioxohexanoic acid) to 4-oxoadipate (also known as 3-oxo-adipic acid) by hydrolysing the methyl ester of 4-oxoadipate methyl ester. In preferred embodiments, the enzyme with Ome activity is OmeA from *Aspergillus niger* (SEQ ID NO: 41).

Enzyme activity can be determined by monitoring for the decrease of reactant concentration or the increase of product concentration using chromatographic techniques known to the skilled person, such as gas chromatography (GC), high performance liquid chromatography (HPLC), or GC or liquid chromatography with advantageously coupled mass spectrometric methods, such as LC-MS or GC-MS. A preferred method for the detection and/or quantification of 1-substituted 4-hydroxy-3-methoxybenzenes is HPLC. As an example, chromatographic separation may be carried out in Dionex ICS-5000+ chromatography system, equipped with an Acclaim MixedMode WAX-1 LC Column (3×150 mm) and a UV detector (310 nm), said system being supplied by Thermo Scientific (USA), with the chromatographic separation being carried out according to manufacturer's recommendation using isocratic elution (30°C; flow rate: 0.25 mL/min) with 25 mM potassium monophosphate, 0.8 mM pyrophosphate, pH 6.0, in 50% acetonitrile, as described in Dilokpimol et al. 2017.

A preferred method for the detection of 4-hydroxy-6-methoxy-6-oxohexa-2,4-dienoic acid is mass spectrometry, preferably LC-MS, more preferably LC-SQMS and Orbitrap. Enzyme activity may also be determined by monitoring of oxidation and/or reduction of enzyme cofactors, such as NAD(H), NADP(H), FAD(H) using spectrophotometric methods known to the skilled person. As an example, conversion of NAD(P)H to NAD(P)+ can be monitored spectroscopically at about 340 nm. Enzyme activity may be determined in vitro or in vivo by measuring the respective catalysed reaction rates using culture broths, whole cells, cell-free growth media supernatants, cell lysates or isolated and/or purified enzymes, preferably culture broths, more preferably cell lysates and most preferably purified enzymes. Methods for the preparation and execution of said measurements are known in the art and are discussed, for example, in Bisswanger H, Practical Enzymology, 2nd edition, Wiley-VCH Verlag GmbH & Co. KgaA, 2012.

As used herein, the term "isolated" means that the material is separated from its original environment. An isolated enzyme polypeptide is a polypeptide that is substantially free of cellular material (when produced by a cell) or chemical precursors or other chemicals (when synthetically made). The enzyme polypeptide may be isolated directly from cultured cells or from the culture medium if it is excreted. A signal sequence may be used to facilitate secretion and isolation, by linking (fusing) it to the enzyme polypeptide of interest using well-known molecular toolbox techniques. Signal sequences are typically characterized by a core of hydrophobic amino acids, which may be cleaved from the mature polypeptide during secretion in one or more cleavage events. Such signal peptides can contain processing sites that allow cleavage of the signal sequence from the mature polypeptides as they pass through the secretory pathway. The signal sequence directs secretion of the polypeptide, such as from a host cell expressing said polypeptide. The polypeptide can then be readily isolated and/or purified from the culture medium. Methods for isolation and/or purification of enzymes are known to the skilled person and are discussed in standard handbooks, such as Sambrook and Russel, Molecular Cloning: A Laboratory Manual, 3rd edition, Cold Spring Harbor Laboratory, Cold Spring Harbor Laboratory Press, NY, 2001 or Ausubel F. et al, eds., Current protocols in molecular biology, Green Publishing and Wiley Interscience, NY, 2003. Examples of isolation and/or purification methods include, chromatographic methods such as gel-filtration chromatography, ion-exchange chromatography, immunoaffinity chromatography, metal affinity chromatography, gel-filtration chromatography, fractionation with precipitants such as ammonium sulfate and polyethylene glycol, gel electrophoresis and salting out and dialysis. Preferably, metal affinity chromatography is used. Isolation and/or purification may optionally be enhanced by linking the enzyme polypeptide to a sequence that facilitates purification, such as with a GST domain, using well-known molecular toolbox techniques. In some embodiments, the enzyme polypeptide is linked (fused) to a hexa-histidine peptide, such as the tag provided in a pET23b vector (Genescript Biotech, Leiden, The Netherlands), among others, many of which are commercially available. As, for example, described in Gentz et al., Proc. Natl. Acad. Sci. USA 86:821-824 (1989), a hexa-histidine peptide provides for convenient purificaton of the fusion protein. In some embodiments, the functional enzyme with Vdh activity, and/or the functional enzyme with Vhy activity, and/or the functional enzyme with Mhd activity is a homologous enzyme. A homologous enzyme is a polypeptide that is native, or encoded by a nucleotide sequence that is native, i.e. naturally present in the same organism, as the host cell.

In some embodiments, the enzyme with Vdh activity, and/or the enzyme with Vhy activity, and/or the enzyme with Mhd activity , and/or the enzyme with Ome activity is a heterologous enzyme. A heterologous enzyme is a polypeptide that is derived from, or encoded by a nucleotide sequence that is derived from a different organism to the host cell. A heterologous enzyme may also be an enzyme encoded by a recombinant nucleotide sequence as discussed below. Molecular toolbox techniques to achieve and/or enhance and/or decrease homologous or heterologous expression of a functional enzyme by a host cell are well-known in the art and are further discussed below.

.A heterologous nucleic acid sequence can be a synthetic nucleic acid sequence. In preferred embodiments, the functional enzyme with Vdh activity is heterologous, more preferably from a filamentous fungus, even more preferably from *Aspergillus,* most preferably from *Aspergillus niger.* In other preferred embodiments, the functional enzyme with Vhy activity is heterologous, more preferably from a filamentous fungus, even more preferably from *Aspergillus,* most preferably from *Aspergillus niger.* In yet other preferred embodiments, the functional enzyme with Mhd activity is heterologous, more preferably from a filamentous fungus, even more preferably from *Aspergillus,* most preferably from *Aspergillus niger.*

In a preferred embodiment the invention provides:
A host cell capable of expressing at least one of:
   i) a functional enzyme with vanillin dehydrogenase (Vdh) activity; and
   ii) a functional enzyme with vanillate hydroxylase (Vhy) activity;
wherein the host cell has reduced or no expression of a functional enzyme with methoxyhydroquinone 1,2-dioxygenase (Mhd) activity and wherein at least one said enzymes is heterologous, preferably from a filamentous fungus, more preferably from *Aspergillus,* even more preferably from *Aspergillus niger.*

In some embodiments, the functional enzyme with Vdh activity has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO:1, or is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100%, sequence identity with SEQ ID NO: 2. In preferred embodiments, the functional enzyme with Vdh activity has at least 90% sequence identity with SEQ ID NO:1 or is encoded by a nucleotide sequence which has at least 90% sequence identity with SEQ ID NO: 2. In still preferred embodiments, the functional enzyme with Vdh activity has at least 95% sequence identity with SEQ ID NO:1 or is encoded by a nucleotide sequence which has at least 95% sequence identity with SEQ ID NO: 2. In more preferred embodiments, the functional enzyme with Vdh activity has at least 96% sequence identity with SEQ ID NO:1 or is encoded by a nucleotide sequence which has at least 96% sequence identity with SEQ ID NO: 2. In still more preferred embodiments, the functional enzyme with Vdh activity has at least 97% sequence identity with SEQ ID NO:1 or is encoded by a nucleotide sequence which has at least 97% sequence identity with SEQ ID NO: 2. In still more preferred embodiments, the functional enzyme with Vdh activity has at least 98% sequence identity with SEQ ID NO:1 or is encoded by a nucleotide sequence which has at least 98% sequence identity with SEQ ID NO: 2. In even more preferred embodiments, the functional enzyme with Vdh activity has at least 99% sequence identity with SEQ ID NO:1 or is encoded by a nucleotide sequence which has at least 99% sequence identity with SEQ ID NO: 2. In most preferred embodiments, the functional enzyme with Vdh activity has 100% sequence identity with SEQ ID NO:1 or is encoded by a nucleotide sequence which has 100% sequence identity with SEQ ID NO: 2.

In some embodiments, the functional enzyme with Vhy activity has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO:3, or is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO: 4. In preferred embodiments, the functional enzyme with Vhy activity has at least 90% sequence identity with SEQ ID NO:3 or is encoded by a nucleotide sequence which has at least 90% sequence identity with SEQ ID NO: 4. In still preferred embodiments, the functional enzyme with Vhy activity has at least 95% sequence identity with SEQ ID NO:3 or is encoded by a nucleotide sequence which has at least 95% sequence identity with SEQ ID NO: 4. In more preferred embodiments, the functional enzyme with Vhy activity has at least 96% sequence identity with SEQ ID NO:3 or is encoded by a nucleotide sequence which has at least 96% sequence identity with SEQ ID NO: 4. In still more preferred embodiments, the functional enzyme with Vhy activity has at least 97% sequence identity with SEQ ID NO:3 or is encoded by a nucleotide sequence which has at least 97% sequence identity with SEQ ID NO: 4. In still more preferred embodiments, the functional enzyme with Vhy activity has at least 98% sequence identity with SEQ ID NO:3 or is encoded by a nucleotide sequence which has at least 98% sequence identity with SEQ ID NO: 4. In even more preferred embodiments, the functional enzyme with Vhy activity has at least 99% sequence identity with SEQ ID NO:3 or is encoded by a nucleotide sequence which has at least 99% sequence identity with SEQ ID NO: 4. In most preferred embodiments, the functional enzyme with Vhy activity has 100% sequence identity with SEQ ID NO:3 or is encoded by a nucleotide sequence which has 100% sequence identity with SEQ ID NO: 4.

In some embodiments, the functional enzyme with Mhd activity has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO:5, or is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO: 6. In preferred embodiments, the functional enzyme with Mhd activity has at least 90% sequence identity with SEQ ID NO:5 or is encoded by a nucleotide sequence which has at least 90% sequence identity with SEQ ID NO: 6. In still preferred embodiments, the functional enzyme with Mhd activity has at least 95% sequence identity with SEQ ID NO:5 or is encoded by a nucleotide sequence which has at least 95% sequence identity with SEQ ID NO: 6. In more preferred embodiments, the functional enzyme with Mhd activity has at least 96% sequence identity with SEQ ID NO:5 or is encoded by a nucleotide sequence which has at least 96% sequence identity with SEQ ID NO: 6. In still more preferred embodiments, the functional enzyme with Mhd activity has at least 97% sequence identity with SEQ ID NO:5 or is encoded by a nucleotide sequence which has at least 97% sequence identity with SEQ ID NO: 6. In still more preferred embodiments, the functional enzyme with Mhd activity has at least 98% sequence identity with SEQ ID NO:5 or is encoded by a nucleotide sequence which has at least 98% sequence identity with SEQ ID NO: 6. In even more preferred embodiments, the functional enzyme with Mhd activity has at least 99% sequence identity with SEQ ID NO:5 or is encoded by a nucleotide sequence which has at least 99% sequence identity with SEQ ID NO: 6. In most preferred embodiments, the functional enzyme with Mhd activity has 100% sequence identity with SEQ ID NO:5 or is encoded by a nucleotide sequence which has 100% sequence identity with SEQ ID NO: 6.

In some embodiments, the functional enzyme with Ome activity has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO: 41, or is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO: 42. In preferred embodiments, the functional enzyme with Ome activity has at least 90% sequence identity with SEQ ID NO: 41 or is encoded by a nucleotide sequence which has at least 90% sequence identity with SEQ ID NO: 42. In still preferred embodiments, the functional enzyme with Ome activity has at least 95% sequence identity with SEQ ID NO: 41 or is encoded by a nucleotide sequence which has at least 95% sequence identity with SEQ ID NO: 42. In more preferred embodiments, the functional enzyme with Ome activity has at least 96% sequence identity with SEQ ID NO: 41 or is encoded by a nucleotide sequence which has at least 96% sequence identity with SEQ ID NO: 42. In still more preferred embodiments, the functional enzyme with Ome activity has at least 97% sequence identity with SEQ ID NO: 41 or is encoded by a nucleotide sequence which has at least 97% sequence identity with SEQ ID NO: 42. In still more preferred embodiments, the functional enzyme with Ome activity has at least 98% sequence identity with SEQ ID NO: 41 or is encoded by a nucleotide sequence which has at least 98% sequence identity with SEQ ID NO: 42. In even more preferred embodiments, the functional enzyme with Ome activity has at least 99% sequence identity with SEQ ID NO: 41 or is encoded by a nucleotide sequence which has at least 99% sequence identity with SEQ ID NO: 42. In most preferred embodiments, the functional enzyme with Ome activity has 100% sequence identity with SEQ ID NO: 41 or is encoded by a nucleotide sequence which has 100% sequence identity with SEQ ID NO: 42.

In a preferred embodiment the invention provides:
A host cell capable of expressing at least one of:
i) a functional enzyme with vanillin dehydrogenase (Vdh) activity, wherein said enzyme has at least 60%, preferably at least 80%, more preferably at least 90%, most preferably 100% sequence identity with SEQ ID NO: 1, or is encoded by a nucleotide sequence which has at least 60%, preferably at least 80%, more preferably at least 90%, most preferably 100% sequence identity with SEQ ID NO: 2; and
ii) a functional enzyme with vanillate hydroxylase (Vhy) activity, wherein said enzyme has at least 60%, preferably at least 80%, more preferably at least 90%, most preferably 100% sequence identity with SEQ ID NO: 3, or is encoded by a nucleotide sequence which has at least 60%, preferably at least 80%, more preferably at least 90%, most preferably 100% sequence identity with SEQ ID NO: 4;
wherein the host cell has reduced or no expression of a functional enzyme with methoxyhydroquinone 1,2-dioxygenase (Mhd) activity, wherein said enzyme has at least 60%, preferably at least 80%, more preferably at least 90%, most preferably 100% sequence identity with SEQ ID NO: 5, or is encoded by a nucleotide sequence which has at least 60%, preferably at least 80%, more preferably at least 90%, most preferably 100% sequence identity with SEQ ID NO: 6.

In some embodiments, the nucleotide sequence encoding at least one of the functional enzymes with Vdh, Vhy, Ome, or Mhd activity is codon optimized. A codon optimized nucleotide sequence is a sequence wherein the codon usage bias has been mitigated through selection of alternative codons without altering the encoded polypeptide. For example, rare codons can be replaced by more common codons, or regions comprising many identical codons can be interrupted by substitution of synonymic codons to reduce demand for the many identical codons. Codon optimization is known in the art, and bioinformatics tools for codon optimization are freely available on the internet from various providers. Accordingly, in some embodiments, the functional enzyme with Vdh activity is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO: 10. In preferred embodiments, the functional enzyme with Vdh activity is encoded by a nucleotide sequence which has at least 90% sequence identity with SEQ ID NO: 10. In still preferred embodiments, the functional enzyme with Vdh activity is encoded by a nucleotide sequence which has at least 95% sequence identity with SEQ ID NO: 10. In more preferred embodiments, the functional enzyme with Vdh activity is encoded by a nucleotide sequence which has at least 96% sequence identity with SEQ ID NO: 10. In still more preferred embodiments, the functional enzyme with Vdh activity is encoded by a nucleotide sequence which has at least 97% sequence identity with SEQ ID NO: 10. In still preferred embodiments, the functional enzyme with Vdh activity is encoded by a nucleotide sequence which has at least 98% sequence identity with SEQ ID NO: 10. In even more preferred embodiments, the functional enzyme with Vdh activity is encoded by a nucleotide sequence which has at least 99% sequence identity with SEQ ID NO: 10. In most preferred embodiments, the functional enzyme with Vdh activity is encoded by a nucleotide sequence which has 100% sequence identity with SEQ ID NO: 10.

In some embodiments the functional enzyme with Vhy activity is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO: 11. In preferred embodiments, the functional enzyme with Vhy activity is encoded by a nucleotide sequence which has at least 90% sequence identity with SEQ ID NO: 11. In still preferred embodiments, the functional enzyme with Vhy activity is encoded by a nucleotide sequence which has at least 95% sequence identity with SEQ ID NO: 11. In more preferred embodiments, the functional enzyme with Vhy activity is encoded by a nucleotide sequence which has at least 96% sequence identity with SEQ ID NO: 11. In still more preferred embodiments, the functional enzyme with Vhy activity is encoded by a nucleotide sequence which has at least 97% sequence identity with SEQ ID NO: 11. In still preferred embodiments, the functional enzyme with Vhy activity is encoded by a nucleotide sequence which has at least 98% sequence identity with SEQ ID NO: 11. In even more preferred embodiments, the functional enzyme with Vhy activity is encoded by a nucleotide sequence which has at least 99% sequence identity with SEQ ID NO: 11. In most preferred embodiments, the functional enzyme with Vhy activity is encoded by a nucleotide sequence which has 100% sequence identity with SEQ ID NO: 11.

In some embodiments the functional enzyme with Mhd activity is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO: 12. In preferred embodiments, the functional enzyme with Mhd activity is encoded by a nucleotide sequence which has at least 90% sequence identity with SEQ ID NO: 12. In still preferred embodiments, the functional enzyme with Mhd activity is encoded by a nucleotide sequence which has at least 95% sequence identity with SEQ ID NO: 12. In more preferred embodiments, the functional enzyme with Mhd activity is encoded by a nucleotide sequence which has at least 96% sequence identity with SEQ ID NO: 12. In still more preferred embodiments, the functional enzyme with Mhd activity is encoded by a nucleotide sequence which has at least 97% sequence identity with SEQ ID NO: 12. In still preferred embodiments, the functional enzyme with Mhd activity is encoded by a nucleotide sequence which has at least 98% sequence identity with SEQ ID NO: 12. In even more preferred embodiments, the functional enzyme with Mhd activity is encoded by a nucleotide sequence which has at least 99% sequence identity with SEQ ID NO: 12. In most preferred embodiments, the functional enzyme with Mhd activity is encoded by a nucleotide sequence which has 100% sequence identity with SEQ ID NO: 12.

In some embodiments the functional enzyme with Ome activity is encoded by a nucleotide sequence which has at least 60%, 65%, 70%, 75%, 80%, 85%, 90%, 91%, 92%, 93%, 94%, 95%, 96%, 97%, 98%, 99%, or 100%, preferably at least 80%, 85%, 90%, 91%, 92%, 93%, 94%, 95%, 96%, 97%, 98%, 99%, or 100%, more preferably at least 80%, even more preferably at least 90%, most preferably 100% sequence identity with SEQ ID NO: 43. In preferred embodiments, the functional enzyme with Ome activity is encoded by a nucleotide sequence which has at least 90% sequence identity with SEQ ID NO: 43. In still preferred embodiments, the functional enzyme with Ome activity is encoded by a nucleotide sequence which has at least 95% sequence identity with SEQ ID NO: 43. In more preferred embodiments, the functional enzyme with Ome activity is encoded by a nucleotide sequence which has at least 96% sequence identity with SEQ ID NO: 43. In still more preferred embodiments, the functional enzyme with Ome activity is encoded by a nucleotide sequence which has at least 97% sequence identity with SEQ ID NO: 43. In still preferred embodiments, the functional enzyme with Ome activity is encoded by a nucleotide sequence which has at least 98% sequence identity with SEQ ID NO: 43. In even more preferred embodiments, the functional enzyme with Ome activity is encoded by a nucleotide sequence which has at least 99% sequence identity with SEQ ID NO: 43. In most preferred embodiments, the functional enzyme with Ome activity is encoded by a nucleotide sequence which has 100% sequence identity with SEQ ID NO: 43.

In the context of the invention, it can be advantageous to use nucleic acid constructs that encode more than one functional enzyme. For example, a polycistronic construct can be used, or an operon wherein more than one enzyme is under the control of a single promoter. Such constructs can be created using recombinant DNA techniques that are well known in the art. Accordingly, in preferred embodiments this aspect provides a cell according to the invention, wherein at least two of the functional enzymes are encoded by a single recombinant polynucleotide construct. Such constructs can be comprised in an expression vector, which may be a plasmid or any other suitable vector known in the art as discussed below. The two functional enzymes may also be a single fusion polypeptide.

A 1-substituted 4-hydroxy-3-methoxybenzene according to the invention is a phenolic compound including, but not limited to, intermediates of metabolic pathways related to 4-hydroxy-3-methoxybenzenes. Examples of such compounds include coniferyl alcohol, ferulic acid, vanillyl alcohol, veratric acid, vanillin, vanillic acid, methoxyhydroquinone, 4-hydroxy-6-methoxy-6-oxohexa-2,4-dienoic acid, and the like, In some embodiments, the 1-substituted 4-hydroxy-3-methoxybenzene is selected from vanillin, vanillic acid and methoxyhydroquinone, preferably from vanillic acid and methoxyhydroquinone. In some embodiments, the 1-substituted 4-hydroxy-3-methoxybenzene is 4-hydroxy-6-methoxy-6-oxohexa-2,4-dienoic acid.

A cell according to the invention is able to produce a 1-substituted 4-hydroxy-3-methoxybenzene when cultured. Preferably, the cell is cultured under oxygen-limited or aerobic conditions, more preferably under aerobic conditions.

In the context of the invention, the term "production" of a compound refers to biosynthetic production and thus it can refer to production of said compound by a cell and/or cell lysate and/or isolated enzyme.

Production of a compound by a cell can be interpreted to encompass accumulation of said compound within a cell and/or excretion of said compound to a culture medium and/or a culture headspace. Production of a compound by a cell lysate and/or a functional enzyme can be interpreted to encompass its formation in a reaction medium. Said compound may accumulate in a culture medium and/or reaction medium or be removed from a culture medium and/or reaction medium simultaneously with its formation by means of isolation and/or purification using known techniques in the art. In some embodiments, a 1-substituted 4-hydroxy-3-methoxybenzene is accumulated within a cell. In preferred embodiments, a 1-substituted 4-hydroxy-3-methoxybenzene is excreted to a culture medium and/or a culture headspace. The excreted 1-substituted 4-hydroxy-3-methoxybenzene may accumulate, preferably accumulates in a culture medium.

Accumulation, as used herein, refers to an increase in the levels of the compound at hand where its production exceeds its consumption. Preferably, an accumulating compound is produced by a cell, and the excess amount has no further substantial role or no further role in any pathway. This can advantageously be achieved by disruption of one or more enzymes that would normally use the compound as a substrate, or by increasing the activity of one or more enzymes that would normally form the compound as a reaction product

Preferred cells according to the invention are capable of producing, preferably producing, more preferably accumulating, even more preferably excreting to the culture medium at least 1.0, 2.0, 3.0, 3.5, 4.0, 4.5, 5.0, 5.5, 6.0, 6.5, 7.0, 7.5, 8.0, 8.5, 9.0, 9.5, 10, 11, 12, 13, 14, 15, 16, 17, 18, 19, 20, 25, 30, 35, 40, 45, 50, 55, 60, 65, 70, 75, 80, 85, 90, 95, or 100 mM of a 1 -substituted 4-hydroxy-3-methoxybenzene, preferably at least 1.0 mM of a 1-substituted 4-hydroxy-3-methoxybenzene, more preferably at least 5.0 mM, even more preferably at least 10 mM. More preferred cells according to the invention are capable of producing, preferably producing, more preferably accumulating, even more preferably excreting to the culture medium at least 150 mM, 200 mM, 250 mM, 300 mM, 350 mM, 400 mM, 450 mM, 500 mM, 550 mM, 600 mM, 650 mM, 700 mM, 750 mM, 800 mM, 850 mM, 900 mM, 950 mM, or 1000 mM of a 1-substituted 4-hydroxy-3-methoxybenzene. Preferred cells according to the invention are able to convert at least 20%, 25%, 30%, 35%, 40%, 45%, 50%, 55%, 60%, 65%, 70%, 75%, 80%, 85%, 90%, 95%, or 100%, preferably at least 40%, more preferably at least 80%, of a carbon source into a 1-substituted 4-hydroxy-3-methoxybenzene. A carbon source to be converted is alternately referred to herein as substrate. Suitable substrates to be converted into an 1-substituted 4-hydroxy-3-methoxybenzene by a cell and/or cell lysate and/or functional enzyme of the invention will be known to the skilled person and are further discussed below. In cell cultures, the substrate may be the same carbon source as the one used for the growth of the cell and/or may be a separate carbon source which is additionally present in the growth medium, preferably it is the same carbon source. In preferred embodiments, the substrate comprises, essentially consists of, or consists of, preferably comprises, at least one of glucose, coniferyl alcohol, ferulic acid, vanillyl alcohol, veratryl alcohol, veratric aldehyde, and veratric acid, preferably at least one of coniferyl alcohol, ferulic acid, vanillyl alcohol, veratryl alcohol, veratric aldehyde, and veratric acid, most preferably at least one of coniferyl alcohol, ferulic acid. The above amount and/or substrate conversion is preferably obtained after 2 weeks, 1 week, 6 days, 5 days, 4 days, 3 days, 2 days, 24 hours, 15 hours, 10 hours, 5 hours, 4 hours, 3 hours, 2 hours or 1 hour of culture, preferably after 24 hours of culture, more preferably after 15 hours of culture and most preferably after 10 hours of culture. The production of 1-substituted 4-hydroxy-3-methoxybenzene can be monitored using chromatographic techniques on samples obtained from the growth medium, culture broth, culture supernatant, and/or culture headspace, preferably culture supernatant, as described elsewhere in the disclosure.

Accordingly, in a preferred embodiment the invention provides a host cell capable of expressing at least one of:
i) a functional enzyme with vanillin dehydrogenase (Vdh) activity; and
ii) a functional enzyme with vanillate hydroxylase (Vhy) activity;
wherein the host cell has reduced or no expression of a functional enzyme with methoxyhydroquinone 1,2-dioxygenase (Mhd) activity and wherein the host cell is capable of producing at least 1.0 mM of a 1-substituted 4-hydroxy-3-methoxybenzene, preferably after 24 hours of culture.

In some embodiments, Mhd is not reduced or absent, and the method is for production of 4-hydroxy-6-methoxy-6-oxohexa-2,4-dienoic acid.

In preferred embodiments, the 1-substituted 4-hydroxy-3-methoxybenzene is selected from vanillin, vanillic acid, and methoxyhydroquinone. In more preferred embodiments, the 1-substituted 4-hydroxy-3-methoxybenzene is selected from vanillic acid and methoxyhydroquinone. In most preferred embodiments, the 1-substituted 4-hydroxy-3-methoxybenzene is methoxyhydroquinone. In still most preferred embodiments, the 1-substituted 4-hydroxy-3-methoxybenzene is vanillic acid.

In a preferred embodiment the invention provides a host cell capable of expressing at least one of:
i) a functional enzyme with vanillin dehydrogenase (Vdh) activity; and
ii) a functional enzyme with vanillate hydroxylase (Vhy) activity; and
iii) a functional enzyme with Mhd activityl
wherein the host cell has reduced or no expression of a functional enzyme with Ome activity and wherein the host cell is capable of producing 6-methoxy-4,6-dioxohexanoic acid, preferably at least 1.0 mM of 6-methoxy-4,6-dioxohexanoic acid, preferably after 24 hours of culture. Preferably, at least two of i), ii), and iii) apply. More preferably all three apply. The host cell is preferably a filamentous fungus, as defined elsewhere herein.

### Method for production of a 1-substituted 4-hydroxy-3-methoxybenzene

The host cell according to the invention is useful for the production of 1-substituted 4-hydroxy-3-methoxybenzenes. Accordingly, in a second aspect the invention provides a method for the production of a 1-substituted 4-hydroxy-3-methoxybenzene, comprising:
- culturing a cell according to the invention under conditions conducive to the production of a 1-substituted 4-hydroxy-3-methoxybenzene, and, optionally,
- isolating and/or purifying the 1-substituted 4-hydroxy-3-methoxybenzene from the cell and/or the culture medium.

The features of this aspect are preferably as defined elsewhere herein. This method may hereinafter alternately be referred to as a process according to the invention. Suitable culturing methods for use in a process according to the invention are known to the skilled person and are discussed, for example, in van't Riet, K. and Tramper, J., 1st edition, Basic Bioreactor Design, CRC Press, NY, 1991. Such methods include, but are not limited to, submerged fermentation in liquid media, surface fermentation on liquid media and solid-state fermentations. Cell culturing may, for example, be performed by cultivation in micro-titer plates, shake-flasks, small-scale benchtop bioreactors, medium-scale bioreactors and/or large-scale bioreactors in a laboratory and/or an industrial setting. Suitable cell culturing modes include, but are not limited to, continuous, batch and/or fed-batch fermentation as well as their combinations. In an embodiment, cell culturing is performed using continuous fermentation. In a preferred embodiment, cell culturing is performed using batch fermentation. In a more preferred embodiment, cell culturing is performed using fed batch fermentation.

In the context of the invention, "culture medium", also alternately referred to herein as "growth medium", can be interpreted to encompass cases wherein the cultured cells are absent as well as cases wherein the cultured cells are present in the culture medium. "Culture broth" refers to the culture medium wherein the cultured cells are present. "Culture supernatant" refers to the culture medium wherein the cultured cells are absent. Cell culturing as part of the process of the invention can be performed under conditions conducive to the production of 1-substituted 4-hydroxy-3-methoxybenzene, which are known to the skilled person. Such conditions depend not only on the chemical composition of the culture medium but also on other process parameters including culture duration, temperature, O₂ levels in the culture broth and/or headspace, CO₂ levels in the culture broth and/or headspace, pH, agitation speed, hydrostatic pressure and the like. Cell culturing can take place using a culture medium comprising suitable nutrients, such as carbon and nitrogen sources and additional compounds such as inorganic salts and vitamins, using procedures known in the art (see, e. g. Bennett, W. and Lasure, L., 1st edition, More Gene Manipulations in Fungi, Academic Press, CA, 1991). Suitable growth media are available from commercial suppliers or may be prepared using published compositions (e.g. in catalogues of the Centraalbureau Voor Schimmelcultures collection (CBS)). An example of a suitable growth medium is a complete medium, comprising about 6 g/L NaNO₃, 1.5 g/L KH₂PO₄, 0.5 g/L KCl, 0.5 g/L MgSO₄*7H₂O, 0.2 mL/L trace elements (stock solution containing: 50 g/L ethylenediamine tetra-acetic acid, 22 g/L ZnSO₄*7H₂O, 5.54 g/L CaCl₂, 5.06 g/L MnCl₂*4H₂O, 4.99 g/L FeSO₄*7H₂O, 1.10 g/L (NH₄)₆Mo₇O₂₄*4H₂O, 1.57 g/L CuSO₄*5H₂O, 1.61 g/L CoCl₂*6H₂O; or else derived from 1 liter 5000 x concentrated which comprises about 10 g EDTA, 4.4 g ZnSO₄7H₂O, 1.01 g MnCl₂·4H₂O, 0.32 g CoCl_{2·}6H₂O, 0.315 g CuSO₂·5H₂O, 0.22 g (NH₄)₆Mo₇O₂₄·4H₂O, 1.47 g CaCl₂·2H₂O, 1.0 g FeSO₄ 7H₂O), 0.2% (mass/vol) tryptone, 0.1% (mass/vol) yeast extract, 0.1% (mass/vol) casamino acids, 0.05% (mass/vol) yeast RNA, and a suitable carbon source, preferably glucose, fructose or an aromatic compound, more preferably an aromatic compound.

The exact composition of the growth medium and the values of process parameters are not critical features of the invention. Any growth medium composition may be contemplated, as long as it allows for growth of the host cell and production of a 1-substituted 4-hydroxy-3-methoxybenzene. Examples of suitable carbon sources known in the art include, but are not limited to, simple sugars such as glucose, maltose, sucrose, xylose, arabinose, complex sugars such as maltodextrins, hydrolysed starch, starch, molasses, second generation feedstocks such as biomass hydrolysates coming from corn stover, wheat straw, corn fiber, oils, and the like. Additional examples of carbon sources that may be contemplated are coniferyl alcohol, ferulic acid, vanillyl alcohol, vanillin, vanillic acid, veratryl alcohol, veratric aldehyde, veratric acid and the like. Carbon sources such as other organic acids, aldehydes, ketones, esters and alcohols may also be contemplated. The use of growth media comprising combinations of multiple different carbon sources may also be contemplated in the process of the invention. Such media could, as a non-limiting example, combine more oxidized carbon sources such as organic acids with more reduced carbon sources such as alcohols. In an embodiment, the growth medium comprises one or more carbon sources selected from coniferyl alcohol, ferulic acid, vanillyl alcohol, veratryl alcohol, veratric aldehyde and veratric acid. In a preferred embodiment, the growth medium comprises one or more carbon sources selected from coniferyl alcohol and ferulic acid. In a more preferred embodiment, the growth medium comprises ferulic acid and/or coniferyl alcohol, most preferably ferulic acid. Examples of suitable nitrogen sources known in the art include soy bean meal, corn steep liquor, yeast extract, whey protein, egg protein, casein hydrolysate, urea, ammonia, ammonium salts and nitrate salts. Examples of additional suitable compounds that may be present in the growth medium and that are known in the art include phosphate, sulphate, metals such as magnesium, trace elements and vitamins. The exact growth medium requirements will vary based on the host cell, e.g. between yeasts, bacteria and filamentous fungi, and the targeted 1-substituted 4-hydroxy-3-methoxybenzene to be produced; said requirements will be known to the skilled person. Accordingly, the growth medium may be a complete (rich) medium or a minimal medium. The growth medium may be a complex medium or a defined medium. In a preferred embodiment, the culture medium is a defined medium, more preferably it is a minimal medium, most preferably it is a minimal medium comprising about 6 g/L NaNO₃, 1.5g g/L KH₂PO₄, 0.5 g/L KCl, 0.5 g/L MgSO₄*7H₂O, 0.2 mL/L trace elements (stock solution containing: 50 g/L ethylenediamine tetra-acetic acid, 22g/L ZnSO₄*7H₂O, 5.54 g/L CaCl₂, 5.06 g/L MnCl₂*4H₂O, 4.99 g/L FeSO₄*7H₂O, 1.10 g/L (NH₄)6MO₇O₂₄*4H₂O, 1.57 g/L CuSO₄*5H₂O, 1.61 g/L CoCl₂*6H₂O or as defined supra), and a suitable carbon source, preferably glucose, fructose or an aromatic compound, more preferably an aromatic compound.

Similarly to the composition of the growth medium, process parameters can be assigned any value, as long as it allows for growth of the host cell and production of a 1-substituted 4-hydroxy-3-methoxybenzene. Typically, said values will differ based on the host cell that is being cultured and will be known to the skilled person.

In a preferred embodiment the process according to the invention is an oxygen-limited or aerobic process, meaning that cell culturing is performed under oxygen-limited or aerobic conditions, more preferably the process is oxygen-limited. Oxygen-limited conditions, also known as a micro-aerobic conditions, are culture conditions in which the oxygen consumption is limited by the availability of oxygen. The degree of oxygen limitation is determined by the amount and composition of the ingoing gas flow as well as the actual mixing/mass transfer properties of the fermentation equipment used. Preferably, under oxygen-limited conditions in a liquid culture, the rate of oxygen consumption is at least about 5.5, more preferably at least about 6 and even more preferably at least about 7 mmol/L/h. Aerobic conditions are culture conditions in which the oxygen consumption is not limited by the availability of oxygen.

Cell culturing is performed at a temperature value that is optimal for the cell. When yeasts or fungal host cells are cultured, the fermentation process is preferably performed at a temperature range of 20-42 °C, more preferably at a temperature range of 22-38 °C, even more preferably at a temperature range of 25-35 °C, most preferably at a temperature range of 28-32 °C. In some most preferred embodiments, a temperature value of about 30 °C is used.

Cell culturing is performed at a pH value that is optimal for the cell. In some embodiments, the culture pH value is about pH 2.5, about pH 3.0, about pH 3.5, about pH 4.0, about pH 4.5, about pH 5, about pH 5.5, about pH 6, about pH 6.5, about pH 7, about pH 7.5, about pH 8.0, about pH 8.5, about pH 9. In preferred embodiments, the pH range is from about pH 3.0 to about pH 9, more preferably from about pH 3.5 to pH 6.5. In some most preferred embodiments, the pH is from about 5.8 to 6, such as about 6.

As a non-limiting example, a temperature of about 30 °C, an agitation speed of about 250 rpm and a pH value of about 6 can be used as process parameters when shake-flask cultivation is performed.

Cell culturing may also be performed by implementation of a multiple step, preferably a two-step, culture method. For example, a production step of a 1-substituted 4-hydroxy-3-methoxybenzene may be preceded by a biomass growth step, wherein only limited production or no production is taking place. The different steps may be carried out using different culture modes and/or different growth media and/or different process parameter values, depending on the goal of each step and/or the cultured cell. The biomass during the production step may or may not be actively growing. Accordingly, in a preferred embodiment, *Aspergillus niger* cells according to the invention are cultured. The starting cells can be fresh or originate from frozen samples. Starting cells can originate from mycelia, protoplasts or spores, preferably from spores. The starting cells can originate from cultures grown on rich medium or minimal medium. In a more preferred embodiment, *Aspergillus niger* spores are inoculated, preferably in shake flasks containing a liquid complete medium with about 2% fructose, more preferably a complete medium as described in the Examples, preferably at a concentration of about 1 x 10⁶ spores/mL and incubated overnight in a rotary shaker at a temperature of about 30 °C and agitation speed of about 250 rpm. Freshly germinated mycelia are harvested, preferably on Miracloth (supplied by Merck KGaA, Darmstadt, Germany), and washed, preferably with a minimal medium, more preferably a minimal medium as described in the Examples. Portions, preferably equal portions, of mycelia are transferred, preferably to shake flasks containing about 50 mL of a minimal medium, more preferably a minimal medium as described in the Examples, and about 0.02% (w/v) of an aromatic compound, preferably selected from coniferyl alcohol, vanillic acid or veratric acid, and incubated for about 2 hours in a rotary shaker at a temperature of about 30 °C and agitation speed of about 250 rpm. Optionally, mycelia are harvested, dried between a tissue paper to remove excess liquid and frozen in liquid nitrogen. Equal portions of fresh or frozen mycelia, preferably fresh mycelia, are transferred, preferably to shake flasks containing about 50 mL of a minimal medium, more preferably a minimal medium as described in the Examples, and about 5 mM of an aromatic compound, preferably selected from coniferyl alcohol, ferulic acid, vanillyl alcohol, vanillin, vanillic acid, veratryl alcohol, veratric aldehyde or veratric acid. The cultures are incubated in a rotary shaker, preferably for about 24 hours at a temperature of about 30 °C and an agitation speed of about 250 rpm.

In the context of the process of the invention the produced 1-substituted 4-hydroxy-3-methoxybenzene may optionally be isolated and/or purified from the cell and/or the culture medium and/or the culture headspace. The relevant downstream processing technology that may be suitable for use is not a critical feature of the invention and will depend on whether the produced 1-substituted 4-hydroxy-3-methoxybenzene is accumulated within the cultured cells or excreted. Said processing technology and the associated choice will be known to the skilled person and is discussed, for example, in Wesselingh, J.A and Krijgsman, J., 1st edition, Downstream Processing in Biotechnology, Delft Academic Press, NL, 2013. In a non-limiting example of a recovery process, the biomass is separated from the culture medium using e.g. centrifugation or filtration. If the produced 1-substituted 4-hydroxy-3-methoxybenzene is accumulated within the cells, it can then be isolated and/or purified from the biomass. If it is excreted, it can be recovered from the cell-free medium or, if the biomass separation step is skipped, directly from the culture broth. Isolation and/or purification may be performed according to any conventional isolation or purification methodology known in the art including, but not limited to, alteration of pH, solvent extraction, dialysis, cell filtration and ultrafiltration, concentration, lyophilisation and the like. In a preferred embodiment, the produced 1-substituted 4-hydroxy-3-methoxybenzene is isolated from the culture medium. This may be realized continuously with the production process or subsequently to it.

The produced 1-substituted 4-hydroxy-3-methoxybenzene may be selected from compounds such as, but not limited to, coniferyl alcohol, ferulic acid, veratric acid, vanillyl alcohol, vanillin, vanillic acid, methoxyhydroquinone and 2-methoxy-4-hydromuconic semialdehyde, preferably vanillin, vanillic acid and methoxyhydroquinone, more preferably vanillic acid and methoxyhydroquinone, most preferably methoxyhydroquinone, still most preferably vanillic acid. In some embodiments, the produced 1-substituted 4-hydroxy-3-methoxybenzene is 2-methoxy-4-hydromuconic semialdehyde.

Accordingly, in an embodiment a method is provided, wherein the method is for the production of vanillin, wherein the method comprises culturing a cell under conditions conducive to the production of vanillin, wherein the cell is a cell capable of expressing, or has reduced or no expression of, a functional enzyme with vanillin dehydrogenase (Vdh) activity and has reduced or no expression a functional enzyme with vanillate hydroxylase (Vhy) activity and has reduced or no expression of a functional enzyme with methoxyhydroquinone 1,2-dioxygenase (Mhd) activity, and, optionally, isolating and/or purifying the vanillin from the cell and/or culture medium. Preferably, the culture medium comprises a carbon source selected from coniferyl alcohol, ferulic acid, vanillyl alcohol, vanillic acid or veratric acid.

In an embodiment a method is provided, wherein the method is forthe production of vanillin, wherein the method comprises culturing a cell under conditions conducive to the production of vanillin, wherein the cell is a cell capable of expressing, or has reduced or no expression of, a functional enzyme with vanillin dehydrogenase (Vdh) activity and has reduced or no expression a functional enzyme with vanillate hydroxylase (Vhy) activity and is capable of expressing a functional enzyme with methoxyhydroquinone 1,2-dioxygenase (Mhd) activity, and, optionally, isolating and/or purifying the vanillin from the cell and/or culture medium. Preferably, the culture medium comprises a carbon source selected from coniferyl alcohol, ferulic acid, vanillyl alcohol, vanillic acid or veratric acid.

In another embodiment a method is provided, wherein the method is for the production of vanillic acid, wherein the method comprises culturing a cell under conditions conducive to the production of vanillic acid, wherein the cell is a cell capable of expressing a functional enzyme with vanillin dehydrogenase (Vdh) activity and has reduced or no expression of a functional enzyme with vanillate hydroxylase (Vhy) activity and has reduced or no expression of a functional enzyme with methoxyhydroquinone 1,2-dioxygenase (Mhd) activity, and, optionally, isolating and/or purifying the vanillic acid from the cell and/or culture medium. Preferably, the culture medium comprises a carbon source selected from coniferyl alcohol, ferulic acid, vanillyl alcohol, vanillin or veratric acid.

In another embodiment a method is provided, wherein the method is for the production of vanillic acid, wherein the method comprises culturing a cell under conditions conducive to the production of vanillic acid, wherein the cell is a cell capable of expressing a functional enzyme with vanillin dehydrogenase (Vdh) activity and has reduced or no expression of a functional enzyme with vanillate hydroxylase (Vhy) activity and is capable of expressing a functional enzyme with methoxyhydroquinone 4,5-dioxygenase (Mhd) activity, and, optionally, isolating and/or purifying the vanillic acid from the cell and/or culture medium. Preferably, the culture medium comprises a carbon source selected from coniferyl alcohol, ferulic acid, vanillyl alcohol, vanillin or veratric acid.

In a further embodiment a method is provided, wherein the method is for the production of methoxyhydroquinone, wherein the method comprises culturing a cell under conditions conducive to the production of methoxyhydroquinone, wherein the cell is a cell capable of expressing a functional enzyme with vanillin dehydrogenase (Vdh) activity and is capable of expressing a functional enzyme with vanillate hydroxylase (Vhy) activity and has reduced or no expression of a functional enzyme with methoxyhydroquinone 1,2-dioxygenase (Mhd) activity, and, optionally, isolating and/or purifying the methoxyhydroquinone from the cell and/or culture medium. Preferably, the culture medium comprises a carbon source selected from coniferyl alcohol, ferulic acid, vanillyl alcohol, vanillin, vanillic acid or veratric acid.

In a further embodiment a method is provided, wherein the method is for the production of methoxyhydroquinone, wherein the method comprises culturing a cell under conditions conducive to the production of methoxyhydroquinone, wherein the cell has reduced or no expression of a functional enzyme with vanillin dehydrogenase (Vdh) activity and is capable of expressing a functional enzyme with vanillate hydroxylase (Vhy) activity and has reduced or no expression of a functional enzyme with methoxyhydroquinone 1,2-dioxygenase (Mhd) activity, and, optionally, isolating and/or purifying the methoxyhydroquinone from the cell and/or culture medium. Preferably, the culture medium comprises a carbon source selected from coniferyl alcohol, ferulic acid, vanillyl alcohol, vanillin, vanillic acid or veratric acid.

In a further embodiment a method is provided, wherein the method is for the production of 4-hydroxy-6-methoxy-6-oxohexa-2,4-dienoic acid, wherein the method comprises culturing a cell under conditions conducive to the production of 4-hydroxy-6-methoxy-6-oxohexa-2,4-dienoic acid, wherein the cell has reduced or no expression of a functional enzyme with vanillin dehydrogenase (Vdh) activity and is capable of expressing a functional enzyme with vanillate hydroxylase (Vhy) activity and a functional enzyme with methoxyhydroquinone 1,2-dioxygenase (Mhd) activity, and, optionally, isolating and/or purifying the 4-hydroxy-6-methoxy-6-oxohexa-2,4-dienoic acid from the cell and/or culture medium. Preferably, the culture medium comprises a carbon source selected from coniferyl alcohol, ferulic acid, vanillyl alcohol, vanillin, vanillic acid, methoxyhydroquinone or veratric acid.

In a further embodiment a method is provided, wherein the method is for the production of 4-hydroxy-6-methoxy-6-oxohexa-2,4-dienoic acid, wherein the method comprises culturing a cell under conditions conducive to the production of 4-hydroxy-6-methoxy-6-oxohexa-2,4-dienoic acid, wherein the cell has reduced or no expression of a functional enzyme with vanillin dehydrogenase (Vdh) activity and a functional enzyme with vanillate hydroxylase (Vhy) activity and is capable of expressing a functional enzyme with methoxyhydroquinone 1,2-dioxygenase (Mhd) activity, and, optionally, isolating and/or purifying the 4-hydroxy-6-methoxy-6-oxohexa-2,4-dienoic acid from the cell and/or culture medium. Preferably, the culture medium comprises a carbon source selected from coniferyl alcohol, ferulic acid, vanillyl alcohol, vanillin, vanillic acid, methoxyhydroquinone or veratric acid.

In an preferred embodiment, the cultured host cells used in the method of the invention are immobilized. Immobilization of cells may be achieved by any means known to the skilled person as for example, discussed in standard handbooks such as Guisan, J.M., Bolivar, J.M., Lopez-Gallego, F., Rocha-Martin, J. (Eds.), Immobilization of Enzymes and Cells: Methods and Protocols, Springer US, USA, 2020. Typically, the host cells can be immobilized on a semi-solid or solid support by three different methods. The first method involves polymerizing or solidifying a spore- or cell-containing solution. Examples of polymerizable or solidifyable solutions include alginate, λ-carrageenan, chitosan, polyacrylamide, polyacrylamide-hydrazide, agarose, polypropylene, polyethylene glycol, dimethyl acrylate, polystyrene divinyle benzene, polyvinyl benzene, polyvinyl alcohol, epoxy carrier, cellulose, cellulose acetate, photocrosslinkable resin, prepolymers, urethane, and gelatin. The second method involves cell adsorption onto a support. Examples of such supports include bone char, cork, clay, resin, sand porous alumina beads, porous brick, porous silica, celite, or wood chips. The host cells can colonize the support and form a biofilm. The third method involves the covalent coupling of the host cells to a support using chemical agents like glutaraldehyde, o-dianisidine (U.S. Pat. No. 3,983,000), polymeric isocyanates (U.S. Pat. No. 4,071,409), silanes (U.S. Pat. Nos. 3,519,538 and 3,652,761), hydroxyethyl acrylate, transition metal-activated supports, cyanuric chloride, sodium periodate, toluene, and the like. Cultured host cells can be immobilized in any phase of their growth, for example after a desired cell density in the culture has been reached. Suitable culture modes and/or different culture process parameter values will be known to the skilled person and are discussed, for example, in standard handbooks, such as Colin R. Phillips C.R., Poon Y. C., Immobilization of Cells: In Biotechnology Monographs book series (Biotechnology, volume 5), Springer, Berlin, Germany, 1988; Tampion J., Tampion M. D., Immobilized Cells: Principles and Applications, Cambridge University Press, UK, 1987. Preferably, immobilized cells are cultured in packed bed bioreactors, also known as plug-flow bioreactors, or expanded (fluidized) bed bioreactors. Suitable growth media. and product isolation and/or purification methods are discussed elsewhere herein.

The 1-substituted 4-hydroxy-3-methoxybenzene produced by a cell according to the invention and by a method according to the invention has specific advantageous properties, such as being free of trace impurities that remain after chemical synthesis and being "natural-like". Accordingly, there is provided for 1-substituted 4-hydroxy-3-methoxybenzene obtainable by a process according to the invention. A 1-substituted 4-hydroxy-3-methoxybenzene obtained using a process according to the invention can conveniently be used in a product. Accordingly, there is provided for a pharmaceutical composition, a flavour composition, a fragrance composition, a cosmetic composition, or a food composition comprising a 1-substituted 4-hydroxy-3-methoxybenzene obtainable by a process according to the invention.

### Nucleic acid constructs and expression vectors

For expression of a functional enzyme in a host cell according to the invention, as well as for genetic modification of a host cell resulting in reduced or no expression of a functional enzyme according to the invention, the cell can be transformed with a nucleic acid or nucleic acid construct described herein by any method known to the person skilled in the art. Such methods are e.g. known from standard handbooks, such as Sambrook and Russel, Molecular Cloning: A Laboratory Manual, 3rd edition, Cold Spring Harbor Laboratory, Cold Spring Harbor Laboratory Press, NY, 2001, Ausubel F. et al, eds., Current protocols in molecular biology, Green Publishing and Wiley Interscience, NY, 2003, Sherman et al., Methods Yeast Genetics, Cold Spring Harbor Laboratory, NY, 1978; Guthrie et al. (Eds.) Guide To Yeast Genetics and Molecular Biology Vol. 194, Academic Press, San Diego, 1991; Sudbery, P. E., Genetic Engineering of Yeast, in Biotechnology Set, Second Edition (eds H.-J. Rehm and G. Reed), Wiley-VCH Verlag GmbH, Weinheim, Germany, 2001. Examples include transformation using competent or supercompetent cells, electroporation, use of transfection lipids, use of transfection polymers, or gymnotic transformation. A preferred method is electroporation. For transformation of filamentous fungal cells, mycelia, spores, and/or protoplasts, preferably protoplasts, may be used, as described in standard handbooks such as Moore, M., Genetic engineering of fungal cells, In Biotechnology Vol. III (eds H. W. Doelle and E. J. Dasilva), EOLSS, Ontario, Canada, 2007. Further suitable methods for transformation of *Aspergillus* cells are described in Kowalczyk et al., 2017, and Yelton et al., 1984, Proceedings of the National Academy of Sciences USA 81: 1470-1474.

A nucleic acid construct may be defined as a nucleotide sequence which is isolated from a naturally occurring (native) gene or which has been modified to contain segments of nucleotide sequences which are combined or juxtaposed in a manner which would not otherwise exist in nature. An isolated nucleotide sequence is a DNA or RNA sequence which is not immediately contiguous with one or both the nucleotide sequences (on the 5' and 3' ends) with which it is naturally immediately contiguous in the genome of the organism it is derived from.

Optionally, mutants of naturally occurring genes are made by modifying the nucleotide sequence encoding the naturally occurring genes using well-known mutagenesis techniques such as random mutagenesis, site-directed mutagenesis, directed evolution, gene shuffling and the like, so that the resulting nucleotide sequence encodes an enzyme that differs by at least one amino acid from the naturally occurring enzyme, said enzyme having improved properties with respect to (stereo)selectivity and/or activity and/or stability and/or solvent resistance and/or pH and/or temperature profiles as compared to the naturally occurring enzyme. Optionally, a nucleotide sequence present in a nucleic acid construct is operably linked to one or more regulatory sequences, which direct expression of said nucleotide sequence in a cell. Such regulatory sequences typically at least include a promoter that functions to control the transcription of the coding sequence, which is usually located upstream of, and preferably operably linked to the coding sequence. In addition to the promoter, the upstream transcription regulatory sequences may comprise further elements such as enhancers, upstream activating sequences, transcription factor binding sites, repressor and activator protein binding sites and the like. The promoter sequence will usually include the transcription initiation site(s).

Downstream of the promoter and transcription initiation site(s), the nucleic acid construct may optionally comprise the translation initiation sequences, such as the eukaryotic Kozak consensus sequence, surrounding the translation initiation codon, i.e. the first codon of the coding sequence. The coding sequence is terminated with a translation stop codon. Downstream of the coding sequence, the expression construct may optionally comprise a 3'-untranslated region containing one or more transcription termination sites, e.g. a terminator, which preferably also includes a polyadenylation site. Preferably, the terminator is endogenous to the host cell (in which the polypeptide is to be expressed).

Nucleic acid sequences described herein may be codon optimized. Codon optimization adapts the codon usage towards the codon bias of the organism where the nucleic acid sequence is expressed in. Codon optimization generally helps to increase the production level of the encoded polypeptide in the host cell. Many algorithms are available to the person skilled in the art for codon optimization. A preferred method is the "Guided random method based on a Monte Carlo alogorithm" available via the internet at genomes.urv.es/OPTIMIZER/ (P. Puigbò, E. Guzman, A. Romeu, and S. Garcia-Vallve. Nucleic Acids Res. 2007 July; 35 (Web Server issue): W126-W131).

As used herein the term "disruption" of a functional enzyme encompasses all possible host cell modifications that result in said enzyme activity being decreased or eliminated as compared to a corresponding cell that has not been modified and/or said enzyme activity no longer being detectable in the host cell and/or culture medium containing the host cell, as compared to a corresponding cell that has not been modified and/or culture medium containing a corresponding cell that has not been modified. Non-limiting examples of host cell modifications include modification of any step of the expression of a functional enzyme such as transcription, post-transcriptional modification, translation, post-translational modification, and secretion. Preferably, a disruption is achieved by deleting a gene encoding for a functional enzyme and/or its regulatory sequences, said disruption resulting in elimination of the enzyme transcript and, thereby, the enzyme activity. Said disruption may preferably be achieved by homologous recombination, following e.g. transformation of a host cell with a marker gene flanked by homologous regions to the targeted locus as described elsewhere herein. The disruption may preferably be assayed using molecular methods known to the skilled person, such as PCR and/or Southern blotting, or by enzymatic activity assays as discussed elsewhere herein.

"Gene" is defined herein as a DNA nucleotide sequence comprising a region (transcribed region), which is transcribed into an RNA molecule (e.g. an mRNA) in a cell, operably linked to suitable regulatory sequences (e.g. a promoter). A gene will usually comprise several operably linked fragments, such as a promoter, a 5' leader sequence, a coding region and 5' a 3'-nontranslated sequence (3'-end) comprising a polyadenylation site.

As used herein the term "heterologous sequence" or "heterologous nucleic acid" also encompasses a sequence that is not naturally found operably linked as neighbouring sequence of said first nucleotide sequence, which is preferably derived from a different organism from the host cell. As used herein, the term "heterologous" may also mean "recombinant". "Recombinant" refers to a genetic entity distinct from that generally found in nature. As applied to a nucleotide sequence or nucleic acid molecule, this means that said nucleotide sequence or nucleic acid molecule is the product of various combinations of cloning, restriction and/or ligation steps, and other procedures that result in the production of a construct that is distinct from a sequence or molecule found in nature. A recombinant nucleic acid can be a nucleic acid that comprises sequences from more than one single source. A recombinant polypeptide such as an enzyme is one expressed from a recombinant nucleic acid.

"Operably linked" is defined herein as a configuration in which a control sequence is appropriately placed at a position relative to the nucleotide sequence coding for the polypeptide of the invention such that the control sequence directs the production/expression polypeptide of the invention in a cell and/or in a subject. "Operably linked" may also be used for defining a configuration in which a sequence is appropriately placed at a position relative to another sequence coding for a functional domain such that a chimeric polypeptide is encoded in a cell and/or in a subject. Throughout this disclosure, any nucleic acid sequence coding for a functional enzyme is preferably operably linked to another such sequence, or to a promoter.

The terms "expression vector" or "expression construct" refer to nucleotide sequences that are capable of effecting expression of a gene in host cells or host organisms compatible with such sequences. These expression vectors typically include at least suitable promoters and optionally, 3' transcription termination signals. Additional factors necessary or helpful in effecting expression may also be present, such as expression enhancer elements. The expression vector will suitable for introduction into a suitable host cell and be able to effect expression of the coding sequence in a cell culture of the host cell.

As used herein, the term "promoter" refers to a nucleic acid fragment that functions to control the transcription of one or more nucleic acid molecules, usually located upstream with respect to the direction of transcription of the transcription initiation site of the nucleic acid molecule, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active under most environmental and developmental conditions. An "inducible" promoter is a promoter that is active under environmental or developmental regulation.

Optional further elements that may be present in a nucleic acid construct according to the invention include, but are not limited to, one or more leader sequences, enhancers, integration factors, and/or reporter genes, intron sequences, centromers, telomers and/or matrix attachment (MAR) sequences. A nucleic acid construct according to the invention can be provided in a manner known per se, which generally involves techniques such as restricting and linking nucleic acids/nucleic acid sequences, for which reference is made to the standard handbooks, such as Sambrook and Russel, Molecular Cloning: A Laboratory Manual, 3rd edition, Cold Spring Harbor Laboratory, Cold Spring Harbor Laboratory Press, NY, 2001.

Suitable elements useful for genetic modification of filamentous fungi are standard and known to those in the art, including e.g. promoters for (over-)expression of genes, other regulatory sequences such as terminators, episomal and/or integrating expression constructs and vectors, selectable markers, and methods and genetic constructs for disrupting and/or deleting endogenous fungal genes or parts thereof, described e.g. in Moore, M., Genetic engineering of fungal cells, In Biotechnology Vol. III (eds H. W. Doelle and E. J. Dasilva), EOLSS, Ontario, Canada, 2007; Lubertozzi, D., Keasling, J. D. (2009), Biotechnology advances, 27(1), 53-75; Meyer, V. (2008), Biotechnology Advances, (26), 177-85; Kuck and Hoff (2010), Applied microbiology and biotechnology, 86(1), 51-62; and WO2014/142647.

Accordingly, in a third aspect the invention provides an expression vector comprising a nucleic acid construct comprising a nucleotide sequence encoding a functional enzyme with vanillin dehydrogenase (Vdh) activity, and/or a functional enzyme with vanillate hydroxylase (Vhy) activity, and/or a functional enzyme with methoxyhydroquinone 4,5-dioxygenase (Mhd) activity, and/or a functional enzyme with Ome activity, preferably encoding a functional enzyme with methoxyhydroquinone 4,5-dioxygenase (Mhd) activity. The features of the nucleic acid constructs are preferably those as described herein. Such expression vectors are referred to herein as expression vectors according to the invention. Suitable expression vectors may be selected from any vector which can bring about the expression of the nucleic acid sequence encoding the enzymes of interest upon its introduction to the host cell according to methods known in the art as described herein. The choice of the expression vector will typically depend on the compatibility of the vector with the cell into which the vector is to be introduced. The expression vectors may be linear or closed circular plasmids. The vector may be an autonomously replicating vector, i.e. a vector which exists as an extra-chromosomal entity, the replication of which is independent of chromosomal replication, e.g., a plasmid, an extra-chromosomal element, a mini-chromosome, or an artificial chromosome. The expression vector may also be an integrative vector, i.e. a vector which integrates into the genome and replicates together with the chromosome(s) into which it has been integrated. The integrative expression vector may integrate at random or at a predetermined target locus in the chromosomes of the host cell. Preferably, the integrative cloning vector comprises a DNA fragment, which is homologous to a DNA sequence in a predetermined target locus in the genome of host cell for targeting the integration of the cloning vector to the predetermined locus. The integrative expression vector may be linearized prior to transformation of the cell. Linearization is preferably performed such that at least one but preferably either end of the cloning vector is flanked by sequences homologous to the target locus. The length of the homologous sequences flanking the target locus may be at least 30 bp, at least 50 bp, at least 0.1 kb, at least 0.2 kb, at least 0.5 kb, at least 1 kb, at least 2 kb. Preferably, the length of the homologous sequences flanking the target locus is at least 0.5 kb, more preferably at least 1kb, even more preferably 2 kb. Preferably, the efficiency of targeted integration into the genome of the host cell, i.e. integration in a predetermined target locus, is increased by augmenting the homologous recombination capability of the host cell, for example by prior disruption of non-homologous end-joining DNA repair machinery. Examples of such methods are discussed in WO2005/095624A2 and WO2005/095624A2.

In some embodiments the invention provides an expression vector comprising a nucleic acid construct comprising a nucleotide sequence that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 2 or 10, or wherein the expression vector consists of a polynucleotide that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 7. In preferred embodiments, the expression vector comprises a nucleic acid construct comprising a nucleotide sequence that has at least 90% sequence identity with SEQ ID NO: 2 or 10, or the expression vector consists of a polynucleotide that has at least 90% sequence identity with SEQ ID NO: 7. In still preferred embodiments, the expression vector comprises a nucleic acid construct comprising a nucleotide sequence that has at least 95% sequence identity with SEQ ID NO: 2 or 10, or the expression vector consists of a polynucleotide that has at least 95% sequence identity with SEQ ID NO: 7. In more preferred embodiments, the expression vector comprises a nucleic acid construct comprising a nucleotide sequence that has at least 96% sequence identity with SEQ ID NO: 2 or 10, or the expression vector consists of a polynucleotide that has at least 96% sequence identity with SEQ ID NO: 7. In still more preferred embodiments, the expression vector comprises a nucleic acid construct comprising a nucleotide sequence that has at least 97% sequence identity with SEQ ID NO: 2 or 10, or the expression vector consists of a polynucleotide that has at least 97% sequence identity with SEQ ID NO: 7. In still more preferred embodiments, the expression vector comprises a nucleic acid construct comprising a nucleotide sequence that has at least 98% sequence identity with SEQ ID NO: 2 or 10, or the expression vector consists of a polynucleotide that has at least 98% sequence identity with SEQ ID NO: 7. In even more preferred embodiments, the expression vector comprises a nucleic acid construct comprising a nucleotide sequence that has at least 99% sequence identity with SEQ ID NO: 2 or 10, or the expression vector consists of a polynucleotide that has at least 99% sequence identity with SEQ ID NO: 7. In most preferred embodiments, the expression vector comprises a nucleic acid construct comprising a nucleotide sequence that has 100% sequence identity with SEQ ID NO: 2 or 10, or the expression vector consists of a polynucleotide that has 100% sequence identity with SEQ ID NO: 7.

In some embodiments the invention provides an expression vector comprising a nucleic acid construct comprising a nucleotide sequence that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 4 or 11, or wherein the expression vector consists of a polynucleotide that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 8. In preferred embodiments, the expression vector comprises a nucleic acid construct comprising a nucleotide sequence that has at least 90% sequence identity with SEQ ID NO: 4 or 11, or the expression vector consists of a polynucleotide that has at least 90% sequence identity with SEQ ID NO: 8. In still preferred embodiments, the expression vector comprises a nucleic acid construct comprising a nucleotide sequence that has at least 95% sequence identity with SEQ ID NO: 4 or 11, or the expression vector consists of a polynucleotide that has at least 95% sequence identity with SEQ ID NO: 8. In more preferred embodiments, the expression vector comprises a nucleic acid construct comprising a nucleotide sequence that has at least 96% sequence identity with SEQ ID NO: 4 or 11, or the expression vector consists of a polynucleotide that has at least 96% sequence identity with SEQ ID NO: 8. In still more preferred embodiments, the expression vector comprises a nucleic acid construct comprising a nucleotide sequence that has at least 97% sequence identity with SEQ ID NO: 4 or 11, or the expression vector consists of a polynucleotide that has at least 97% sequence identity with SEQ ID NO: 8. In still more preferred embodiments, the expression vector comprises a nucleic acid construct comprising a nucleotide sequence that has at least 98% sequence identity with SEQ ID NO: 4 or 11, or the expression vector consists of a polynucleotide that has at least 98% sequence identity with SEQ ID NO: 8. In even more preferred embodiments, the expression vector comprises a nucleic acid construct comprising a nucleotide sequence that has at least 99% sequence identity with SEQ ID NO: 4 or 11, or the expression vector consists of a polynucleotide that has at least 99% sequence identity with SEQ ID NO: 8. In most preferred embodiments, the expression vector comprises a nucleic acid construct comprising a nucleotide sequence that has 100% sequence identity with SEQ ID NO: 4 or 11, or the expression vector consists of a polynucleotide that has 100% sequence identity with SEQ ID NO: 8.

In preferred embodiments the invention provides an expression vector comprising a nucleic acid construct comprising a nucleotide sequence that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 6 or 12, or wherein the expression vector consists of a polynucleotide that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 9. In preferred embodiments, the expression vector comprises a nucleic acid construct comprising a nucleotide sequence that has at least 90% sequence identity with SEQ ID NO: 6 or 12, or the expression vector consists of a polynucleotide that has at least 90% sequence identity with SEQ ID NO: 9. In still preferred embodiments, the expression vector comprises a nucleic acid construct comprising a nucleotide sequence that has at least 95% sequence identity with SEQ ID NO: 6 or 12, or the expression vector consists of a polynucleotide that has at least 95% sequence identity with SEQ ID NO: 9. In more preferred embodiments, the expression vector comprises a nucleic acid construct comprising a nucleotide sequence that has at least 96% sequence identity with SEQ ID NO: 6 or 12, or the expression vector consists of a polynucleotide that has at least 96% sequence identity with SEQ ID NO: 9. In still more preferred embodiments, the expression vector comprises a nucleic acid construct comprising a nucleotide sequence that has at least 97% sequence identity with SEQ ID NO: 6 or 12, or the expression vector consists of a polynucleotide that has at least 97% sequence identity with SEQ ID NO: 9 In still more preferred embodiments, the expression vector comprises a nucleic acid construct comprising a nucleotide sequence that has at least 98% sequence identity with SEQ ID NO: 6 or 12, or the expression vector consists of a polynucleotide that has at least 98% sequence identity with SEQ ID NO: 9. In even more preferred embodiments, the expression vector comprises a nucleic acid construct comprising a nucleotide sequence that has at least 99% sequence identity with SEQ ID NO: 6 or 12, or the expression vector consists of a polynucleotide that has at least 99% sequence identity with SEQ ID NO: 9. In most preferred embodiments, the expression vector comprises a nucleic acid construct comprising a nucleotide sequence that has 100% sequence identity with SEQ ID NO: 6 or 12, or the expression vector consists of a polynucleotide that has 100% sequence identity with SEQ ID NO: 9.

In preferred embodiments the invention provides an expression vector comprising a nucleic acid construct comprising a nucleotide sequence that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 42 or 43, or wherein the expression vector consists of a polynucleotide that has at least 60%, 65%, 70%, 75%, 80%, 85%, 90%, 95%, 96%, 97%, 98%, 99%, or 100%, preferably at least 60%, more preferably at least 80%, even more preferably at least 90%, most preferably 100% sequence identity with SEQ ID NO: 7, wherein the nucleotides of SEQ ID NO: 7 that overlap with SEQ ID NO: 10 are replaced by the nucleotides of SEQ ID NO: 42 or 43, which is referred to below as a (7+42/43=vector). In preferred embodiments, the expression vector comprises a nucleic acid construct comprising a nucleotide sequence that has at least 90% sequence identity with SEQ ID NO: 42 or 43, or the expression vector consists of a polynucleotide that has at least 90% sequence identity with a (7+42/43=vector). In still preferred embodiments, the expression vector comprises a nucleic acid construct comprising a nucleotide sequence that has at least 95% sequence identity with SEQ ID NO: 42 or 43, or the expression vector consists of a polynucleotide that has at least 95% sequence identity with a (7+42/43=vector). In more preferred embodiments, the expression vector comprises a nucleic acid construct comprising a nucleotide sequence that has at least 96% sequence identity with SEQ ID NO: 42 or 43, or the expression vector consists of a polynucleotide that has at least 96% sequence identity with a (7+42/43=vector). In still more preferred embodiments, the expression vector comprises a nucleic acid construct comprising a nucleotide sequence that has at least 97% sequence identity with SEQ ID NO: 42 or 43, or the expression vector consists of a polynucleotide that has at least 97% sequence identity with a (7+42/43=vector). In still more preferred embodiments, the expression vector comprises a nucleic acid construct comprising a nucleotide sequence that has at least 98% sequence identity with SEQ ID NO: 42 or 43, or the expression vector consists of a polynucleotide that has at least 98% sequence identity with a (7+42/43=vector). In even more preferred embodiments, the expression vector comprises a nucleic acid construct comprising a nucleotide sequence that has at least 99% sequence identity with SEQ ID NO: 42 or 43, or the expression vector consists of a polynucleotide that has at least 99% sequence identity with a (7+42/43=vector). In most preferred embodiments, the expression vector comprises a nucleic acid construct comprising a nucleotide sequence that has 100% sequence identity with SEQ ID NO: 42 or 43, or the expression vector consists of a polynucleotide that has 100% sequence identity with a (7+42/43=vector).

Alternatively or in combination, isolated nucleotide sequences or nucleic acid constructs comprising nucleotide sequences encoding for the functional enzymes according to the invention may be introduced to the host cells. Said nucleotide sequences and/or nucleic acid constructs may be flanked by sequences homologous to the target locus, similarly to when integrative expression vectors are used. Any nucleotide sequence and/or nucleic acid construct may be contemplated as long as it can bring about the expression of the functional enzymes according to the invention upon its introduction to the host cell according to methods known in the art as previously described herein. Preferably, such a nucleotide sequence and/or nucleic acid construct is integrated into the chromosome(s) of the host cell after transformation of the host cells. In some embodiments, the isolated nucleotide sequences or nucleic acid constructs are introduced to the host cells as repair fragments following CRISPR/Cas-mediated transformation according to methods known in the art, described for example in WO2016/110453.

The nucleic acid constructs and/or expression vectors according to the invention may further comprise a selectable marker. For expression vectors a selectable marker is preferred. The term "marker" refers herein to a gene encoding a trait or a phenotype which permits the selection of, or the screening for, a cell containing the marker. A marker gene may be an antibiotic resistance gene whereby the appropriate antibiotic can be used to select for transformed cells from among cells that are not transformed. A preferred selection marker is kanamycin and its corresponding resistance gene. Preferably however, a non-antibiotic resistance marker is used, such as an auxotrophic marker (Ura3, Trp1, Leu2). Other selectable markers, particularly useful for use in a filamentous fungal cell, are preferably selected from the group including, but not limited to, AmdS (acetamidase), ArgB (ornithine carbamoyltransferase), Bar (phosphinothricinacetyltransferase), BleA (phleomycin binding), HygB (hygromycin phosphotransferase), Nial (nitrate reductase), PyrG (orotidine-5'-phosphate decarboxylase), SC (sulfate adenyltransferase), and TrpC (anthranilate synthase), as well as equivalents. Preferably, the selectable marker is PyrG from *Aspergillus oryzae.* A preferred cell according to the invention, e.g. transformed with a nucleic acid construct, is marker gene free. Methods for constructing recombinant marker gene free microbial host cells are known to the skilled person and examples are available in handbooks referred to elsewhere herein.) Alternatively, a screenable marker such as Green Fluorescent Protein, LacZ, luciferase, chloramphenicol acetyltransferase, beta-glucuronidase may be incorporated into a nucleic acid construct according to the invention allowing to screen for transformed cells.

Expression of a functional enzyme by the host cell can be considered to include any step involved in the production of said enzyme including, but not limited to, transcription, post-transcriptional modification, translation, post-translational modification, and secretion. Expression of a functional enzyme can further include embodiments wherein any of the abovementioned steps are increased as compared to a corresponding cell that has not been modified in this way, resulting in increased levels of enzyme transcript, enzyme polypeptide, and/or enzyme activity as compared to said non-modified cell. Suitable methods to achieve said increase are known to the skilled person and are described, for example, in handbooks referred to elsewhere herein. Examples include replacing native promoters with strong constitutive or inducible promoters to drive gene expression, codon optimization, evolutionary engineering, random or directed mutagenesis, transformation with multiple copies of the relevant nucleotide sequences, nucleic acid constructs and/or expression vectors and the like.

Reduced expression of a functional enzyme refers to embodiments wherein any step involved in the production of said enzyme including, but not limited to, transcription, post-transcriptional modification, translation, post-translational modification, and secretion are reduced as compared to a corresponding cell that has not been modified in this way, resulting in decreased levels of enzyme transcript, enzyme polypeptide, and/or enzyme activity compared to a non-modified cell. Suitable methods to achieve said decrease are known to the skilled person. Examples include replacing native promoters with weak constitutive or inducible promoters to drive gene expression, replacing native genes with heterologous genes, random or directed mutagenesis, fusion of nucleic acid constructs with nucleotide sequences encoding for degradation tags, activating a transcriptional repressor, repressing a repressible promoter, decreasing RNA stability, activating a post-transcriptional inhibitor (for example, expressing a ribozyme or antisense oligonucleotide) or inhibiting translation (for example, via a regulatable RNA) and the like.

No or lack of expression of a functional enzyme refers to embodiments wherein the activity of the functional enzyme is not detectable in the host cell and/or culture medium, for instance because it has been eliminated, and can be the result of a disruption of any step involved in the production of said enzyme including, but not limited to, transcription, post-transcriptional modification, translation, post-translational modification, and secretion. For example, the gene encoding for said enzyme may be inactivated or deleted. Gene inactivation may be achieved by any suitable means as known to a skilled person, with examples being gene deletion, random or directed mutagenesis, PCR or site-directed mutagenesis, for example to disturb the open reading frame, activating a transcriptional repressor, repressing a repressible promoter, activating a post-transcriptional inhibitor (for example, expressing a ribozyme or antisense oligonucleotide) or inhibiting translation (for example, via a regulatable RNA) and the like. Gene deletion or inactivation should also be interpreted as to include embodiments wherein only its regulatory sequences (such as enhancer, promoter and terminator sequences) are deleted or inactivated instead of the complete gene (which includes the open reading frame), and embodiments wherein only the open reading frame is deleted or inactivated instead of the complete gene. Gene deletion may be achieved by any suitable means known to a skilled person including, for example, excising a gene or fragment thereof, and/or its regulatory sequences (such as enhancer, promoter and terminator sequences) using a FLP-FRT or Cre-Lox cassette, homologous recombination or CRIPR-Cas9 activity or through loss or degradation of a plasmid. Preferably, homologous recombination is used for gene deletion. In a preferred embodiment, the deleted nucleotide sequence is replaced by a selectable marker as described herein which is flanked by homologous sequences to the target locus as described herein, more preferably said selectable marker is PyrG, most preferably PyrG from *Aspergillus oryzae.*

In the context of the invention, no or lack of expression should not be interpreted as to exclude embodiments wherein the transcript encoding for the enzyme or the enzyme polypeptide are still present in the host cell. For example, an enzyme polypeptide might require a post-translational modification in order to be converted to a functional enzyme and the host cell might lack the capability of performing this post-translation modification while the polypeptide is present or an mRNA transcript may be translated to a polypeptide unable to fold in a way as to result in a functional enzyme.

In a preferred embodiment *Aspergillus niger* cells are transformed. Preferably, said cells are deficient in expression of ATP-dependent DNA helicase II subunit 1 (kusA). Gene deletion is preferably performed using homologous recombination using deletion cassettes comprising homologous sequences of at least 900 bp, preferably at least 1000 bp, upstream and downstream of the target gene, fused to an orotidine 5'-phosphate decarboxylase (pyrG) from *Aspergillus oryzae.* For protoplast preparation, young mycelia from overnight culture are harvested, preferably by vacuum filtration, washed with 0.4-0.8 M such as about 0.6 M MgSO₄ and dried, preferably between two sheets of paper. The mycelium is then dissolved, preferably in PS buffer (0.1-0.3 M such as about 0.2 M sodium phosphate buffer, 0.6-1 M such as about 0.8 M L-sorbitol, about pH 6) containing VinoTaste^{®} Pro lysing enzyme (about 0.5 g enzyme/g of mycelia) and incubated, preferably in a rotary shaker at an agitation speed of about 100 rpm. When protoplasts are abundantly present, the mixture is filtrated, preferably through glass wool, and undigested mycelia debris is removed. The protoplasts are collected, preferably by centrifugation (about 10 min, 1811 ×g, 4 °C), washed, preferably twice with ice-cold SC solution (about 182.2 g L⁻¹ sorbitol, 7.35 g L⁻¹ CaCl2*2H₂O), and resuspended, preferably in SC, to a final concentration of about 2*10⁷ protoplasts/mL. For transformation, about 200 µL of fresh protoplast suspension, 20 µL of 0.2-0.6 M such as about 0.4 M ATA (aurintricarboxylic acid ammonium salt), 100 µL of 10-30% such as about 20% PEG-4000 are mixed with about 5 µg of deletion cassette DNA and incubated for about 10 min. After addition of about 1.5 mL 50-70% such as about 60% PEG-4000 the mixture is incubated for about 20 min. Next, about 5 mL of 1-1.4 M such as about 1.2 M sorbitol solution is added and the mixture is incubated for about another 10 min. Transformed protoplasts are collected, preferably by centrifugation (about 10 min, 3220 ×g), resuspended in about 1 mL 1-1.4 M such as about 1.2 M sorbitol and spread over selective plates using a cell spreader. Growing colonies are observed after about 4 days.

### Polypeptides

In a fourth aspect, the invention provides a polypeptide product expressed from the expression vector according to the third aspect. Features and definitions are provided elsewhere herein. The polypeptide is preferably at least 70% pure, more preferably at least 75%, 80%, 85%, 90%, 95%, 96%, 97%, 98%, 99% pure, most preferably it is substantially pure. Optionally, the polypeptide product can be in a composition comprising it, preferably further comprising a buffered solution. Suitable compositions will be known to the skilled person and are discussed in standard handbooks, for example in and Bisswanger H, Practical Enzymology, 2nd edition, Wiley-VCH Verlag GmbH & Co. KgaA, 2012. An example of a suitable buffered solution is a phosphate-buffered saline solution.

### Uses

In a fifth aspect, the invention provides the use of a functional enzyme with methoxyhydroquinone 1,2-dioxygenase (Mhd) activity as defined earlier herein, forthe production of 4-hydroxy-6-methoxy-6-oxohexa-2,4-dienoic acid. The features of this aspect are preferably those of the previous aspects of the invention.

The functional enzyme may be used in any form including, but not limited to, a crude enzyme, a commercially available enzyme preparation, an enzyme further purified from a commercially available preparation, an enzyme obtained from its source by a combination of known isolation and/or purification methods, an immobilized enzyme, whole (optionally permeabilized and/or immobilized) cells that naturally or through genetic modification express the enzyme, or a cell lysate comprising the enzyme. The functional enzyme may be an isolated, synthetic or recombinant enzyme. The functional enzyme may be present in a suitable composition that allows for optimal enzymatic stability and/or activity. Preferably, such compositions will further comprise salt buffers and antifreezes such as glycerol. In a preferred embodiment, a functional enzyme isolated from its source, more preferably an immobilized enzyme, is used. In another preferred embodiment, whole (optionally permeabilized and/or immobilized) cells that naturally or through genetic modification express the functional enzyme are used. Means of immobilizing cells and enzymes are known in the art and are discussed in standard handbooks, such as Guisan, J.M., Bolivar, J.M., López-Gallego, F., Rocha-Martin, J. (Eds.), Immobilization of Enzymes and Cells: Methods and Protocols, Springer US, USA, 2020. For example, an enzyme may be bound directly to a membrane, granules or a polymer such as a resin having one or more functional groups or it may be bound to the resin through bridging compounds having one or more functional groups such as glutaraldehyde. Suitable forms also include isolated and/or purified naturally occurring enzymes, i.e. as they can be isolated and/or purified from a wild type source organism. The term "wild type" refers to the organism as it occurs in nature. Suitable forms may additionally include mutants of naturally occurring enzymes, which can for example be synthetically made or made by modifying the nucleotide sequence encoding for said enzymes using mutagenesis techniques known to the person skilled in the art, such as, random mutagenesis, site-directed mutagenesis, directed evolution, gene shuffling, CRISPR/Cas-mediated mutagenesis and the like, so that the resulting nucleotide sequence encodes an enzyme that differs by at least one amino acid from the naturally occurring enzyme, said enzyme having improved properties with respect to (stereo)selectivity and/or activity and/or stability and/or solvent resistance and/or pH and/or temperature profiles as compared to the naturally occurring enzyme.

In an embodiment, this aspect provides the use of a functional enzyme with methoxyhydroquinone 1,2-dioxygenase (Mhd) activity as defined earlier herein, forthe production of 4-hydroxy-6-methoxy-6-oxohexa-2,4-dienoic acid by a microbial cell, preferably a fungal cell, more preferably a filamentous fungal cell, most preferably an *Aspergillus niger* cell. The nucleotide sequence encoding for the functional enzyme with Mhd activity may be homologous or heterologous to the host cell, preferably it is heterologous. Preferably, the nucleotide sequence encoding for the functional enzyme with Mhd activity is codon-optimized.

The term reaction medium should be interpreted to encompass any suitable medium that can enable the enzymatic reaction to take place. Such media, as well as reaction conditions such as temperature, pH, ionic strength and the like, are known to the skilled person, and discussed for example in standard handbooks which are referred to elsewhere herein. Typically, besides the cell lysate and/or the functional enzyme and/or the composition comprising said enzyme, reaction media will comprise the reactants, enzyme co-factors such as metals where applicable, and suitable buffer solutions.

In an embodiment, a cell lysate comprising a functional enzyme with methoxyhydroquinone 1,2-dioxygenase (Mhd) activity or an isolated and/or a purified functional enzyme with methoxyhydroquinone 1,2-dioxygenase (Mhd) activity and/or a composition comprising a functional enzyme with methoxyhydroquinone 1,2-dioxygenase (Mhd) activity, preferably an isolated and/or purified functional enzyme, is used for the production of 4-hydroxy-6-methoxy-6-oxohexa-2,4-dienoic acid. Said production may occur in a suitable reaction medium. In a preferred embodiment, the reaction medium comprises at least 1 U (µmol/min) of a functional enzyme with Mhd activity, 100 µM, preferably at least 500 µM methoxyhydroquinone, 20-40 µM, preferably about 25 µM HEPES buffer and 40-60 µM, preferably about 50 µM FeSO₄, and the reaction is allowed to take place at about pH 6.0 and a temperature of about 30 °C for at least 10 min, preferably at least 30 min, more preferably at least 1 hour.

In an embodiment, a cell lysate comprising a functional enzyme with Ome activity or an isolated and/or a purified functional enzyme with Ome activity and/or a composition comprising a functional enzyme with Ome activity, preferably an isolated and/or purified functional enzyme, is used for the production of 3-oxo-adipic acid. Said production may occur in a suitable reaction medium. In a preferred embodiment, the reaction medium comprises at least 1 U (µmol/min) of a functional enzyme with Ome activity, 100 µM, preferably at least 500 µM 6-methoxy-4,6-dioxohexanoic acid, 20-40 µM, preferably about 25 µM HEPES buffer and 40-60 µM, preferably about 50 µM FeSO₄, and the reaction is allowed to take place at about pH 6.0 and a temperature of about 30 °C for at least 10 min, preferably at least 30 min, more preferably at least 1 hour.

A cell with reduced Ome activity may be used for the production of 6-methoxy-4,6-dioxohexanoic acid. This product will accumulate in the absence of sufficient Ome activity.

Cell lysing refers to a process of rupturing the cell wall and/or cell membrane of a cell as to obtain a cell lysate which comprises intracellular material and cellular debris. The exact method of cell lysing is not a critical feature of the invention. Such methods are well-known in the art (examples can be found in standard handbooks such as Sambrook and Russel, Molecular Cloning: A Laboratory Manual, 3rd edition, Cold Spring Harbor Laboratory, Cold Spring Harbor Laboratory Press, NY, 2001 or Ausubel F. et al, eds., Current protocols in molecular biology, Green Publishing and Wiley Interscience, NY, 2003) and include mechanical, chemical, enzymatic or physical treatment of cells or combinations of said treatments. Examples of mechanical treatment include cell homogenization using a French pressure cell press, a sonicator, a homogenizer, a ball mill, a rod mill, a pebble mill, a bead mill, a high pressure grinding roll, a vertical shaft impactor, an industrial blender, a high shear mixer, a paddle mixer, a polytron homogenizer and the like. Examples of chemical treatment include raising a pH of a cell, for example by adding a base to a cell culture, contacting a cell with a chemical and the like. Examples of enzymatic treatment include lysis of a cell wall or cell membrane of a cell by contacting the cell with one or more enzymes, such as proteases, cellulases, hemicellulases, chitinases, pectinases, and combinations thereof. Examples of physical treatment include heating a cell, drying a cell and the like. The cell lysate used may or may not still comprise the cellular debris, preferably it does not comprise the cellular debris. Methods for separating and/or removing cellular debris are well known in the art, for example centrifugation or filtration can be used. As an example, centrifugation for 5-10 min at 3000-4000 x g may be used to separate the cellular debris. A cell lysate not comprising the cellular debris may be called a cell-free extract. Methods for isolation and/or purification of enzymes are discussed elsewhere herein.

In an embodiment, the functional enzyme with Mhd activity is used in an immobilized state with an appropriate carrier. Suitable means of immobilizing enzymes are known in the art are discussed elsewhere herein. As an example, the enzyme may be bound directly to a membrane, granules or a polymer such as a resin having one or more functional groups or it may be bound to the resin through bridging compounds having one or more functional groups such as glutaraldehyde.

### General information

Unless stated otherwise, all technical and scientific terms used herein have the same meaning as customarily and ordinarily understood by a person of ordinary skill in the art to which this invention belongs, and read in view of this disclosure.

"Sequence identity" or "identity" in the context of amino acid- or nucleic acid-sequence is herein defined as a relationship between two or more amino acid (peptide, polypeptide, or protein) sequences or two or more nucleic acid (nucleotide, polynucleotide) sequences, as determined by comparing the sequences. In the art, "identity" also means the degree of sequence relatedness between amino acid or nucleotide sequences, as the case may be, as determined by the match between strings of such sequences.

Within the present invention, sequence identity with a particular sequence preferably means sequence identity over the entire length of said particular polypeptide or polynucleotide sequence. Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSUM62 from Hentikoffand Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps).

Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons. Moderate conditions are herein defined as conditions that allow a nucleic acid sequences of at least 50 nucleotides, preferably of about 200 or more nucleotides, to hybridize at a temperature of about 45° C in a solution comprising about 1 M salt, preferably 6×SSC or any other solution having a comparable ionic strength, and washing at room temperature in a solution comprising about 1 M salt, preferably 6×SSC or any other solution having a comparable ionic strength. Preferably, the hybridization is performed overnight, i.e. at least for 10 hours, and preferably washing is performed for at least one hour with at least two changes of the washing solution. These conditions will usually allow the specific hybridization of sequences having up to 50% sequence identity. The person skilled in the art will be able to modify these hybridization conditions in order to specifically identify sequences varying in identity between 50% and 90%.

In this document and in its claims, the verb "to comprise" and its conjugations is used in its non-limiting sense to mean that items following the word are included, but items not specifically mentioned are not excluded. In addition, the verb "to consist" may be replaced by "to essentially consist of" meaning that a composition, medium and/or substrate as described herein may comprise additional components) than the ones specifically identified, said additional components) not altering the unique characteristic of the invention. In addition, reference to an element by the indefinite article "a" or "an" or the definite article "the" does not exclude the possibility that more than one of the element is present, unless the context clearly requires that there be one and only one of the elements. The indefinite article "a" or "an" and the definite article 'the" thus usually mean "at least one".

The word "about" or "approximately" when used in association with a numerical value (e.g. about 10) preferably means that the value may be the given value (of 10) more or less 10% of the value. The sequence information as provided herein should not be so narrowly construed as to require inclusion of erroneously identified bases. The skilled person is capable of identifying such erroneously identified bases and knows how to correct for such errors. In case of sequence errors, the sequence of the enzymes obtainable by expression of the nucleotide sequences as represented by SEQ ID NO's 2, 4, 6, 7, 8, 9, 10, 11, 12, 13, 14 and 15 should prevail.

Each nucleotide sequence or amino acid sequence described herein by virtue of its identity percentage with a given nucleotide sequence or amino acid sequence respectively has in a further preferred embodiment an identity of at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% with the given nucleotide or amino acid sequence, respectively.

All patent and literature references cited in the present specification are hereby incorporated by reference in their entirety.

### Examples

### Example 1 - Production of purified recombinant VdhA, VhyA and MhdA

Full-length *vdhA, vhyA* and *mhdA* were synthesized based on the reference A. *niger* sequence (NRRL3_3887, NRRL3_9897 and NRRL3_10111, respectively) in pET23a containing a C-terminal hexa His-tag (Genscript Biotech, Leiden, the Netherlands) and were transformed in the E. *coli* protein production strain BL-21 DE3 (New England Biolabs, Ipswich, MA).

*E. coli* BL-21 DE3 pET23b-*vdhA*, pET23b-*vhyA* and pET23b-*mhdA* were grown in LB medium supplemented with 50 µg/mL ampicillin at 37 °C, 160 rpm, until an OD600 of 0.6-0.8 was reached. After incubation, 100 µM IPTG was added to the culture, which was further incubated for 24 hours at 12°C, 160 rpm. The culture was centrifuged at 3.200 x g, at 4 °C for 10 min. The pellet was dissolved in 5 mL (per 100 mL culture) BugBuster Protein Extraction Reagent (Novagen), containing 1 KU Lysozyme/ml (Sigma-Aldrich), 25 U Benzonase^{®} Nuclease and complete^{™}, EDTA-free Protease Inhibitor Cocktail and was incubated for 20 min at 4°C, shaking. The cell debris was centrifuged at 4 °C and the supernatant containing the soluble fraction of proteins was loaded to a HisTrap FF 1 mL column coupled with the ÄKTA start system (GE Healthcare Life Sciences, Uppsala, Sweden). The column was equilibrated with a buffer containing 20 mM HEPES, 0.4 M NaCl, and 20 mM imidazole, pH 7.5, at a flow rate of 1 mL/min. After washing with the equilibrating buffer, the protein was eluted with 5 mL of 20 mM HEPES, 0.4 M NaCl, and 400 mM imidazole, pH 7.5. Fractions containing the enzymes, confirmed by SDS-PAGE, were pooled, concentrated, and buffer-exchanged to 20 mM HEPES, pH 7.0. All purification steps were performed at 4°C. After purification, 0.5 mM FAD was added to VhyA.

### Example 2 - Enzyme assay of VdhA, VhyA and MhdA

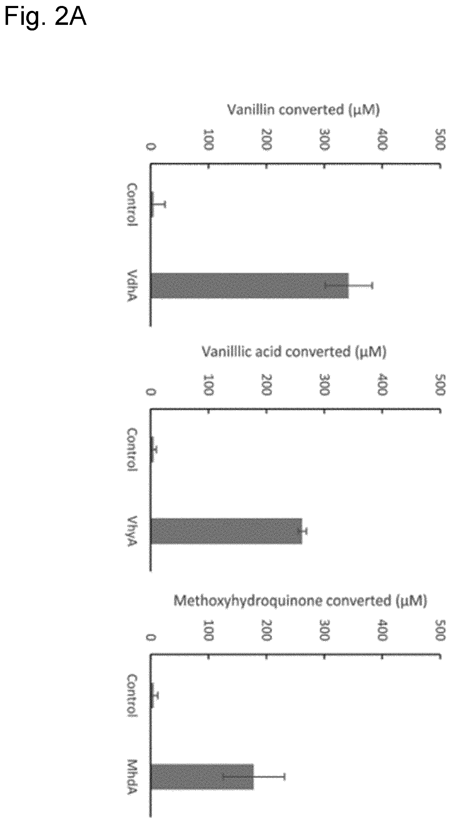

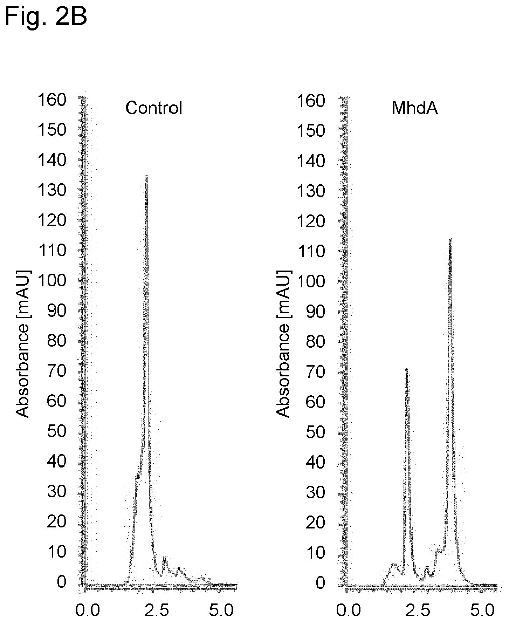

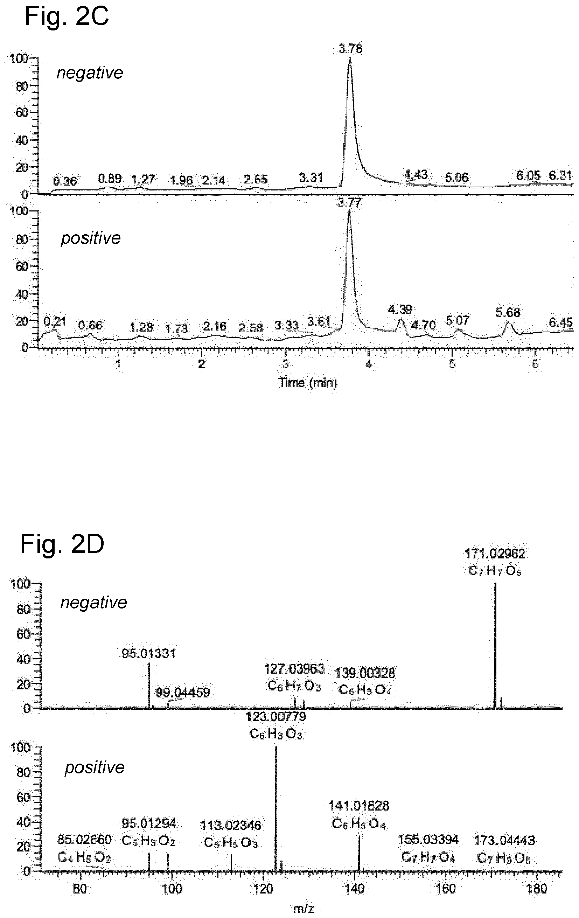

Purified enzymes as obtained in Example 1 were used for the assays. The reaction mixture for VdhA contained McIlvaine buffer, pH 7.0, consisting of 0.1 M citric acid and 0.2 M phosphate buffer, 500 µM vanillin, 500 µM NAD⁺ and 5 µL purified VdhA-His. The reaction mixture for VhyA contained Mcllvaine buffer, pH 7.0, consisting of 0.1 M citric acid and 0.2 M phosphate buffer, 500 µM vanillic acid, 500 µM NADH and 5 µL purified VhyA-His. The reaction mixture for MhdA contained 25 µM HEPES buffer, pH 6.0, 500 µM methoxyhydroquinone, 50 µM FeSO₄ and 5 µL purified MhdA-His. All reactions were incubated at 30 °C for 1 hour and product formation was analyzed by HPLC. All enzymes had activity on their corresponding substrate (Figure 2a). The mixture containing MhdA-His was reduced in methoxyhydroquinone and the formation of a compound was detected. The formed compound was identified as 4-hydroxy-6-methoxy-6-oxohexa-2,4-dienoic acid with LC-SQMS and Orbitrap (Figure 2b). Here, all his-tags were hiss-tags.

### Example 3 - Construction of AVdhA, ΔVhyA and ΔMhdA Aspergillus niger strains

*A. niger* strains used in this example are shown in Table 1. All strains generated herein have been deposited in the Centraalbureau Voor Schimmelcultures collection (CBS) of the Westerdijk Fungal Biodiversity Institute, The Netherlands. Their unique identification numbers are shown in Table 1.

**Table 1. Strains used in this example**

| **Strains** | **CBS number** | **Genotype** | **Reference** |
|---|---|---|---|
| N402 | CBS 141247 | *cspA1* | (Bos et al. 1988) |
| N593 *ΔkusA* | CBS 138852 | *cspA1*, *pyrG, ΔkusA::amdS* | (Meyer et al. 2007) |
| Reference | CBS 145984 | *cspA1*, *pyrG,* Δ*kusA::amdS,* Δ*pyrG::pyrG* | This example |
| *ΔvdhA* | CBS 145978 | *cspA1*, *pyrG, ΔkusA::amdS, ΔvdhA::pyrG* | This example |
| *ΔvhyA* | CBS 145979 | *cspA1*, *pyrG, ΔkusA::amdS, ΔvhyA::pyrG* | This example |
| *ΔmhdA* | CBS 145981 | *cspA1*, *pyrG, ΔkusA::amdS, ΔmhdA::pyrG* | This example |
| *ΔomeA* | CBS 145980 | *cspA1*, *pyrG, ΔkusA::amdS, ΔomeA::pyrG* | This example |

All deletion strains were made through protoplast-mediated transformation of *A*. *niger* N593 *ΔkusA* by homologous recombination using deletion cassettes containing 900-1000 bp upstream and downstream of the target gene fused to an orotidine 5'-phosphate decarboxylase (*pyrG*) from *Aspergillus oryzae* RIB40. The protoplast-mediated transformation and isolation of the selected transformants was performed as previously described (Kowalczyk et al. 2017). For protoplast preparation, young mycelia from overnight culture were harvested by vacuum filtration, washed with 0.6 M MgSO₄ and dried between two sheets of paper. The mycelium was then dissolved in PS buffer (0.2 M sodium phosphate buffer, 0.8 M L-sorbitol, pH 6) containing VinoTaste^{®} Pro lysing enzyme (0.5 g enzyme/g of mycelia) and incubated in a rotary shaker at an agitation speed of 100 rpm. When protoplasts were abundantly present, the mixture was filtrated through glass wool and undigested mycelia debris was removed. The protoplasts were collected by centrifugation (10 min, 1811x g, 4 °C), washed twice with ice-cold SC solution (182.2 g L⁻¹ sorbitol, 7.35 g L⁻¹ CaCl₂* 2H₂O) and resuspended in SC to a final concentration of 2 *10⁷ protoplasts/mL. For transformation, 200 µL of fresh protoplast suspension, 20 µL of 0.4 M ATA (aurintricarboxylic acid ammonium salt), 100 µL of 20% PEG-4000 was mixed with 5 µg of deletion cassette DNA and incubated for 10 min. After addition of 1.5 mL 60% PEG-4000 the mixture was incubated for 20 min. Next, 5 mL of 1.2 M sorbitol solution was added and the mixture was incubated for another 10 min. Transformed protoplasts were collected by centrifugation (10 min, 3220 ×g), resuspended in 1 mL 1.2 M sorbitol and were spread evenly over selective plates using a cell spreader. Growing colonies were observed after 4 days. Genomic DNA of two independent transformants per strain was isolated using standard phenol/chloroform extraction. Homologous integrations of the deletion cassette were verified by Southern blotting.

### Example 4 - ΔVdhA, ΔVhyA and ΔMhdA Aspergillus niger strains demonstrate reduced growth on vanillin and its metabolites

The fungi were grown on complete medium (CM, 2% (mass/vol) fructose, 6 g/L NaNO₃, 1.5g g/L KH₂PO₄, 0.5 g/L KCI, 0.5 g/L MgSO₄*7H₂O, 0.2 mL/L trace elements (stock solution containing: 50 g/L ethylenediamine tetra-acetic acid, 22 g/L ZnSO₄*7H₂O, 5.54 g/L CaCl₂, 5.06 g/L MnCl₂*4H₂O, 4.99 g/L FeSO₄*7H₂O, 1.10 g/L (NH₄)₆Mo₇O₂₄*4H₂O, 1.57 g/L CuSO₄*5H₂O, 1.61 g/L CoCl₂*6H₂O), 0.2% (mass/vol) tryptone, 0.1% (mass/vol) yeast extract, 0.1% (mass/vol) casamino acids, 0.05% (mass/vol) yeast RNA) agar (1.5 % w/v) plates at 30 °C for 4 days.

Spores were harvested with 10 mL *N*-(2-Acetamido)-2-aminoethanesulfonic acid buffer and minimal medium (MM, 6 g/L NaNO₃, 1.5g g/L KH₂PO₄, 0.5 g/L KCI, 0.5 g/L MgSO₄*7H₂O, 0.2 mL/L trace elements (stock solution containing: 50 g/L ethylenediamine tetra-acetic acid, 22 g/L ZnSO₄*7H₂O, 5.54 g/L CaCl₂, 5.06 g/L MnCl₂*4H₂O, 4.99 g/L FeSO₄*7H₂O, 1.10 g/L (NH₄)6Mο₇O₂₄*4H₂O, 1.57 g/L CuSO₄*5H₂O, 1.61 g/L CoCl₂*6H₂O)) agar (1.5% w/v) plates were inoculated with 10³ freshly isolated spores. MM plates for growth experiments were supplemented with aromatic compounds as sole carbon source. In the case of benzoic acid, ferulic acid and vanillin 2 mM was used while 5 mM was used for the remaining aromatic compounds. All aromatic compounds and chemicals were purchased from Sigma Aldrich. Agar plates were incubated at 30 °C for 10 days.

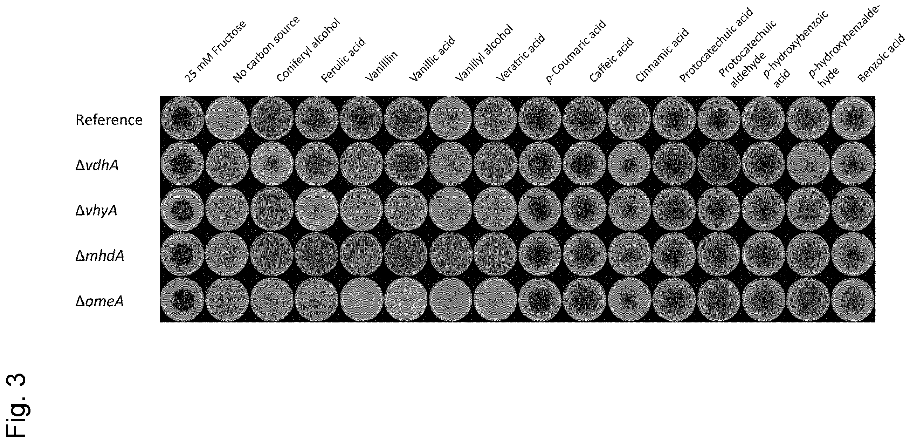

The deletion mutants were screened for their phenotypes on growth on 14 aromatic compounds (Figure 3). Deletion of *vdhA* resulted in abolished growth on vanillin, showing that this gene is involved in the conversion of vanillin. In addition, reduced growth on p-hydroxybenzaldehyde and protocatechuic aldehyde was observed. Deletion of *vhyA, mhdA* and *OmeA* resulted in reduced growth on coniferyl alcohol, ferulic acid and vanillic acid and abolished growth on vanillin. This demonstrates that *vhyA, mhdA* and *OmeA* are involved in the relevant metabolic pathway. In addition, deletion of *mhdA* resulted in a red-brownish coloration of the medium when grown on ferulic acid and vanillic acid which demonstrates that methoxyhydroquinone is accumulated. After 10 days of growth, *ΔmhdA* appears to have a slightly reduced growth and spore formation on vanillyl alcohol and veratric acid demonstrating that both compounds are converted towards methoxyhydroquinone. In addition, light red-brownish coloration in the media was observed when grown on vanillyl alcohol. Deletion of VhyA, MhdA or OmeA did not result in a phenotype on benzoic acid, caffeic acid, cinnamic acid, *p*-coumaric acid, *p*-hydroxybenzoic acid, *p*-hydroxybenzaldehyde, protocatechuic aldehyde or protocatechuic acid. Deletion of OmeA resulted in accumulation of 6-methoxy-4,6-dioxohexanoic acid (4-oxo-monomethyl adipate).

### Example 5 - ΔVdhA, ΔVhyA and ΔMhdA Aspergillus niger strains accumulate 1-substituted 4-hydroxy-3-methoxybenzenes in the media

*A. niger* spores (1 x 10⁶ spores/mL) were inoculated in shake flasks containing 200 mL of complete medium with 2% fructose and incubated overnight in a rotary shaker at 30 °C and 250 rpm. Freshly germinated mycelia were harvested on Miracloth (Merck KGaA, Darmstadt, Germany) and washed with minimal medium. Equal portions of mycelia were transferred to flasks containing 50 mL minimal medium and 5 mM coniferyl alcohol, ferulic acid, vanillyl alcohol, vanillin, vanillic acid, veratryl alcohol, veratric aldehyde or veratric acid. The cultures were incubated on a rotary shaker for 24 hours at 30°C, 250 rpm. Supernatant samples were collected after 24 hours and diluted 50 times with acetonitrile before HPLC analysis using the setup as described previously (Dilokpimol et al. 2017).

An HPLC (Dionex ICS-5000+ chromatography system; Thermo Scientific, Sunnyvale, CA) equipped with an Acclaim Mixed-Mode WAX-1 LC Column (3×150 mm; Thermo Scientific) and a UV detector (225 nm; Thermo Scientific) was used. The chromatographic separation was carried out according to manufacturer's recommendation using isocratic elution (30 °C; flow rate: 0.25 mL/min) with 25 mM potassium monophosphate, 0.8 mM pyrophosphate, pH 6.0, in 50% acetonitrile. Ferulic and p-coumaric acids (0.25-50 µM) were used as standards for identification and quantification.

The results are shown in Table 2. After 24 hours of incubation, no coniferyl alcohol, ferulic acid or vanillic acid was detected in the reference strain supernatant samples, which indicates that these compounds are fully consumed. Veratric acid and veratryl alcohol appears to be slowly consumed compared to the other tested substrates.

Deletion of *vdhA* did not result in the accumulation of vanillin when grown on coniferyl alcohol or ferulic acid. This indicated that coniferyl alcohol and ferulic acid are not converted to vanillin. On vanillin, almost no conversion was observed, however small amount of vanillyl alcohol was detected. This indicated that vanillin can be converted to vanillyl alcohol. On vanillyl alcohol, only trace amounts of vanillin were detected.

Deletion of *vhyA* resulted in the accumulation of vanillic acid in the medium when the *ΔvhyA* strain was cultured on coniferyl alcohol, ferulic acid, vanillin, vanillyl alcohol, vanillic acid, veratryl alcohol, veratric aldehyde and veratric acid. Similar results were observed for deletion of *mhdA* which resulted in the accumulation of methoxyhydroquinone in the medium when the *ΔmhdA* strain was cultured on these compounds.

The results demonstrate that disruption of enzyme expression according to the invention results in the accumulation of useful 1-substituted 4-hydroxy-3-methoxybenzenes in the media.

**Table 2. Accumulation of vanillin, vanillic acid and methoxyhydroquinone using the ΔvdhA, ΔvhyA and ΔmhdA deletion mutants.**

| | | **Reference** | Δ***vdhA*** | Δ***vhyA*** | Δ***mhdA*** |
|---|---|---|---|---|---|
| **Starting substrate** | **Detected products** | **Conc. (mM)** | **Conc. (mM)** | **Conc. (mM)** | **Conc. (mM)** |
| **Coniferyl alcohol** | Coniferyl alcohol | - | - | - | |
| | Vanillic acid | - | - | 478 ± 0.07 | |
| | MHQ | - | - | - | 3.67 ± 0.14 |
| **Ferulic acid** | Ferulic acid | - | - | - | - |
| | Vanillic acid | - | - | 4.80 ± 0.09 | - |
| | MHQ | - | - | - | 3.93 ± 0.12 |
| **Vanillyl** | Vanillyl alcohol | 1.79 ±0.37 | 1.24 ± 0.25 | 2.13 ±0.15 | 2.75 ± 0.14 |
| **alcohol** | Vanillin | - | 0.08 ± 0.04 | - | - |
| | Vanillic acid | - | - | 2.80 ± 0.06 | - |
| | MHQ | - | - | - | 3.13 ± 0.13 |
| **Vanillin** | Vanillin | - | 4.02 ±0.13 | 2.98 ± 0.75 | 0.29 ± 0.37 |
| | Vanillyl alcohol | 0.23 ±0.05 | 0.64 ± 0.17 | 0.48 ± 0.09 | - |
| | Vanillic acid | - | - | 1.58 ± 0.71 | 0.07 ± 0.00 |
| | MHQ | - | - | - | 3.39 ± 1.28 |
| **Vanillic acid** | Vanillic acid | - | - | 4.82 ± 0.19 | 0.80 ± 0.89 |
| | MHQ | - | - | - | 4.04 ± 0.41 |
| **Veratryl alcohol** | Veratryl alcohol | 3.83 ± 0.34 | 3.73 | 4.04 ±0.18 | 3.76 ± 0.44 |
| | Veratric acid | 0.09 ± 0.02 | 0.65 | - | 0.16 ± 0.07 |
| | Vanillic acid | | - | 1.11 ± 0.05 | - |
| | MHQ | | - | - | 2.11 ± 0.32 |
| **Veratric aldehyde** | Veratric aldehyde | 0.62 ± 0.02 | 2.00 ± 0.19 | 0.76 ± 0.00 | 0.75 ± 0.05 |
| | Veratric acid | 0.17 ± 0.13 | 0.40 ± 0.04 | - | 0.39 ± 0.13 |
| | Vanillic acid | - | - | 3.48 ± 0.04 | |
| | MHQ | - | - | - | 3.27 ± 0.30 |
| **Veratric acid** | Veratric acid | 3.95 ± 1.06 | 4.27 ± 1.28 | 3.87 ± 1.14 | 1.51 ± 0.71 |
| | Vanillic acid | - | - | 2.39 ± 0.95 | - |
| | MHQ | - | - | - | 2.43 ± 0.54 |

| | | | | | |
|---|---|---|---|---|---|
| MHQ, Methoxyhydroquinone | | | | | |

### References

1. Kaur B, Chakraborty D (2013) Biotechnological and molecular approaches for vanillin production: A review. Appl Biochem Biotechnol 169:1353-1372
2. Chow KT, Pope MK, Davies J (1999) Characterization of a vanillic acid non-oxidative decarboxylation gene cluster from Streptomyces sp. D7. Microbiology 145:2393-2403
3. Rosini E, D'Arrigo P, Pollegioni L (2016) Demethylation of vanillic acid by recombinant LigM in a one-pot cofactor regeneration system. Catal Sci Technol 6:7729-7737
4. El-Mansi EMT, Anderson SCK (2004) The hydroxylation of vanillate and its conversion to methoxyhydroquinone by a strain of Pseudomonas fluorescens devoid of demethylase and methylhydroxylase activities. World J Microbiol Biotechnol 20:827-832
5. Guiraud P, Steiman R, Seigle-Murandi F, Benoit-Guyod JL (1992) Metabolism of vanillic acid by Micromycetes. World J Microbiol Biotechnol 8:270-275
6. Yajima Y, Enoki A, Mayfield MB, Gold MH (1979) Vanillate Hydroxylase from the White Rot Basidiomycete Phanerochaete chrysosporium
7. Busswell JA, Eriksson K-E, Petterson B (1981) Purification and partial characterization of vanillate hydroxylase(decarboxylating) from Sporotrichum pulverulentum. J Chromatogr 215:99-108
8. Baqueiro-Peña I, Rodriguez-Serrano G, Gonzalez-Zamora E, Augur C, Loera O, Saucedo-Castañeda G (2010) Biotransformation of ferulic acid to 4-vinylguaiacol by a wild and a diploid strain of Aspergillus niger. Bioresour Technol 101:4721-4724
9. Lubbers RJM, Liwanag AJ, Peng M, Dilokpimol A, Benoit-Gelber I, de Vries RP (2020) Evolutionary adaptation of Aspergillus niger for increased ferulic acid tolerance. J Appl Microbiol 128:735-746. https://doi.org/10.1111/jam.14505
10. Lesage-Meessen L, Delattre M, Haon M, Thibault JF, Ceccaldi BC, Brunerie P, Asther M (1996) A two-step bioconversion process for vanillin production from ferulic acid combining Aspergillus niger and Pycnoporus cinnabarinus. J Biotechnol 50:107-113
11. Dilokpimol A, Mäkelä MR, Mansouri S, Belova O, Waterstraat M, Bunzel M, de Vries RP, Hilden KS (2017) Expanding the feruloyl esterase gene family of Aspergillus niger by characterization of a feruloyl esterase, FaeC. N Biotechnol 37:200-209
12. Kowalczyk JE, Lubbers RJM, Peng M, Battaglia E, Visser J, De Vries RP (2017) Combinatorial control of gene expression in Aspergillus niger grown on sugar beet pectin. Sci Rep 7:1-12
13. Nødvig CS, Nielsen JB, Kogle ME, Mortensen UH (2015) A CRISPR-Cas9 System for genetic engineering of filamentous fungi. PLOS ONE 10(7): e0133085
14. Florea S, Andreeva K, Machado C, Mirabito PM, Schardl CL (2009) Elimination of marker genes from transformed filamentous fungi by unselected transient transfection with a Cre-expressing plasmid. Fung Gen Biol 46(10):721-730
15. Bos CJ, Debets AJM, Swart K, Huybers A, Kobus G, Slakhorst SM (1988) Genetic analysis and the construction of master strains for assignment of genes to six linkage groups in Aspergillus niger. Curr Genet 14:437-443
16. Meyer V, Arentshorst M, El-Ghezal A, Drews AC, Kooistra R, van den Hondel CAMJJ, Ram AFJ (2007) Highly efficient gene targeting in the Aspergillus niger kusA mutant. J Biotechnol 128:770-775

Sequences referred to in this document, the first column referring to the SEQ ID NO.

| | **Description** | **Sequence** |
|---|---|---|
| 1 | VdhA enzyme | See *sequence listing* |
| 2 | VdhA nucleic acid | See *sequence listing* |
| 3 | VhyA enzyme | See *sequence listing* |
| 4 | VhyA nucleic acid | See *sequence listing* |
| 5 | MhdA enzyme | See *sequence listing* |
| 6 | MhdA nucleic acid | See *sequence listing* |
| 7 | VdhA vector (codon opt Vdh) | See *sequence listing* |
| 8 | VhyA vector (codon opt Vhy) | See *sequence listing* |
| 9 | MhdA vector (codon opt Mhd) | See *sequence listing* |
| 10 | VdhA nucleic acid (codon opt) | See *sequence listing* |
| 11 | VhyA nucleic acid (codon opt) | See *sequence listing* |
| 12 | MhdA nucleic acid (codon opt) | See *sequence listing* |
| 13 | deletion cassette CBS 145984 used to delete pyrG | See *sequence listing* |
| 14 | deletion cassette CBS 145978 used to delete vdhA | See *sequence listing* |
| 15 | deletion cassette CBS 145979 used to delete vhyA | See *sequence listing* |
| 16 | deletion cassette CBS 145981 used to delete mhdA | See *sequence listing* |
| 17 | pyrG-5'F | CGAACTACTTTCGGCATCTTCTAG |
| 18 | pyrG-5'R-pyrg | GCACTTACCTTCGCATTTTCTGGTATATTC GTAAATCCAGAGACGTGCG |
| 19 | pyrG-3'F-pyrg | CCTGTGTTGGTTCTCAGGAACTGCGAATA TGGATACGGAAGCGGAAACG |
| 20 | pyrG-3'R | CCGCCATGTTAGATCTTGGC |
| 21 | pyrG Forward verification | CGCAAGTTCATTGACATCGGC |
| 22 | pyrG Reverse verification | GGAAGAAATGTTCACACCAGTCG |
| 23 | VdhA-5'F | GCACAGCAGGTATCAGAGAGAG |
| 24 | VdhA-5'R-pyrg | GCACTTACCTTCGCATTTTCTGGTATATTT CCAAAGGTTCTGACAAAGAGGT |
| 25 | VdhA-3'F-pyrg | CCTGTGTTGGTTCTCAGGAACTGCGAATA TCGTTCCTTCTTCAACATGATTGGG |
| 26 | VdhA-3'R | GCTTCTAGCCAACACCATGC |
| 27 | VdhA-Forward verification | CGAAACATGATTGATCCTGCAGA |
| 28 | VdhA-Reverse verification | GGCTAGATTCAGTAGTAAACGTCG |
| 29 | VhyA-5'F | GCACAGCAGGTATCAGAGAGAG |
| 30 | VhyA-5'R-pyrg | GCACTTACCTTCGCATTTTCTGGTATATTT CCAAAGGTTCTGACAAAGAGGT |
| 31 | VhyA-3'F-pyrg | CCTGTGTTGGTTCTCAGGAACTGCGAATA TCGTTCCTTCTTCAACATGATTGGG |
| 32 | VhyA-3'R | GCTTCTAGCCAACACCATGC |
| 33 | VhyA-Forward verification | GCGAGGACATCATGTTACCA |
| 34 | VhyA-Reverse verification | CAAATCATGGTTGCGCACTG |
| 35 | MhdA-5'F | CCTGCTATTCTTGTTTGACGG |
| 36 | MhdA-5'R-pyrg | GCACTTACCTTCGCATTTTCTGGTATATT GCATGATGGGTGATAGGTCTCAG |
| 37 | MhdA-3'F-pyrg | CCTGTGTTGGTTCTCAGGAACTGCGAATA TCCTTGAGCTTAGCCATTCCTCC |
| 38 | MhdA-3'R | GGTAACCAAGCCGCAATTATGG |
| 39 | MhdA-Forward verification | GCCGACCCCAAAGAATACC |
| 40 | MhdA-Reverse verification | TCTCAAGGATGTAACCACGAG |
| 41 | OmeA enzyme | See *sequence listing* |
| 42 | OmeA nucleic acid | See *sequence listing* |
| 43 | OmeA nucleic acid (codon opt) | See *sequence listing* |
| 44 | OmeA-5'F | GTGGAGGAAAAGGAGGCACG |
| 45 | OmeA-5'R-pyrg | GCACTTACCTTCGCATTTTCTGGTATATTT GCTGTATCTGCGGAGATGC |
| 46 | OmeA-3'F-pyrg | CCTGTGTTGGTTCTCAGGAACTGCGAATA TGCCACACTGAATACAGGAATCTCC |
| 47 | OmeA-3'R | CATCAGCCAGTTGAATCGCT |
| 48 | OmeA-Verification-F | CATCTCCGCAGATACAGCA |
| 49 | OmeA-Verification-R | GACGGCCCTCTTGACTG |

## Claims

1. A host cell capable of expressing at least one of:
i) a functional enzyme with vanillin dehydrogenase (Vdh) activity; and
ii) a functional enzyme with vanillate hydroxylase (Vhy) activity;
wherein the host cell has reduced or no expression of a functional enzyme with methoxyhydroquinone 4,5-dioxygenase (Mhd) activity.

2. The host cell according to claim 1, wherein the host cell is a microbial cell, preferably a fungal cell, more preferably a filamentous fungal cell.

3. The cell according to claim 1 or 2, wherein:
- the enzyme with Vdh activity is heterologous, preferably VdhA from *Aspergillus niger;* and/or
- the enzyme with Vhy activity is heterologous, preferably VhyA from *Aspergillus niger;* and/or
- the enzyme with Mhd activity is heterologous, preferably MhdA from *Aspergillus niger.*

4. The cell according to any one of claims 1-3, wherein:
- the enzyme with Vdh activity has at least 60%, preferably at least 80%, more preferably at least 90%, most preferably 100% sequence identity with SEQ ID NO: 1, or is encoded by a nucleotide sequence which has at least 60%, preferably at least 80%, more preferably at least 90%, most preferably 100% sequence identity with SEQ ID NO: 2;
- the enzyme with Vhy activity has at least 60%, preferably at least 80%, more preferably at least 90%, most preferably 100% sequence identity with SEQ ID NO: 3, or is encoded by a nucleotide sequence which has at least 60%, preferably at least 80%, more preferably at least 90%, most preferably 100% sequence identity with SEQ ID NO: 4;
- the enzyme wit Mhd activity has at least 60%, preferably at least 80%, more preferably at least 90%, most preferably 100% sequence identity with SEQ ID NO: 5, or is encoded by a nucleotide sequence which has at least 60%, preferably at least 80%, more preferably at least 90%, most preferably 100% sequence identity with SEQ ID NO: 6.

5. The cell according to any one of claims 1-4, wherein the nucleotide sequence encoding at least one of said enzymes is codon optimized.

6. The cell according to any one of claims 1-5, wherein the cell is capable of expressing a functional enzyme with vanillin dehydrogenase (Vdh) activity and has reduced or no expression of a functional enzyme with vanillate hydroxylase (Vhy) activity and has reduced or no expression of a functional enzyme with methoxyhydroquinone 1,2-dioxygenase (Mhd) activity.

7. The cell according to any one of claims 1-5, wherein the cell is capable of expressing a functional enzyme with vanillin dehydrogenase (Vdh) activity and is capable of expressing a functional enzyme with vanillate hydroxylase (Vhy) activity and has reduced or no expression of a functional enzyme with methoxyhydroquinone 1,2-dioxygenase (Mhd) activity.

8. The cell according to any one of claims 1-7, capable of producing at least 1.0 mM of a 1-substituted 4-hydroxy-3-methoxybenzene.

9. The cell according to claim 8, wherein the a 1-substituted 4-hydroxy-3-methoxybenzene is selected from vanillin, vanillic acid, and methoxyhydroquinone, preferably selected from vanillic acid and methoxyhydroquinone.

10. A method for the production of a 1-substituted 4-hydroxy-3-methoxybenzene, comprising the steps of:
- culturing a cell according to any one of claims 1-9 under conditions conducive to the production of a 1-substituted 4-hydroxy-3-methoxybenzene, and, optionally,
- isolating and/or purifying the 1-substituted 4-hydroxy-3-methoxybenzene from the cell and/or the culture medium.

11. The method according to claim 10, wherein the method is for the production of vanillic acid, and wherein the cell is a cell according to claim 6.

12. The method according to claim 10, wherein the method is for the production of methoxyhydroquinone, and wherein the cell is a cell according to claim 7.

13. An expression vector comprising a first nucleotide sequence that has at least 60%, 65%, 70%, 75%, 80%, 85%, 90%, 95%, 96%, 97%, 98%, 99%, or 100% sequence identity with SEQ ID NO: 6 or 12, or wherein the expression vector consists of a polynucleotide that has at least 60%, 65%, 70%, 75%, 80%, 85%, 90%, 95%, 96%, 97%, 98%, 99%, or 100% sequence identity with SEQ ID NO: 9.

14. A polypeptide product expressed from the expression vector according to claim 13.

15. Use of a functional enzyme with Mhd activity as specified in any one of claims 1, 3, or 4, for the production of 4-hydroxy-6-methoxy-6-oxohexa-2,4-dienoic acid.


SEQUENCE LISTING

<110>  Koninklijke Nederlandse Akademie van Wetenschappen

<120>  New enzymes and methods for vanillic acid-related metabolic
       pathways

<130>  P6095867EP

<160>  50

<170>  PatentIn version 3.5

<210>  1
<211>  478
<212>  PRT
<213>  Aspergillus niger

<400>  1

Met Val Thr Thr Thr Ala Ile Pro Leu Ile Ile His Gly Lys Asp Val
1               5                   10                  15

Leu Ile Ser Asn Ser Asp Arg Lys His Pro Ile Thr Ser Thr Ser Ala
            20                  25                  30

Pro His Leu Lys Ser Phe Gln Gly Ala Thr Pro Asp Leu Ala Ile Gln
        35                  40                  45

Ala Val Glu Ser Cys Ala Ser Ala Phe Pro Thr Trp Ser Gln Thr Pro
    50                  55                  60

Pro Glu Glu Arg Arg Arg Leu Leu Leu Asn Leu Ala Lys Leu Leu Arg
65                  70                  75                  80

Glu Lys Ser Asn Thr Ile Gln Thr Leu Ile Lys Asp Glu Ile His Cys
                85                  90                  95

Thr Pro Leu Trp Ser His Leu Asn Val Glu Ile Ala Ala Gly Ile Val
            100                 105                 110

Glu Glu Thr Ala Ser Val Ile Thr Asp Ala Ile Ser Gly Thr Ile Pro
        115                 120                 125

Ser Ser Gln Gly Asn Thr Tyr Ala Leu Val Met Lys Lys Pro Leu Gly
    130                 135                 140

Val Val Leu Gly Ile Ala Pro Trp Asn Ala Pro Val Ile Leu Gly Leu
145                 150                 155                 160

Arg Ser Val Ile Pro Val Ile Ala Ala Gly Asn Thr Ala Ile Leu Lys
                165                 170                 175

Gly Ser Glu Leu Ser Pro Gln Thr His Tyr Phe Leu Ala Ala Leu Phe
        180                 185                 190

Arg Glu Ala Gly Phe Pro Pro Gly Val Ile Asn Phe Val Leu His Arg
    195                 200                 205

Pro Gln Asp Ala Ala Glu Ile Tyr Glu Cys Met Ile Gln His Pro Ala
    210                 215                 220

Val Lys Lys Cys Asn Phe Thr Gly Ser Thr Gln Val Gly Arg Val Ile
225                 230                 235                 240

Ala Ser Lys Ala Ala Met Ala Leu Lys Pro Val Leu Leu Glu Leu Gly
                245                 250                 255

Gly Lys Asn Pro Val Val Val Leu Glu Asp Ala Asp Leu Asp Gln Ala
            260                 265                 270

Ala Asp Glu Ile Val Ala Ala Gly Tyr Leu Asn Asn Gly Gln Ile Cys
        275                 280                 285

Met Ser Thr Asp Leu Val Tyr Val Val Arg Ser Val Ala Glu Glu Leu
    290                 295                 300

Thr Lys Lys Leu Leu Glu Arg Leu His Ser Glu Asp Arg Pro His Lys
305                 310                 315                 320

Val Ile Ser Ala Ala Ser Arg Ala Arg Leu Thr Gln Leu Val Asp Asp
                325                 330                 335

Ala Arg Ser Lys Gly Gly Val Ile His Gln Ala Arg Asn Pro Leu Ala
            340                 345                 350

Glu Asp Pro Leu Ala Phe Pro Ala Thr Val Ile Glu Gly Val Thr Glu
        355                 360                 365

Glu Met Glu Phe Tyr Thr Ile Glu Ser Phe Gly Pro Val Phe Gly Ile
    370                 375                 380

Ile Ala Val Asp Ser Ala Glu Lys Gly Leu Gln Met Val Lys Arg Ser
385                 390                 395                 400

Ala Tyr Gly Leu Ser Ser Ala Val Phe Thr Arg Asp His Phe Lys Ala
                405                 410                 415

Leu Glu Met Ser Ala Asp Ile Ala Val Gly Ala Val His Val Asn Gly
            420                 425                 430

Gly Thr Val His Asp Glu Pro Thr Leu Pro His Gly Gly Val Gly Asp
        435                 440                 445

Ser Gly Tyr Gly Arg Phe Gly Gly Lys Trp Gly Leu Asn Glu Phe Leu
    450                 455                 460

Gln Thr Gln Thr Val Ile Leu Asn Arg Val Arg Ala Ser Leu
465                 470                 475

<210>  2
<211>  1437
<212>  DNA
<213>  Aspergillus niger

<400>  2
atggtcacca caaccgcaat tcccctgata atccacggca aggatgtcct tatctccaat      60

agtgaccgca aacatcccat cacgtctaca tccgcacctc atttgaaatc cttccaaggc     120

gcaacgcccg atctcgccat ccaagccgtc gaatcctgcg caagtgcctt cccaacatgg     180

tcgcaaacac cccccgagga acgtcgacgt ttactactga atctagccaa gctccttcgc     240

gaaaaatcca acaccattca gaccctcatc aaagatgaaa tccactgcac accactctgg     300

tctcacctca acgttgaaat cgcagccggc attgtcgaag agaccgcttc agtaataaca     360

gacgccatta gcggcactat cccttcgtcg cagggaaata cgtacgccct tgtgatgaag     420

aagccgctcg gtgtggtgct gggcatcgcg ccgtggaacg cgccggtaat tttgggactg     480

agatcggtga ttccggtaat tgcggcgggc aacacagcta tcttgaaggg ctctgagttg     540

agtccccaaa cgcattactt cctcgcggca ctgttccggg aggcagggtt cccgccagga     600

gtcattaact tcgttctgca tcggccgcag gacgcggcag agatctacga gtgtatgatt     660

cagcatccgg ctgtgaagaa atgtaacttc accggatcaa cccaggtggg acgtgtgatc     720

gcgtccaagg cggcgatggc gttgaagccg gtgctgttgg aattgggtgg caagaacccc     780

gtggtggtgt tggaggatgc ggatttggat caggcggcgg atgagattgt tgcggcggga     840

tatttgaata atggccagat ttgcatgtcg actgatctcg tctacgttgt tcgctcggtg     900

gcggaggagt tgactaagaa gcttttagag cgcttgcaca gcgaagatcg gccacacaag     960

gttatttcgg cagcgagccg agcccgtctt acgcagttgg tagatgatgc gcggtctaag    1020

gggggtgtca ttcaccaggc tcgtaaccct ctcgctgagg atcccttggc attcccagct    1080

actgtgattg aaggagtcac ggaggagatg gagttctata cgatcgagtc gtttggaccg    1140

gtgtttggaa tcatcgctgt tgattcggca gagaagggtt tgcaaatggt taagcggtcc    1200

gcgtacggcc tttcatcggc ggtgtttacg cgtgatcatt tcaaggctct ggaaatgtct    1260

gcagatattg ctgtgggagc ggttcatgtc aacgggggca cggtacatga cgaaccgacg    1320

ttaccgcatg gaggagtggg cgacagtgga tatggtcggt ttggaggaaa atggggcttg 1380

aatgagtttt tgcagacgca aacggtaatt ttgaatcggg ttcgggcttc cctctga 1437

<210> 3
<211> 430
<212> PRT
<213> Aspergillus niger

<400> 3

Met Glu Gln Ala Lys Thr Pro Leu Gln Val Ile Val Val Gly Ala Gly
1               5                   10                  15

Ile Gly Gly Met Ala Ala Ala Leu Thr Leu Gly Leu Arg Gly His His
            20                  25                  30

Val Thr Ile Leu Glu Ser Ala Pro Lys Leu Met Glu Val Gly Ala Gly
        35                  40                  45

Ile Gln Val Ser Pro Asn Met Leu Thr Leu Phe Asp Arg Trp Gly Val
    50                  55                  60

Ser Pro Leu Ile His Ala Gln Asp Val Ala Leu Glu His Ile His Val
65                  70                  75                  80

Arg Arg Trp Glu Asp Gly Ser Met Leu Gly Thr Leu Pro Val Asn Lys
                85                  90                  95

Thr Tyr Gly Gln Gln Thr Val Ile His Arg Ala Asp Leu His Asn Ala
            100                 105                 110

Leu Ile Glu Lys Ala Leu Ala Leu Ser Asn Val Glu Leu Arg Val Asn
        115                 120                 125

Ser Thr Val Thr Gly Val Gln Phe Asp His Ala Ala Val Gln Leu Ala
    130                 135                 140

Asn Gly Thr Ile Val Lys Gly Asp Ile Val Ile Ala Ala Asp Gly Ile
145                 150                 155                 160

Lys Ser Ala Leu Arg Asp His Leu Leu Glu Glu Ala Ser Val Ala Ile
                165                 170                 175

Pro Thr Gly Asp Ala Ala Tyr Arg Ile Met Leu Pro Arg Ser Ala Leu
            180                 185                 190

Glu Asn Asp Pro Glu Leu Lys Ala Leu Val Asp Glu Pro Gln Ala Thr
        195                 200                 205

Arg Trp Leu Gly Pro Gly Arg His Val Ile Ala Tyr Pro Val Arg Asn
    210                 215                 220

His Asp Leu Tyr Asn Val Val Leu Leu His Pro Asp Ser His Gly Val
225                 230                 235                 240

Glu Glu Ser Trp Thr Thr Lys Gly Ser Lys Gln Ala Met Val Asp Asn
                245                 250                 255

Tyr Lys Gly Trp Asp Arg Arg Val Thr Lys Leu Ile Asp Leu Val Pro
            260                 265                 270

Asp Asp Glu Val Leu Glu Trp Lys Leu Cys Leu His Ala Pro Leu Lys
        275                 280                 285

Thr Trp Ile Arg Gly Ser Val Ala Leu Ile Gly Asp Ala Cys His Pro
    290                 295                 300

Met Leu Pro Tyr Val Ala Gln Gly Ala Ala Gln Ala Val Glu Asp Ala
305                 310                 315                 320

Ala Ala Leu Gly Val Leu Leu Ser Thr Ile Ser Ser Arg Asp Glu Ile
                325                 330                 335

Pro Leu Ala Leu Lys Ala Tyr Glu Lys Ser Arg Lys Pro Arg Ala Asp
            340                 345                 350

Thr Val Gln Gln Ser Gly Ser Glu Asn Arg Ile Thr Leu His Leu Pro
        355                 360                 365

Asp Gly Pro Glu Gln Arg Ala Arg Asp Glu Gln Phe Arg Ser Ser Leu
    370                 375                 380

Lys Ser Gly Ser Asn Pro Asp Arg Trp Thr Asp Leu Glu Thr Gln Lys
385                 390                 395                 400

Phe Leu Trp Gly Trp Asp Ala Glu Lys Ala Ala Leu Asp Ala Trp Asn
                405                 410                 415

Glu Leu His Gly Asn His Val Ser Glu Leu Arg His His Leu
            420                 425                 430

<210>  4
<211>  1293
<212>  DNA
<213>  Aspergillus niger

<400>  4
atggagcaag caaagactcc tctgcaggtg attgtggttg gtgccggcat tggcggcatg      60

gctgcggccc tgaccctggg tctgcgagga catcatgtta ccatcctcga gtccgctccc     120

aagctcatgg aagttggtgc tggtattcaa gtctcaccga atatgctgac actgtttgac     180

cggtggggcg tctccccgtt gatccacgcc caggatgttg ccctcgagca cattcatgta     240

agacggtggg aggatggcag catgcttggc accttgccag tgaacaagac ctacggccaa     300

cagactgtca ttcaccgcgc agacctccac aacgccctca tcgaaaaggc gctggcgctg     360

tcgaatgtgg agctgcgcgt gaactcgacc gtcactggag ttcaattcga ccatgccgcg     420

gtgcaattgg ccaacggcac tattgttaaa ggagacatcg tgatcgcggc tgacggcatc     480

aagtctgccc tccgtgatca cttgctggaa gaagcatctg tcgccattcc tacgggcgac      540

gcagcctacc ggattatgct gcctcgtagt gctctagaga atgaccctga gctaaaggca     600

ctggtagatg agccacaagc cactcggtgg ctcggacctg gccgtcatgt catcgcttat     660

ccagtgcgca accatgattt gtacaatgtt gtcctgcttc atccagacag ccatggtgtc     720

gaggagtcgt ggacgaccaa gggttccaag caggccatgg tcgacaacta caagggctgg      780

gaccggcgtg tgacgaagct tatcgacctg gtaccggacg atgaagtcct ggaatggaag     840

ctgtgccttc acgctccgct gaagacctgg attaggggtt ccgtggccct catcggtgac     900

gcctgccacc caatgctgcc atatgttgct caaggtgctg cccaggccgt cgaagacgct     960

gctgccctcg gtgtcctgct ctccactatc tcttcccggg atgaaatccc tcttgcactc    1020

aaagcgtacg agaagtcccg caagcctcgc gcagacaccg tccagcaatc tgggtcggag    1080

aaccgcatca cgctccacct gcccgatggc ccggagcaac gcgcgcgtga cgaacagttc    1140

cgttcttccc tgaagagcgg atcaaacccc gaccggtgga cagacctgga aacccagaag    1200

ttcctctggg gctgggatgc tgagaaggca gctctggatg catggaatga actccatggc    1260

aaccacgtgt ccgaactgcg gcaccacctg tga                                 1293

<210>  5
<211>  488
<212>  PRT
<213>  Aspergillus niger

<400>  5

Met Pro Ser Thr Ser Asp Leu Ala Asp Pro Lys Glu Tyr Gln Lys Ile
1               5                   10                  15

Phe His Trp Ala Glu Thr Gln Lys Asp Gly Thr Ile Pro Ser Phe Ala
            20                  25                  30

Thr Arg Arg Asn Asp Pro Tyr Glu Tyr Gln Ala Gly Phe Gly Asn Ser
        35                  40                  45

Phe Ala Ser Glu Ala Val Pro Gly Thr Leu Pro Gln Gly Gln Asn Asn
    50                  55                  60

Pro Arg Asn Val Arg Phe Gly Leu Tyr Ala Glu Gln Met Thr Ala Thr
65                  70                  75                  80

Ala Phe Ile Ala Pro Arg His Cys Asn Lys Lys Ala Trp Leu Tyr Arg
                85                  90                  95

Ala Arg Pro Ala Val Ala His Gln Gly Phe Thr Asp Leu Pro Asp Asn
            100                 105                 110

Lys Asp Thr Glu Ser Asn Phe Leu Pro Leu Asn Pro Arg Val His Val
        115                 120                 125

Ser Pro Thr Gln Leu Ala Trp Tyr Pro Phe Asp Ile Pro Thr Asn Gly
    130                 135                 140

Glu Val Asp Phe Val Ser Gly Leu Lys Thr Val Ala Gly Ser Gly Glu
145                 150                 155                 160

Pro Thr Leu Arg Glu Gly Leu Ala Thr His Val Tyr Val Ala Asn Thr
                165                 170                 175

Ser Met Thr Lys Lys Ala Phe Val Asn Ser Asp Gly Glu Met Ala Ile
            180                 185                 190

Val Pro Gln Gln Gly Ala Leu Asp Ile Gln Thr Glu Phe Gly Pro Leu
        195                 200                 205

Phe Val Gln Pro Gly Glu Ile Val Ile Ile Gln Arg Gly Ile Arg Phe
    210                 215                 220

Arg Val Glu Leu Pro Asp Gly Pro Ser Arg Gly Tyr Ile Leu Glu Ile
225                 230                 235                 240

Trp Gly Ser Gln Phe Glu Leu Pro Glu Leu Gly Pro Leu Gly Ala Asn
                245                 250                 255

Gly Leu Ala Asn Ala Arg Asp Phe Leu Tyr Pro Thr Ala Lys Tyr Glu
            260                 265                 270

Ile Ser Lys Glu Pro Trp Glu Val Val Tyr Lys Leu Gly Gly Lys Phe
        275                 280                 285

Phe Lys Ser Thr Gln Asn His Ser Pro Phe Asp Val Val Ala Trp His

290                 295                 300

Gly Asn Tyr Val Pro Tyr Lys Tyr Asp Met Thr Lys Phe Val Asn Val
305                 310                 315                 320

Gly Ser Ile Ser Val Asp His Ile Asp Pro Ser Ile Phe Cys Val Leu
                325                 330                 335

Thr Ala Lys Ser Arg Asp Pro Thr Ala Pro Leu Ala Asp Leu Leu Ile
            340                 345                 350

Phe Ser Pro Arg Trp Asp Val Ala Ser His Thr Tyr Arg Pro Pro Tyr
        355                 360                 365

Tyr His Arg Asn Ala Ala Ser Glu Leu Met Gly Leu Ile Tyr Gly Asp
    370                 375                 380

Tyr Gly Gly Arg Ser Asp Ala Phe Gln Pro Gly Ser Val Ser Phe Glu
385                 390                 395                 400

Cys Gly Met Val Pro His Gly Val Ala Tyr Glu Glu Phe Lys Ala Ala
                405                 410                 415

Thr Glu Val Asp Pro Pro Ala Met Arg Ile Ser Glu Ala Ser Met Ala
            420                 425                 430

Phe Met Phe Glu Ser Ser Arg Pro Phe Thr Ile Thr Asp Tyr Ala Trp
        435                 440                 445

Thr Ser Asn Lys Arg His Glu His Glu Pro Lys Met Trp Asp Asn Leu
    450                 455                 460

Val Asp Asn Phe Ser Lys His Glu Lys Glu Val Glu Glu Leu Leu Ala
465                 470                 475                 480

Lys Lys Ala Gln Gly Met Lys Ile
                485

<210>  6
<211>  1467
<212>  DNA
<213>  Aspergillus niger

<400>  6
atgccctcca caagtgacct cgccgacccc aaagaatacc agaagatctt ccactgggca      60

gagacacaga aggatggtac cattccctcc ttcgctacta gacggaatga tccctatgaa     120

taccaagccg gattcggcaa ctcgttcgcc tctgaggctg tgcctggcac ccttccacag     180

ggccaaaaca accctcgtaa cgtccgcttt ggcctgtacg ctgagcagat gacggccacg 240

gccttcattg ccccgagaca ttgcaacaag aaggcatggc tgtaccgtgc ccgtccggct 300

gttgcccacc agggattcac cgatcttccc gacaacaagg acacagaatc caactttctc 360

cctctgaacc ctcgcgtcca tgtctctcct acccagcttg cctggtaccc cttcgacatc 420

ccaaccaatg gcgaagtaga ctttgtgtct ggtctgaaga ctgtggctgg ctcaggcgag 480

ccaacccttc gtgagggtct ggccacccac gtctatgttg ctaacaccag catgaccaag 540

aaggcttttg tcaactctga tggtgagatg gctatcgtcc ctcaacaggg tgcactggat 600

attcagaccg aatttggtcc cttgttcgtc cagcctggag agattgtcat tatccaacgt 660

ggcatccgtt tccgcgtaga gcttcctgat gggccttctc gtggttacat ccttgagatc 720

tggggtagtc agtttgaact ccccgagctg ggacccctgg gtgccaacgg tctcgccaat 780

gcacgggact tcctctaccc cacggcaaag tatgagatct ccaaggagcc ttgggaggtt 840

gtgtacaagc tgggcggaaa gttcttcaag agcacacaaa accacagtcc gtttgacgtg 900

gttgcctggc acggcaacta tgtgccctac aagtacgaca tgaccaagtt cgtcaacgtc 960

ggttctattt ccgttgatca catcgatccc tctatcttct gtgttcttac agccaagtcc 1020

cgggatccca cggctccttt ggccgatcta ctcatcttct ctcctcgctg ggatgttgca 1080

tcgcacacct accgacctcc atactaccac cgcaacgccg cctccgaact gatgggtctc 1140

atctacggcg actacggcgg tcgttccgac gccttccagc ctggaagtgt ctccttcgaa 1200

tgtggaatgg tccctcacgg tgtagcctac gaagaattca aagccgccac ggaagtcgac 1260

ccgccggcca tgcggatctc cgaggcctca atggcattca tgttcgagtc gagtcgccca 1320

ttcaccatca cagactacgc ctggacaagc aacaagcgac acgagcacga acctaagatg 1380

tgggataacc tcgtcgacaa cttctccaag cacgagaaag aagtcgaaga gttattggcc 1440

aagaaggccc aggggatgaa gatctaa 1467

<210> 7
<211> 5007
<212> DNA
<213> Artificial Sequence

<220>
<223> Synthetic oligonucleotide

<400> 7
tggcgaatgg gacgcgccct gtagcggcgc attaagcgcg gcgggtgtgg tggttacgcg 60

cagcgtgacc gctacacttg ccagcgccct agcgcccgct cctttcgctt tcttcccttc 120

ctttctcgcc acgttcgccg gctttccccg tcaagctcta aatcgggggc tccctttagg 180

gttccgattt agtgctttac ggcacctcga ccccaaaaaa cttgattagg gtgatggttc 240

acgtagtggg ccatcgccct gatagacggt ttttcgccct ttgacgttgg agtccacgtt 300

ctttaatagt ggactcttgt tccaaactgg aacaacactc aaccctatct cggtctattc 360
ttttgattta taagggattt tgccgatttc ggcctattgg ttaaaaaatg agctgattta 420
acaaaaattt aacgcgaatt ttaacaaaat attaacgttt acaatttcag gtggcacttt 480
tcggggaaat gtgcgcggaa cccctatttg tttatttttc taaatacatt caaatatgta 540
tccgctcatg agacaataac cctgataaat gcttcaataa tattgaaaaa ggaagagtat 600
gagtattcaa catttccgtg tcgcccttat tccctttttt gcggcatttt gccttcctgt 660
ttttgctcac ccagaaacgc tggtgaaagt aaaagatgct gaagatcagt tgggtgcacg 720
agtgggttac atcgaactgg atctcaacag cggtaagatc cttgagagtt ttcgccccga 780
agaacgtttt ccaatgatga gcacttttaa agttctgcta tgtggcgcgg tattatcccg 840
tattgacgcc gggcaagagc aactcggtcg ccgcatacac tattctcaga atgacttggt 900
tgagtactca ccagtcacag aaaagcatct tacggatggc atgacagtaa gagaattatg 960
cagtgctgcc ataaccatga gtgataacac tgcggccaac ttacttctga caacgatcgg 1020
aggaccgaag gagctaaccg cttttttgca caacatgggg gatcatgtaa ctcgccttga 1080
tcgttgggaa ccggagctga atgaagccat accaaacgac gagcgtgaca ccacgatgcc 1140
tgcagcaatg gcaacaacgt tgcgcaaact attaactggc gaactactta ctctagcttc 1200
ccggcaacaa ttaatagact ggatggaggc ggataaagtt gcaggaccac ttctgcgctc 1260
ggcccttccg gctggctggt ttattgctga taaatctgga gccggtgagc gtgggtctcg 1320
cggtatcatt gcagcactgg ggccagatgg taagccctcc cgtatcgtag ttatctacac 1380
gacggggagt caggcaacta tggatgaacg aaatagacag atcgctgaga taggtgcctc 1440
actgattaag cattggtaac tgtcagacca agtttactca tatatacttt agattgattt 1500
aaaacttcat ttttaattta aaaggatcta ggtgaagatc ctttttgata atctcatgac 1560
caaaatccct taacgtgagt tttcgttcca ctgagcgtca gaccccgtag aaaagatcaa 1620
aggatcttct tgagatcctt tttttctgcg cgtaatctgc tgcttgcaaa caaaaaaacc 1680
accgctacca gcggtggttt gtttgccgga tcaagagcta ccaactcttt ttccgaaggt 1740
aactggcttc agcagagcgc agataccaaa tactgtcctt ctagtgtagc cgtagttagg 1800
ccaccacttc aagaactctg tagcaccgcc tacatacctc gctctgctaa tcctgttacc 1860
agtggctgct gccagtggcg ataagtcgtg tcttaccggg ttggactcaa gacgatagtt 1920
accggataag gcgcagcggt cgggctgaac ggggggttcg tgcacacagc ccagcttgga 1980
gcgaacgacc tacaccgaac tgagatacct acagcgtgag ctatgagaaa gcgccacgct 2040
tcccgaaggg agaaaggcgg acaggtatcc ggtaagcggc agggtcggaa caggagagcg 2100
cacgagggag cttccagggg gaaacgcctg gtatctttat agtcctgtcg ggtttcgcca 2160

cctctgactt gagcgtcgat ttttgtgatg ctcgtcaggg gggcggagcc tatggaaaaa 2220

cgccagcaac gcggcctttt tacggttcct ggccttttgc tggccttttg ctcacatgtt 2280

ctttcctgcg ttatcccctg attctgtgga taaccgtatt accgcctttg agtgagctga 2340

taccgctcgc cgcagccgaa cgaccgagcg cagcgagtca gtgagcgagg aagcggaaga 2400

gcgcctgatg cggtattttc tccttacgca tctgtgcggt atttcacacc gcatatatgg 2460

tgcactctca gtacaatctg ctctgatgcc gcatagttaa gccagtatac actccgctat 2520

cgctacgtga ctgggtcatg gctgcgcccc gacacccgcc aacacccgct gacgcgccct 2580

gacgggcttg tctgctcccg gcatccgctt acagacaagc tgtgaccgtc tccgggagct 2640

gcatgtgtca gaggttttca ccgtcatcac cgaaacgcgc gaggcagctg cggtaaagct 2700

catcagcgtg gtcgtgaagc gattcacaga tgtctgcctg ttcatccgcg tccagctcgt 2760

tgagtttctc cagaagcgtt aatgtctggc ttctgataaa gcgggccatg ttaagggcgg 2820

ttttttcctg tttggtcact gatgcctccg tgtaaggggg atttctgttc atgggggtaa 2880

tgataccgat gaaacgagag aggatgctca cgatacgggt tactgatgat gaacatgccc 2940

ggttactgga acgttgtgag ggtaaacaac tggcggtatg gatgcggcgg gaccagagaa 3000

aaatcactca gggtcaatgc cagcgcttcg ttaatacaga tgtaggtgtt ccacagggta 3060

gccagcagca tcctgcgatg cagatccgga acataatggt gcagggcgct gacttccgcg 3120

tttccagact ttacgaaaca cggaaaccga agaccattca tgttgttgct caggtcgcag 3180

acgttttgca gcagcagtcg cttcacgttc gctcgcgtat cggtgattca ttctgctaac 3240

cagtaaggca accccgccag cctagccggg tcctcaacga caggagcacg atcatgcgca 3300

cccgtggcca ggacccaacg ctgcccgaga tctcgatccc gcgaaattaa tacgactcac 3360

tatagggaga ccacaacggt ttccctatgg ttaccaccac cgcgatcccg ctgatcattc 3420

acggcaagga tgtgctgatc agcaacagcg accgtaaaca cccgattacc agcaccagcg 3480

cgccgcacct gaagagcttt cagggcgcga ccccggatct ggcgattcaa gcggttgaga 3540

gctgcgcgag cgcgttcccg acctggagcc aaaccccgcc ggaggaacgt cgtcgtctgc 3600

tgctgaacct ggcgaagctg ctgcgtgaga aagcaacacc catccaaacc ctgattaaag 3660

atgaaatcca ctgcaccccg ctgtggagcc acctgaacgt tgaaattgcg gcgggtatcg 3720

ttgaggaaac cgcgagcgtg attaccgacg cgatcagcgg taccattccg agcagccagg 3780

gcaacaccta cgcgctggtt atgaagaaac cgctgggtgt ggttctgggt attgcgccgt 3840

ggaacgcgcc ggtgattctg ggcctgcgta gcgttatccc ggtgattgcg gcgggtaaca 3900

ccgcgatcct gaagggcagc gagctgagcc cgcaaaccca ctacttcctg gcggcgctgt 3960

ttcgtgaagc gggtttcccg ccgggcgtta ttaactttgt tctgcaccgt ccgcaggatg 4020

cggcggagat ctatgaatgc atgattcaac acccggcggt taagaaatgc aacttcaccg 4080

gtagcaccca ggttggccgt gtgatcgcga gcaaggcggc gatggcgctg aaaccggtgc 4140

tgctggagct gggtggcaaa aacccggtgg ttgttctgga ggatgcggat ctggaccagg 4200

cggcggatga aatcgttgcg gcgggttacc tgaacaacgg ccaaatttgc atgagcaccg 4260

acctggttta tgttgtgcgt agcgtggcgg aggaactgac caagaaactg ctggagcgtc 4320

tgcacagcga agaccgtccg cataaagtga tcagcgcggc gagccgtgcg cgtctgaccc 4380

agctggttga cgatgcgcgt agcaaaggtg gcgtgattca ccaagcgcgt aacccgctgg 4440

cggaggaccc gctggcgttt ccggcgaccg ttatcgaagg tgtgaccgag gaaatggagt 4500

tctacaccat tgaaagcttc ggtccggttt ttggcatcat tgcggtggac agcgcggaga 4560

agggtctgca gatggttaaa cgtagcgcgt atggcctgag cagcgcggtg tttacccgtg 4620

atcacttcaa ggcgctggag atgagcgcgg acatcgcggt tggtgcggtt cacgtgaacg 4680

gtggcaccgt gcacgatgaa ccgaccctgc cgcatggtgg cgttggtgac agcggttatg 4740

gccgttttgg tggcaaatgg ggcctgaacg agttcctgca gacccaaacc gttatcctga 4800

accgtgtgcg tgcgagcctg tccgtcgaca agcttgcggc cgcactcgag caccaccacc 4860

accaccactg agatccggct gctaacaaag cccgaaagga agctgagttg gctgctgcca 4920

ccgctgagca ataactagca taaccccttg gggcctctaa acgggtcttg aggggttttt 4980

tgctgaaagg aggaactata tccggat 5007

<210> 8
<211> 4863
<212> DNA
<213> Artificial Sequence

<220>
<223> Synthetic oligonucleotide

<400> 8
tggcgaatgg gacgcgccct gtagcggcgc attaagcgcg gcgggtgtgg tggttacgcg 60

cagcgtgacc gctacacttg ccagcgccct agcgcccgct cctttcgctt tcttcccttc 120

ctttctcgcc acgttcgccg gctttccccg tcaagctcta aatcgggggc tccctttagg 180

gttccgattt agtgctttac ggcacctcga ccccaaaaaa cttgattagg gtgatggttc 240

acgtagtggg ccatcgccct gatagacggt ttttcgccct ttgacgttgg agtccacgtt 300

ctttaatagt ggactcttgt tccaaactgg aacaacactc aaccctatct cggtctattc 360

ttttgattta taagggattt tgccgatttc ggcctattgg ttaaaaaatg agctgattta 420

acaaaaattt aacgcgaatt ttaacaaaat attaacgttt acaatttcag gtggcacttt 480

tcggggaaat gtgcgcggaa cccctatttg tttatttttc taaatacatt caaatatgta 540

tccgctcatg agacaataac cctgataaat gcttcaataa tattgaaaaa ggaagagtat 600

gagtattcaa catttccgtg tcgcccttat tccctttttt gcggcatttt gccttcctgt 660

ttttgctcac ccagaaacgc tggtgaaagt aaaagatgct gaagatcagt tgggtgcacg 720

agtgggttac atcgaactgg atctcaacag cggtaagatc cttgagagtt ttcgccccga 780

agaacgtttt ccaatgatga gcacttttaa agttctgcta tgtggcgcgg tattatcccg 840

tattgacgcc gggcaagagc aactcggtcg ccgcatacac tattctcaga atgacttggt 900

tgagtactca ccagtcacag aaaagcatct tacggatggc atgacagtaa gagaattatg 960

cagtgctgcc ataaccatga gtgataacac tgcggccaac ttacttctga caacgatcgg 1020

aggaccgaag gagctaaccg cttttttgca caacatgggg gatcatgtaa ctcgccttga 1080

tcgttgggaa ccggagctga atgaagccat accaaacgac gagcgtgaca ccacgatgcc 1140

tgcagcaatg gcaacaacgt tgcgcaaact attaactggc gaactactta ctctagcttc 1200

ccggcaacaa ttaatagact ggatggaggc ggataaagtt gcaggaccac ttctgcgctc 1260

ggcccttccg gctggctggt ttattgctga taaatctgga gccggtgagc gtgggtctcg 1320

cggtatcatt gcagcactgg ggccagatgg taagccctcc cgtatcgtag ttatctacac 1380

gacggggagt caggcaacta tggatgaacg aaatagacag atcgctgaga taggtgcctc 1440

actgattaag cattggtaac tgtcagacca agtttactca tatatacttt agattgattt 1500

aaaacttcat ttttaattta aaaggatcta ggtgaagatc ctttttgata atctcatgac 1560

caaaatccct taacgtgagt tttcgttcca ctgagcgtca gaccccgtag aaaagatcaa 1620

aggatcttct tgagatcctt tttttctgcg cgtaatctgc tgcttgcaaa caaaaaaacc 1680

accgctacca gcggtggttt gtttgccgga tcaagagcta ccaactcttt ttccgaaggt 1740

aactggcttc agcagagcgc agataccaaa tactgtcctt ctagtgtagc cgtagttagg 1800

ccaccacttc aagaactctg tagcaccgcc tacatacctc gctctgctaa tcctgttacc 1860

agtggctgct gccagtggcg ataagtcgtg tcttaccggg ttggactcaa gacgatagtt 1920

accggataag gcgcagcggt cgggctgaac ggggggttcg tgcacacagc ccagcttgga 1980

gcgaacgacc tacaccgaac tgagatacct acagcgtgag ctatgagaaa gcgccacgct 2040

tcccgaaggg agaaaggcgg acaggtatcc ggtaagcggc agggtcggaa caggagagcg 2100

cacgagggag cttccagggg gaaacgcctg gtatctttat agtcctgtcg ggtttcgcca 2160

cctctgactt gagcgtcgat ttttgtgatg ctcgtcaggg gggcggagcc tatggaaaaa 2220

cgccagcaac gcggcctttt tacggttcct ggccttttgc tggccttttg ctcacatgtt 2280

ctttcctgcg ttatcccctg attctgtgga taaccgtatt accgcctttg agtgagctga 2340

taccgctcgc cgcagccgaa cgaccgagcg cagcgagtca gtgagcgagg aagcggaaga 2400

gcgcctgatg cggtattttc tccttacgca tctgtgcggt atttcacacc gcatatatgg 2460

tgcactctca gtacaatctg ctctgatgcc gcatagttaa gccagtatac actccgctat 2520

cgctacgtga ctgggtcatg gctgcgcccc gacacccgcc aacacccgct gacgcgccct 2580

gacgggcttg tctgctcccg gcatccgctt acagacaagc tgtgaccgtc tccgggagct 2640

gcatgtgtca gaggttttca ccgtcatcac cgaaacgcgc gaggcagctg cggtaaagct 2700

catcagcgtg gtcgtgaagc gattcacaga tgtctgcctg ttcatccgcg tccagctcgt 2760

tgagtttctc cagaagcgtt aatgtctggc ttctgataaa gcgggccatg ttaagggcgg 2820

ttttttcctg tttggtcact gatgcctccg tgtaaggggg atttctgttc atgggggtaa 2880

tgataccgat gaaacgagag aggatgctca cgatacgggt tactgatgat gaacatgccc 2940

ggttactgga acgttgtgag ggtaaacaac tggcggtatg gatgcggcgg gaccagagaa 3000

aaatcactca gggtcaatgc cagcgcttcg ttaatacaga tgtaggtgtt ccacagggta 3060

gccagcagca tcctgcgatg cagatccgga acataatggt gcagggcgct gacttccgcg 3120

tttccagact ttacgaaaca cggaaaccga agaccattca tgttgttgct caggtcgcag 3180

acgttttgca gcagcagtcg cttcacgttc gctcgcgtat cggtgattca ttctgctaac 3240

cagtaaggca accccgccag cctagccggg tcctcaacga caggagcacg atcatgcgca 3300

cccgtggcca ggacccaacg ctgcccgaga tctcgatccc gcgaaattaa tacgactcac 3360

tatagggaga ccacaacggt ttccctatgg agcaagcgaa aaccccgctg caagttattg 3420

ttgttggtgc gggcatcggt ggtatggcgg cggcgctgac cctgggcctg cgtggtcacc 3480

acgttaccat cctggagagc gcgccgaagc tgatggaagt gggtgcgggc attcaggtta 3540

gcccgaacat gctgaccctg tttgatcgtt ggggcgtgag cccgctgatc catgcgcaag 3600

atgtggcgct ggagcacatt cacgttcgtc gttgggaaga cggtagcatg ctgggcaccc 3660

tgccggttaa caagacctac ggtcagcaaa ccgtgatcca ccgtgcggat ctgcacaacg 3720

cgctgattga gaaagcgctg gcgctgagca acgtggaact gcgtgttaac agcaccgtga 3780

ccggcgttca gtttgaccac gcggcggttc aactggcgaa cggtaccatc gtgaaggcgg 3840

acatcgttat tgcggcggat ggtattaaaa gcgcgctgcg tgaccacctg ctggaggaag 3900

cgagcgttgc gatcccgacc ggtgatgcgg cgtaccgtat tatgctgccg cgtagcgcgc 3960

tggagaacga cccggaactg aaggcgctgg ttgacgaacc gcaggcgacc cgttggctgg 4020

gtccgggtcg tcatgtgatt gcgtacccgg ttcgtaacca cgacctgtat aacgtggttc 4080

tgctgcaccc ggatagccac ggtgttgagg aaagctggac caccaagggc agcaaacaag 4140

cgatggttga caactataag ggttgggatc gtcgtgtgac caaactgatt gacctggtgc 4200

cggacgatga ggttctggaa tggaagctgt gcctgcacgc gccgctgaaa acctggattc 4260

gtggtagcgt ggcgctgatt ggcgatgcgt gccatccgat gctgccgtat gtggcgcagg 4320

gtgcggcgca agcggttgag gatgctgcgg cgctgggcgt gctgctgagc accatcagca 4380

gccgtgatga gattccgctg gcgctgaagg cgtatgaaaa gagccgtaaa ccgcgtgcgg 4440

acaccgtgca gcaaagcggt agcgaaaacc gtatcaccct gcacctgccg gatggtccgg 4500

agcagcgtgc gcgtgatgaa caattccgta gcagcctgaa aagcggtagc aacccggacc 4560

gttggaccga tctggaaacc cagaagtttc tgtggggctg ggatgcggaa aaagcggcgc 4620

tggatgcgtg gaacgaactg cacggcaatc atgttagcga actgcgtcac cacctgtccg 4680

tcgacaagct tgcggccgca ctcgagcacc accaccacca ccactgagat ccggctgcta 4740

acaaagcccg aaaggaagct gagttggctg ctgccaccgc tgagcaataa ctagcataac 4800

cccttggggc ctctaaacgg gtcttgaggg gttttttgct gaaaggagga actatatccg 4860

gat 4863

<210> 9
<211> 5037
<212> DNA
<213> Artificial Sequence

<220>
<223> Synthetic oligonucleotide

<400> 9
tggcgaatgg gacgcgccct gtagcggcgc attaagcgcg gcgggtgtgg tggttacgcg 60

cagcgtgacc gctacacttg ccagcgccct agcgcccgct cctttcgctt tcttcccttc 120

ctttctcgcc acgttcgccg gctttccccg tcaagctcta aatcgggggc tccctttagg 180

gttccgattt agtgctttac ggcacctcga ccccaaaaaa cttgattagg gtgatggttc 240

acgtagtggg ccatcgccct gatagacggt ttttcgccct ttgacgttgg agtccacgtt 300

ctttaatagt ggactcttgt tccaaactgg aacaacactc aaccctatct cggtctattc 360

ttttgattta taagggattt tgccgatttc ggcctattgg ttaaaaaatg agctgattta 420

acaaaaattt aacgcgaatt ttaacaaaat attaacgttt acaatttcag gtggcacttt 480

tcggggaaat gtgcgcggaa cccctatttg tttatttttc taaatacatt caaatatgta 540

tccgctcatg agacaataac cctgataaat gcttcaataa tattgaaaaa ggaagagtat 600

gagtattcaa catttccgtg tcgcccttat tccctttttt gcggcatttt gccttcctgt 660

ttttgctcac ccagaaacgc tggtgaaagt aaaagatgct gaagatcagt tgggtgcacg 720

agtgggttac atcgaactgg atctcaacag cggtaagatc cttgagagtt ttcgccccga 780

agaacgtttt ccaatgatga gcacttttaa agttctgcta tgtggcgcgg tattatcccg 840

tattgacgcc gggcaagagc aactcggtcg ccgcatacac tattctcaga atgacttggt 900

tgagtactca ccagtcacag aaaagcatct tacggatggc atgacagtaa gagaattatg 960

cagtgctgcc ataaccatga gtgataacac tgcggccaac ttacttctga caacgatcgg 1020

aggaccgaag gagctaaccg cttttttgca caacatgggg gatcatgtaa ctcgccttga 1080

tcgttgggaa ccggagctga atgaagccat accaaacgac gagcgtgaca ccacgatgcc 1140

tgcagcaatg gcaacaacgt tgcgcaaact attaactggc gaactactta ctctagcttc 1200

ccggcaacaa ttaatagact ggatggaggc ggataaagtt gcaggaccac ttctgcgctc 1260

ggcccttccg gctggctggt ttattgctga taaatctgga gccggtgagc gtgggtctcg 1320

cggtatcatt gcagcactgg ggccagatgg taagccctcc cgtatcgtag ttatctacac 1380

gacggggagt caggcaacta tggatgaacg aaatagacag atcgctgaga taggtgcctc 1440

actgattaag cattggtaac tgtcagacca agtttactca tatatacttt agattgattt 1500

aaaacttcat ttttaattta aaaggatcta ggtgaagatc ctttttgata atctcatgac 1560

caaaatccct taacgtgagt tttcgttcca ctgagcgtca gaccccgtag aaaagatcaa 1620

aggatcttct tgagatcctt tttttctgcg cgtaatctgc tgcttgcaaa caaaaaaacc 1680

accgctacca gcggtggttt gtttgccgga tcaagagcta ccaactcttt ttccgaaggt 1740

aactggcttc agcagagcgc agataccaaa tactgtcctt ctagtgtagc cgtagttagg 1800

ccaccacttc aagaactctg tagcaccgcc tacatacctc gctctgctaa tcctgttacc 1860

agtggctgct gccagtggcg ataagtcgtg tcttaccggg ttggactcaa gacgatagtt 1920

accggataag gcgcagcggt cgggctgaac ggggggttcg tgcacacagc ccagcttgga 1980

gcgaacgacc tacaccgaac tgagatacct acagcgtgag ctatgagaaa gcgccacgct 2040

tcccgaaggg agaaaggcgg acaggtatcc ggtaagcggc agggtcggaa caggagagcg 2100

cacgagggag cttccagggg gaaacgcctg gtatctttat agtcctgtcg ggtttcgcca 2160

cctctgactt gagcgtcgat ttttgtgatg ctcgtcaggg gggcggagcc tatggaaaaa 2220

cgccagcaac gcggcctttt tacggttcct ggccttttgc tggccttttg ctcacatgtt 2280

ctttcctgcg ttatcccctg attctgtgga taaccgtatt accgcctttg agtgagctga 2340

taccgctcgc cgcagccgaa cgaccgagcg cagcgagtca gtgagcgagg aagcggaaga 2400

gcgcctgatg cggtattttc tccttacgca tctgtgcggt atttcacacc gcatatatgg 2460

tgcactctca gtacaatctg ctctgatgcc gcatagttaa gccagtatac actccgctat 2520

cgctacgtga ctgggtcatg gctgcgcccc gacacccgcc aacacccgct gacgcgccct 2580

gacgggcttg tctgctcccg gcatccgctt acagacaagc tgtgaccgtc tccgggagct 2640

gcatgtgtca gaggttttca ccgtcatcac cgaaacgcgc gaggcagctg cggtaaagct 2700

catcagcgtg gtcgtgaagc gattcacaga tgtctgcctg ttcatccgcg tccagctcgt 2760

tgagtttctc cagaagcgtt aatgtctggc ttctgataaa gcgggccatg ttaagggcgg 2820

ttttttcctg tttggtcact gatgcctccg tgtaaggggg atttctgttc atgggggtaa 2880

tgataccgat gaaacgagag aggatgctca cgatacgggt tactgatgat gaacatgccc 2940

ggttactgga acgttgtgag ggtaaacaac tggcggtatg gatgcggcgg gaccagagaa 3000

aaatcactca gggtcaatgc cagcgcttcg ttaatacaga tgtaggtgtt ccacagggta 3060

gccagcagca tcctgcgatg cagatccgga acataatggt gcagggcgct gacttccgcg 3120

tttccagact ttacgaaaca cggaaaccga agaccattca tgttgttgct caggtcgcag 3180

acgttttgca gcagcagtcg cttcacgttc gctcgcgtat cggtgattca ttctgctaac 3240

cagtaaggca accccgccag cctagccggg tcctcaacga caggagcacg atcatgcgca 3300

cccgtggcca ggacccaacg ctgcccgaga tctcgatccc gcgaaattaa tacgactcac 3360

tatagggaga ccacaacggt ttccctatgc cgagcaccag cgacctggcg gacccgaagg 3420

agtaccagaa aatcttccac tgggcggaaa cccaaaagga tggtaccatt ccgagctttg 3480

cgacccgtcg taacgacccg tacgagtatc aagcgggttt cggcaacagc ttcgcgagcg 3540

aagcggtgcc gggtaccctg ccgcagggtc aaaacaaccc gcgtaacgtt cgtttcggcc 3600

tgtacgcgga gcagatgacc gcgaccgcgt ttattgcgcc gcgtcactgc aacaagaaag 3660

cgtggctgta tcgtgcgcgt ccggcggtgg cgcaccaagg tttcaccgac ctgccggata 3720

acaaggacac cgagagcaac tttctgccgc tgaacccgcg tgtgcacgtt agcccgaccc 3780

agctggcgtg gtacccgttc gatatcccga ccaacggcga agtggacttt gttagcggtc 3840

tgaaaaccgt tgcgggtagc ggtgaaccga ccctgcgtga aggtctggcg acccacgtgt 3900

atgttgcgaa caccagcatg accaagaaag cgttcgtgaa cagcgatggc gagatggcga 3960

tcgttccgca gcaaggtgcg ctggacattc aaaccgagtt cggcccgctg tttgtgcagc 4020

cgggtgaaat cgttatcatt caacgtggca ttcgttttcg tgtggagctg ccggatggtc 4080

cgagccgtgg ttacatcctg gaaatttggg gtagccagtt tgagctgccg gaactgggtc 4140

cgctgggtgc gaacggtctg gcgaacgcgc gtgactttct gtacccgacc gcgaagtatg 4200

aaattagcaa agagccgtgg gaagtggttt acaagctggg tggcaagttc tttaaagcaa 4260

cccagaacca cagcccgttc gatgtggttg cgtggcacgg caactatgtg ccgtacaagt 4320

atgacatgac caaattcgtg aacgttggta gcatcagcgt ggatcacatc gacccgagca 4380

tcttctgcgt tctgaccgcg aaaagccgtg atccgaccgc gccgctggcg gacctgctga 4440

ttttcagccc gcgttgggac gttgcgagcc acacctatcg tccgccgtac tatcaccgta 4500

acgcggcgag cgaactgatg ggcctgatct acggtgatta tggtggccgt agcgatgcgt 4560

tccaaccggg cagcgtgagc tttgagtgcg gcatggtgcc gcacggtgtt gcgtacgagg 4620

agttcaaggc ggcgaccgaa gttgatccgc cggcgatgcg tatcagcgag gcgagcatgg 4680

cgttcatgtt tgaaagcagc cgtccgttca ccattaccga ctatgcgtgg accagcaaca 4740

agcgtcacga gcacgaaccg aaaatgtggg ataacctggt ggacaacttt agcaagcacg 4800

agaaagaagt tgaggaactg ctggcgaaga aagcgcaggg tatgaaaatc tccgtcgaca 4860

agcttgcggc cgcactcgag caccaccacc accaccactg agatccggct gctaacaaag 4920

cccgaaagga agctgagttg gctgctgcca ccgctgagca ataactagca taaccccttg 4980

gggcctctaa acgggtcttg aggggttttt tgctgaaagg aggaactata tccggat 5037

<210> 10
<211> 1434
<212> DNA
<213> Artificial Sequence

<220>
<223> Synthetic oligonucleotide

<400> 10
atggttacca ccaccgcgat cccgctgatc attcacggca aggatgtgct gatcagcaac 60

agcgaccgta aacacccgat taccagcacc agcgcgccgc acctgaagag ctttcagggc 120

gcgaccccgg atctggcgat tcaagcggtt gagagctgcg cgagcgcgtt cccgacctgg 180

agccaaaccc cgccggagga acgtcgtcgt ctgctgctga acctggcgaa gctgctgcgt 240

gagaaagcaa acaccatcca aaccctgatt aaagatgaaa tccactgcac cccgctgtgg 300

agccacctga acgttgaaat tgcggcgggt atcgttgagg aaaccgcgag cgtgattacc 360

gacgcgatca gcggtaccat tccgagcagc cagggcaaca cctacgcgct ggttatgaag 420

aaaccgctgg gtgtggttct gggtattgcg ccgtggaacg cgccggtgat tctgggcctg 480

cgtagcgtta tcccggtgat tgcggcgggt aacaccgcga tcctgaaggg cagcgagctg 540

agcccgcaaa cccactactt cctggcggcg ctgtttcgtg aagcgggttt cccgccgggc 600

gttattaact ttgttctgca ccgtccgcag gatgcggcgg agatctatga atgcatgatt 660

caacacccgg cggttaagaa atgcaacttc accggtagca cccaggttgg ccgtgtgatc 720

gcgagcaagg cggcgatggc gctgaaaccg gtgctgctgg agctgggtgg caaaaacccg 780

gtggttgttc tggaggatgc ggatctggac caggcggcgg atgaaatcgt tgcggcgggt 840

tacctgaaca acggccaaat ttgcatgagc accgacctgg tttatgttgt gcgtagcgtg 900

gcggaggaac tgaccaagaa actgctggag cgtctgcaca gcgaagaccg tccgcataaa 960

gtgatcagcg cggcgagccg tgcgcgtctg acccagctgg ttgacgatgc gcgtagcaaa 1020

ggtggcgtga ttcaccaagc gcgtaacccg ctggcggagg acccgctggc gtttccggcg 1080

accgttatcg aaggtgtgac cgaggaaatg gagttctaca ccattgaaag cttcggtccg 1140

gtttttggca tcattgcggt ggacagcgcg gagaagggtc tgcagatggt taaacgtagc 1200

gcgtatggcc tgagcagcgc ggtgtttacc cgtgatcact tcaaggcgct ggagatgagc 1260

gcggacatcg cggttggtgc ggttcacgtg aacggtggca ccgtgcacga tgaaccgacc 1320

ctgccgcatg gtggcgttgg tgacagcggt tatggccgtt ttggtggcaa atggggcctg 1380

aacgagttcc tgcagaccca aaccgttatc ctgaaccgtg tgcgtgcgag cctg          1434

<210>  11
<211>  1290
<212>  DNA
<213>  Artificial Sequence

<220>
<223>  Synthetic oligonucleotide

<400>  11
atggagcaag cgaaaacccc gctgcaagtt attgttgttg gtgcgggcat cggtggtatg      60

gcggcggcgc tgaccctggg cctgcgtggt caccacgtta ccatcctgga gagcgcgccg     120

aagctgatgg aagtgggtgc gggcattcag gttagcccga acatgctgac cctgtttgat     180

cgttggggcg tgagcccgct gatccatgcg caagatgtgg cgctggagca cattcacgtt     240

cgtcgttggg aagacggtag catgctgggc accctgccgg ttaacaagac ctacggtcag     300

caaaccgtga tccaccgtgc ggatctgcac aacgcgctga ttgagaaagc gctggcgctg     360

agcaacgtgg aactgcgtgt taacagcacc gtgaccggcg ttcagtttga ccacgcggcg     420

gttcaactgg cgaacggtac catcgtgaag ggcgacatcg ttattgcggc ggatggtatt     480

aaaagcgcgc tgcgtgacca cctgctggag gaagcgagcg ttgcgatccc gaccggtgat     540

gcggcgtacc gtattatgct gccgcgtagc gcgctggaga acgacccgga actgaaggcg     600

ctggttgacg aaccgcaggc gacccgttgg ctgggtccgg gtcgtcatgt gattgcgtac     660

ccggttcgta accacgacct gtataacgtg gttctgctgc acccggatag ccacggtgtt     720

gaggaaagct ggaccaccaa gggcagcaaa caagcgatgg ttgacaacta taagggttgg      780

gatcgtcgtg tgaccaaact gattgacctg gtgccggacg atgaggttct ggaatggaag     840

ctgtgcctgc acgcgccgct gaaaacctgg attcgtggta gcgtggcgct gattggcgat     900

gcgtgccatc cgatgctgcc gtatgtggcg cagggtgcgg cgcaagcggt tgaggatgct     960

gcggcgctgg gcgtgctgct gagcaccatc agcagccgtg atgagattcc gctggcgctg    1020

aaggcgtatg aaaagagccg taaaccgcgt gcggacaccg tgcagcaaag cggtagcgaa    1080

aaccgtatca ccctgcacct gccggatggt ccggagcagc gtgcgcgtga tgaacaattc    1140

cgtagcagcc tgaaaagcgg tagcaacccg gaccgttgga ccgatctgga aacccagaag    1200

tttctgtggg gctgggatgc ggaaaaagcg gcgctggatg cgtggaacga actgcacggc    1260

aatcatgtta gcgaactgcg tcaccacctg                                     1290

<210>  12
<211>  1464
<212>  DNA
<213>  Artificial Sequence

<220>

<223>  Synthetic oligonucleotide

<400>  12
atgccgagca ccagcgacct ggcggacccg aaggagtacc agaaaatctt ccactgggcg      60

gaaacccaaa aggatggtac cattccgagc tttgcgaccc gtcgtaacga cccgtacgag     120

tatcaagcgg gtttcggcaa cagcttcgcg agcgaagcgg tgccgggtac cctgccgcag     180

ggtcaaaaca acccgcgtaa cgttcgtttc ggcctgtacg cggagcagat gaccgcgacc     240

gcgtttattg cgccgcgtca ctgcaacaag aaagcgtggc tgtatcgtgc gcgtccggcg     300

gtggcgcacc aaggtttcac cgacctgccg gataacaagg acaccgagag caactttctg     360

ccgctgaacc cgcgtgtgca cgttagcccg acccagctgg cgtggtaccc gttcgatatc     420

ccgaccaacg gcgaagtgga ctttgttagc ggtctgaaaa ccgttgcggg tagcggtgaa     480

ccgaccctgc gtgaaggtct ggcgacccac gtgtatgttg cgaacaccag catgaccaag     540

aaagcgttcg tgaacagcga tggcgagatg gcgatcgttc cgcagcaagg tgcgctggac     600

attcaaaccg agttcggccc gctgtttgtg cagccgggtg aaatcgttat cattcaacgt     660

ggcattcgtt ttcgtgtgga gctgccggat ggtccgagcc gtggttacat cctggaaatt     720

tggggtagcc agtttgagct gccggaactg ggtccgctgg gtgcgaacgg tctggcgaac     780

gcgcgtgact ttctgtaccc gaccgcgaag tatgaaatta gcaaagagcc gtgggaagtg     840

gtttacaagc tgggtggcaa gttctttaaa agcacccaga accacagccc gttcgatgtg     900

gttgcgtggc acggcaacta tgtgccgtac aagtatgaca tgaccaaatt cgtgaacgtt     960

ggtagcatca gcgtggatca catcgacccg agcatcttct gcgttctgac cgcgaaaagc    1020

cgtgatccga ccgcgccgct ggcggacctg ctgattttca gcccgcgttg ggacgttgcg    1080

agccacacct atcgtccgcc gtactatcac cgtaacgcgg cgagcgaact gatgggcctg    1140

atctacggtg attatggtgg ccgtagcgat gcgttccaac cgggcagcgt gagctttgag    1200

tgcggcatgg tgccgcacgg tgttgcgtac gaggagttca aggcggcgac cgaagttgat    1260

ccgccggcga tgcgtatcag cgaggcgagc atggcgttca tgtttgaaag cagccgtccg    1320

ttcaccatta ccgactatgc gtggaccagc aacaagcgtc acgagcacga accgaaaatg    1380

tgggataacc tggtggacaa ctttagcaag cacgagaaag aagttgagga actgctggcg    1440

aagaaagcgc agggtatgaa aatc                                           1464


<210>  13
<211>  3239
<212>  DNA
<213>  Artificial Sequence

<220>
<223>  Synthetic oligonucleotide

<400>  13

cgaactactt tcggcatctt ctagacttcc taataccgcc tagtcatagc agattcaagc 60

tgagaacacc acaagtaaat atcacccatc atgcttaccc tgaccgtccc tgaaaactac 120

gggtatgtgc caattctaca attccttgca gacaatgcca ttctccccat gaagtctgat 180

gctaactatc ctgcagctct gtcattgccg tcgctctggg tgccatcccc gtcctgagct 240

tcgtccatgg cgccgtcgtg tctcgtctcc gcaaggaagc tgattgcccc taccctcact 300

gctatgcgac cgtagagcag tgcaagacca acgtaagcca acctcacaca aacaggattc 360

ctcgagctaa catacattcc gaaccgtgca gcccaaggcc gagcagttca actgcgctca 420

gcgcgctcat gccaacttcc ttgagaactc cagccaaact atgctcttcc tcctggtagc 480

tggactgaag tacccccagt tggcgactgg cctcggaagc atctgggtcc tcggtcgctc 540

actgttcctt tacggatatg tgtactccgg caagccgcgg ggtcgcggtc gtttgtacgg 600

cagcttctac ttgcttgcac agggagctct ctggggcttg acgtcttttg gagttgcgag 660

ggagttgatt tcctacttct aagtttggac tgaatccgtg gtgtgattga ggtgattggc 720

gatgtttggc tataccagct atatgtaata atctctactg tatactacta ttcaacgcat 780

tttactatgc gtgctgctag ggtcggcaat gacaatggca atctgactga cgtggtctat 840

ttctccatgt gcagcaggga atacgagctc caatggacct cgggagtggc acagtcaatg 900

gcaaggaaac tccgcctttg caggtgtggc tgaaccccac gggtcggagg cggagcaatc 960

cacccccgat gtggctggtg cgtggagggg ctcgcgatga ttttactgag cttgcttttc 1020

ttgtcgacat tgaacattgt ccttggtctt ccttcagatt taagggtcag tcactgctac 1080

atttctcagt agtatccgcg cacgtctctg gatttacgaa tcagggtcca ccagtcgaaa 1140

cttcgaacta ctctcattat acaatcctct ttccattccc gcattaaccc ctccatcaac 1200

accatgtcct ccaagtcgca attgacctac actgcccgtg ccagcaagca tcccaatgct 1260

ctggcgaaga ggctgttcga gattgccgag gccaagaaga ccaatgtgac tgtctcggct 1320

gacgttacca ccactaagga gctactagat cttgctgacc gtaggccgac ccgctactct 1380

gcctgattat gctgcatgca aacttattaa cggtgatacc ggactgcagg tctcggtccc 1440

tacattgccg tgatcaaaac ccacatcgat atcctctctg atttcagcaa cgagaccatt 1500

gagggactta aggctctcgc gcagaagcac aactttctca tcttcgagga ccgcaagttc 1560

attgacatcg gcaacacggt ccagaagcaa taccacggcg gtaccctccg tatctcggaa 1620

tgggcccaca tcatcaactg cagcattctc cctggtgagg gtatcgtcga ggctctcgct 1680

cagacggcgt ctgcaccgga cttcgcctac ggccccgaac gcggtctgtt gatcttggca 1740

gagatgacct ctaagggctc cttggctacc ggccagtaca ctacttcctc ggtcgattat 1800

gcccggaaat acaagaactt cgttatggga ttcgtgtcga cgcgcgcgtt gggtgaggtg 1860

cagtcggaag tcagctctcc ttcggatgag gaggactttg tggtcttcac gactggtgtg 1920

aacatttctt ccaagggaga taagcttggt cagcagtacc agacgcccgg atcggctatc 1980

ggccggggtg ctgacttcat tatcgcgggt cgcggtatct acgccgcgcc ggatccggtg 2040

caggctgcgc aacagtatca gaaggagggg tgggaagcct acctggcccg tgtcggcgga 2100

aactaatact ataaaaggag gatcgaagtt ctgatggtta tgaatgatat agaaatgcaa 2160

cttgccgcaa cggatacgga agcggaaacg gaccaatgtc gagcacgggt agtcagactg 2220

cggcatcgga tgtccaaacg gtattgatcc tgcaggctac tatggtgtgg cacaaggatc 2280

aatgcggtac gacgatttga tgcagataag caggctgcga agtagtaact cttgcgtaga 2340

gaaaatggcg acgggtggct gataagggcg gtgataagct taattgtcat cgcagataag 2400

cactgctgtc ttgcatccaa gtcagcgtca gcagaaatac gggacttccg aaagtatatg 2460

gcaaaattaa agaacttgac tctccagcaa tgttttgccc tgaccgtcgc taaaacgtta 2520

ctacccctat acccgtctgt ttgtcccagc ccgaggcatt aggtctgact gacagcacgg 2580

cgccatgcgg gcttgggacg ccatgtccgt cgcgtgataa gggttgatcc atgcagctac 2640

tatccttcca tcgttccatt cccatccttg tcctatctcc atccttgaaa ctttactagt 2700

ttagttggat gctcgagctt gctctcggct actccgtcca atggataaga ccccgatgcc 2760

ggtcctcatt ggtctccagc tggtatcgcc ccaaccttcg tgtgatcgcc tctctgcttc 2820

ccctcatcat cattactaac tagtacatcc aaaagccatc ccagtgcttc ccctcaccct 2880

tgcccaagac attccaagtg ggccttcggc tggaaaacat ggacccattg gttccatcga 2940

taagctagct cctcgtccgt taccccagat tgataccaga taacattgac cagcggctta 3000

tcaccgaggt ctgcgggtga gaccccccct gcgacaagtt agataaaaga aactcgcctc 3060

attgtgcttc cgatggggtc ggatgacgag ccttcggaaa gagctggcgc ctctttaaag 3120

gggacagctg tcgccaagtt gtgaaattct ccgataacta ctaacaatct ctcccttcct 3180

tcccgctact gtggtcacca aatcaactct cttttctcgg ccaagatcta acatggcgg 3239

<210> 14
<211> 3743
<212> DNA
<213> Artificial Sequence

<220>
<223> Synthetic oligonucleotide

<400> 14
gtggagacgt ataccctgaa ggtggacgac cactcgacta ttggggggct aacataaaaa 60

tataagtaag aatattgtct cttgactcgc actcccaagc ttgtagaaca tgacattcta 120

gccctaaccg ctgagaccac catcgcggaa tcaatgttgt cttcgaggcc cacttgcaat 180

tcgtcaggtc caggttcgga cttagacatc ggactccgtt gactcaggtt attatatgcc 240

tatatatggg ttaagcagct atgtgcctaa taggtgggtc attcgagaag ctatggatac 300
cacgtccctt ccttgcccct gacattcacg gaatacgatt aagatctccg atacacagcg 360
gagataaccc cggacatcga tctaaagccg ggcccaggtc ccacggtttg tggagtcaag 420
tgccgacctt ccgcaaactg catttcctgc tatcaccact cttcacacac tatgtacatg 480
aggatgctac tgcgatagat tgcgaaagat agtaatgcag taccggctta ccttattact 540
ttaatatctg ccatgtgtca tacaatcagg ccaaacgtct cattaaatag atcagaatac 600
cccaactttt gaggcagttt agtctgaaat gacagccgtg gttcactcgc cgccccacca 660
cgctaacgct cgccatcgcc cgtatatccg aactcctcac caaatctctg ccagtttcgg 720
atatacacag ccagcaacca gcaataagcc ggatatagca ccccgcaagg accttaactc 780
taatatgcca cagcgggtcc ggatagaaga cgcggcaacg tctcgacctg cagactcctc 840
tcgctgggcg ctaactgcgc gctcgaattc cgaagcggaa ccaaaccctg aagaagtgac 900
agggggttgc ttcaacggtt actaatgtcg gtagtggcgt ctgtgtttcc ttcaaataga 960
agagggggtg cccccagaaa aacgaaacat gattgatcct gcagaaaaac aaatcacaca 1020
ctccccggac ataccaagat ggtcaccaca accgcaattc ccctgataat ccacggcaag 1080
gatgtcctta tctccaatag tgaccgcaaa catcccatca cgtctacatc cgcacctcat 1140
ttgaaatcct tccaaggcgc aacgcccgat ctcgccatcc aagccgtcga atcctgcgca 1200
agtgccttcc caacatggtc gcaaacaccc cccgaggaac gtcgacgttt actactgaat 1260
ctagccaagg taagtaattc catgcactaa atttatggaa aagtaattgt gtctaataag 1320
aagtgaatgt acagctcctt cgcgaaaaat ccaacaccat tcagaccctc atcaaagatg 1380
aaatccactg cacaccactc tggtctcacc tcaacgttga aatcgcagcc ggcattgtcg 1440
aagagaccgc ttcagtaata acagacgcca ttagcggcac tatcccttcg tcgcagggaa 1500
atacgtacgc ccttgtgatg aagaagccgc tcggtgtggt gctgggcatc gcgccgtgga 1560
acgcgccggt aattttggga ctgagatcgg tgattccggt aattgcggcg ggcaacacag 1620
ctatcttgaa ggtgagaact tgagttacta ttatcaccta tgaggaaggg gaatttgttg 1680
ctgacaagag tagggctctg agttgagtcc ccaaacgcat tacttcctcg cggcactgtt 1740
ccgggaggca gggttcccgc caggagtcat taacttcgtt ctgcatcggc cgcaggacgc 1800
ggcagagatc tacgagtgta tgattcagca tccggctgtg aagaaatgta acttcaccgg 1860
atcaacccag gtgggacgtg tgatcgcgtc caaggcggcg atggcgttga agccggtgct 1920
gttggaattg ggtggcaaga accccgtggt ggtgttggag gatgcggatt tggatcaggc 1980
ggcggatgag attgttgcgg cgggatattt gaatgtgagt atctgcatct atccatggtg 2040
ctgtagtgga ggagaatgtg tgctgacaaa ttttgccaac agaatggcca gatttgcatg 2100
tcgactgatc tcgtctacgt tgttcgctcg gtggcggagg agttgactaa gaagctttta 2160

gagcgcttgc acagcgaaga tcggccacac aaggttattt cggcagcgag ccgagcccgt 2220

cttacgcagt tggtagatga tgcgcggtct aaggggggtg tcattcacca ggctcgtaac 2280

cctctcgctg aggatccctt ggcattccca gctactgtga ttgaaggagt cacggaggag 2340

atggagttct atacgatcga gtcgtttgga ccggtgtttg gaatcatcgc tgttgattcg 2400

gcagagaagg gtttgcaaat ggttaagcgg tccgcgtacg gcctttcatc ggcggtgttt 2460

acgcgtgatc atttcaaggc tctggaaatg tctgcagata ttgctgtggg agcggttcat 2520

gtcaacgggg gcacggtaca tgacgaaccg acgttaccgc atggaggagt gggcgacagt 2580

ggatatggtc ggtttggagg aaaatggggc ttgaatgagt ttttgcagac gcaaacggta 2640

attttgaatc gggttcgggc ttccctctga tacatgaaaa gagatattag gcatgatctg 2700

catgatatac gtgcattggt tacatgtgga gagatatatc agctgggcat atagtatgaa 2760

gggtcacaca cagccactaa ttcaactctc ttcattacct gtcgtcctct ccactgtctc 2820

aaataccaga aagccctctt gataactcaa acactatttc atctgagtgc attagatgaa 2880

atgctcgcct ggagccagat cacactctgc acatgaatga atttggaacc agagagtccg 2940

atggttactg atgcagccat gtttggaggg ccgacgttgg agaaggagac atctggctaa 3000

ctatcggagg taaattggat gttaaacttc gaggcatcac ctggatcatt cgcgaatcga 3060

cttgatgtct attttctcta gcttgaaacc actacaaatc aggtaaccca tgcaacgaag 3120

actaactcac ggcttcaata cttcgagtga tctggtcagt atccaatcct cctgtagctc 3180

ggattgatta gatagtgtag aacaacacca aatttcccac catcccaaac ccaattacgg 3240

tgtagaatca gccgggttca atcatcaata ttgccgtaac caagggataa ttgtttcagt 3300

ctactgtgac ctctcgatga cgacccatat cacatagcgt caaatatatt ttgtaatcta 3360

gggaggcgcg ttgttgtgcc agctacctgc caaactcttg acctcatgta gctcatgtaa 3420

cagctatggg tatgcaaaat aattgctcac gcgatcttga gccacatgat attgtcatga 3480

ctccccgtta acctcatact tttgactttc accgcatctc tacctcacct tcgtctcagt 3540

ctccagaatc tacttcaccc tttcaaaaac ctttcacttc gatatcttcc cacatctact 3600

ctcgccacac agtacaaaat ggatatgcat gaccccaagg gtatacaggg acagggagaa 3660

atcgacatga ctgcccttgt accttatttc agacttggtc tcggtctcac tcgggctcag 3720

ggtgaggaag cggagaagcc atg 3743

<210> 15
<211> 3698
<212> DNA
<213> Artificial Sequence

<220>
<223> Synthetic oligonucleotide

<400>  15
gcacagcagg tatcagagag agattgaaaa ggaaggagga ggaggagagg agatgacgat      60

cctaccgttc aacccactga gtagatagtc ggagtatcag gactgaggat gactgtagtc     120

tggaacaaag gcattatgga agggcaatcg gcaatgggag ggccaggcgt ttgccgcacg     180

agggcgagag aagcgaagac ggaaacagtg gagaaaaggc ggcgtgagca aacaattttg     240

gaggccggca aatgaaggcg ggcgaacagc cggctttgca ttgtttgcaa tctcaaatca     300

tgccccgtga ttggctcccc aggaaaaccg tttggccatt tgcccgtttt ctgcctcagc     360

gccttcgctg gcctgccttg cagctcaacg agaagactaa gatttctggt cttactaacc     420

tcctgtacac aatgcagcgc actaatttga catcatgcat ccttagtatc aggtggagcc     480

tcgcgcccac cacttccggg gtcgacatga ccctcgcgga tggttggcct gctcctcctc     540

ctttactagc catccaggtc tcaatattgg tccaatgatg cgcttctggt ctgacctata     600

ctcaccgggg ttgtggaggg ctggtctggt cttgcttccc tcggtggaga agcgatctgg     660

gggggttcgc gtcctgccgt catttccggc tggagggagt aaggacaacc gaagtctgag     720

cctgactctg gaattattta tcaggagcgt gcttgcccat cttgactttc tctgttgttg     780

ctattgttat tgtcaatctc atcaatctat cagtgttttc aatgaactcc cgtacctgag     840

tggcgaaccc tcatttatcc acggatattg tacggcccaa atttgccctt agcctaggac     900

ctccgaattt ttccccaaat ttttaaatct ttattttttc ttataactcc ggccctctct     960

ttcgcttcct ttatcatttt gatttccatt tccttgtaat ccaacctctt tgtcagaacc    1020

tttggattca ttcgcgatta tggagcaagc aaagactcct ctgcaggtga ttgtggttgg    1080

gtaagatcaa accatcctgt catctgagac tatgtattgt tactgacgtt accatatagt    1140

gccggtagga caactgtctc atagacttga tagcgaatga agcatgtaac taatgaaatc    1200

aggcattggc ggcatggctg cggccctgac cctgggtctg cgaggacatc atgttaccat    1260

cctcgagtcc gctcccaagg tgcgtatacc actccatgct atgatacata gtgctgactg    1320

acctctcgta gctcatggaa gttggtgctg gtattcaagt ctcaccgaat atgctgacac    1380

tgtttgaccg taagcgcccc ttacttctcc tgttactcaa ccccatctga tgatatgact    1440

cagggtgggg cgtctccccg ttgatccacg cccaggatgt tgccctcgag cacattcatg    1500

taagacggtg ggaggatggc agcatgcttg gcaccttgcc agtgaacaag acctacggcc    1560

aacagactgt cattcaccgc gcagacctcc acaacgccct catcgaaaag gcgctggcgc    1620

tgtcgaatgt ggagctgcgc gtgaactcga ccgtcactgg agttcaattc gaccatgccg    1680

cggtgcaatt ggccaacggc actattgtta aaggagacat cgtgatcgcg gctgacggca    1740

tcaagtctgc cctccgtgat cacttgctgg aagaagcatc tgtcgccatt cctacgggcg    1800

acgcagccta ccggattatg ctgcctcgta gtgctctaga gaatgaccct gagctaaagg    1860

cactggtaga tgagccacaa gccactcggt ggctcggacc tggccgtcat gtcatcgctt 1920

atccagtgcg caaccatgat ttgtacaatg ttgtcctgct tcatccagac agccatggtg 1980

tcgaggagtc gtggacgacc aagggttcca agcaggccat ggtcgacaac tacaagggct 2040

gggaccggcg tgtgacgaag cttatcgacc tggtaccgga cgatgaagtc ctggaatgga 2100

agctgtgcct tcacgctccg ctgaagacct ggattagggg ttccgtggcc ctcatcggtg 2160

acgcctgcca cccaatgctg taagcaccct tcattttaga cattctggct agcactaacg 2220

tcttctaggc catatgttgc tcaaggtgct gcccaggccg tcgaagacgc tgctgccctc 2280

ggtgtcctgc tctccactat ctcttcccgg gatgaaatcc ctcttgcact caaagcgtac 2340

gagaagtccc gcaagcctcg cgcagacacc gtccagcaat ctgggtcgga gaaccgcatc 2400

acgctccacc tgcccgatgg cccggagcaa cgcgcgcgtg acgaacagtt ccgttcttcc 2460

ctgaagagcg gatcaaaccc cgaccggtgg acagacctgg aaacccagaa gttcctctgg 2520

ggctgggatg ctgagaaggc agctctggat gcatggaatg gtaagttgcc atcgccattc 2580

acctacctat ggcaagatat gctaacaaaa accagaactc catggcaacc acgtgtccga 2640

actgcggcac cacctgtgat ctgtgatttg caataatatc acatgcacat aagaaatacg 2700

ttccttcttc aacatgattg ggattgatta acacttttag ccctggcgtt ctcatcaacg 2760

tttcattagc gattatcata tacttttatg aatcactgta catacttacc atcaggcata 2820

tagaatgcac atatgaacct ctttctctgc tttgctttct ttttctgaat tttgaattta 2880

tcataactga cggacgatgt gaaggggtgc tgtcgtgcag ttgctaggaa tttgtaatgg 2940

ctcatgacct gtacgagctg acgtagacaa gacaaaaagc acaacttatg gtggatagct 3000

gaattagacc attgcttttc tgatatggaa gggtttacac aggacaaaag cacagaaaat 3060

tagaggggac gatcgatttc tggcggataa gaagtgaaac gtgacttagg atgttgcgga 3120

cagggagagc ctggctagaa tacagcgggt gactggttac ccgttacaca gagaggtagg 3180

atctcactca agtacagtgt aagaattatt actcctagga tgtacataag ctaattacaa 3240

aaatatctat attcaccgtc taccagatcc tcttggccac catctcatat cgttggacat 3300

gatccatgtt caggtcctcc ttggtattct tgtctttctc ttctttggcc ttcagccgcc 3360

gcagtccctc agggtcatcc gcatagatcc atcgtcgatg atcatcgtta acctcgacca 3420

cgccccatcc gccgaaccgt cggctgccct caactgcaaa tgctcgtcca cgggggcctg 3480

tcccgttgga ttcatcagca ggtgctgcag atgaggcatt ggcttgactg gatgattcgc 3540

gcagcttcat cgccttgtac cgagaccgtg cgtacgctag caagaccatg cccagaagaa 3600

gcttcaggat taggagtacc acgaagcccg ttaagatgag gactatggtg aagatgtata 3660

ctgattgggc tggtgacggc atggtgttgg ctagaagc 3698

<210>  16
<211>  3789
<212>  DNA
<213>  Artificial Sequence

<220>
<223>  Synthetic oligonucleotide

<400>  16
cctgctattc ttgtttgacg gcgccgacct gtccttcaac cctgctgcac aagcggttgg      60

tcaggagatg cggagccgtt gggtgcagtt cgtcaacggt ggtagtccgt ggtctaccga     120

gcagagattc gcgtacggac ctctggggag ctgcggagag atttctgaag cacagtttgc     180

atctagacgg cgggttgagc atgttaaagt tcttagggag gctggaatgg gtgtctatat     240

gcctattgta tttgccttga cggcaggcaa gattagcttg ttgaattaga cagtgatata     300

gttcaataca gctacataac caattcgcca cactgaatac aggaatctcc acttatgata     360

ttctttacct tttctttctt tgtattgccg taagctgtga gatattggtg atacaaagca     420

gatagttaga tactattgct tcttggagta tatgcagaag agcagtatac atcggcaatt     480

ccagcgatgg ctgctacccg ggggtgtccc cgcaagcgca aaggcgggca aacttcccca     540

ccgttactca ggcctgactc atgtggggtc gctccactga ctgatgcctg aggtgcctga     600

tgttgcctga gtcgttcatt gcttgtacta ctctcggcaa cactcccccc tcgacatctg     660

ccgaacatcc ccccaattgc tcgccaactt ccaatatcgt atgcaatcca ttcaatactc     720

ccaaactctg gtctcatcca tcaatcctga cttgctgtct ttgaatcagc tggcccggat     780

atccggatgg ccagcggtct gattaattgc tctataatgg cctcccgctt ggaccgactc     840

caccagctca agtatatgag cctgctggaa cctaccccgg ctgtcctccc catatatcac     900

gattcatgac tctataaaca ccgtgttccc ttacacaaac cctttttcca cctacaatag     960

ctactttcct gagacctatc acccatcatg ccctccacaa gtgacctcgc cgaccccaaa    1020

gaataccaga agatcttcca ctgggcagag acacagaagg atggtaccat tccctccttc    1080

gctactagac ggaatgatcc ctatgaagta agtgttctgt gccaatgcct accctcttct    1140

taccgtcact aactcaagcg caacagtacc aagccggatt cggcaactcg tatgtcaaag    1200

cgctcccctg catctagcga ttcaactggc tgatgtagtt aatctactag gttcgcctct    1260

gaggctgtgc ctggcaccct tccacagggc caaaacaacc ctcgtaacgt ccgctttggc    1320

ctgtacgctg agcagatgac ggccacggcc ttcattgccc cgagacattg caacaagaag    1380

gcatggctgt accgtgcccg tccggctgtt gcccaccagg gattcgtatg tcttgcagct    1440

ctgtcatcga cgattgctat ctactgacag tgactgtaga ccgatcttcc cgacaacaag    1500

gacacagaat ccaactttct ccctctgaac cctcgcgtcc atgtctctcc tacccagctt    1560

gcctggtacc ccttcgacat cccaaccaat ggcgaagtag actttgtgtc tggtctgaag    1620

actgtggctg gctcaggcga gccaaccctt cgtgagggtc tggccaccca cgtctatgtt 1680

gctaacacca gcatgaccaa gaaggctttt gtcaactctg atggtgagat ggctatcgtc 1740

cctcaacagg gtgcactgga tattcagacc gaatttggtc ccttgttcgt ccagcctgga 1800

gagattgtca ttatccaacg tggcatccgt ttccgcgtag agcttcctga tgggccttct 1860

cgtggttaca tccttgagat ctggggtagt cagtttgaac tccccgagct gggacccctg 1920

ggtgccaacg gtctcgccaa tgcacgggac ttcctctacc ccacggcaaa gtatgagatc 1980

tccaaggagc cttgggaggt tgtgtacaag ctgggcggaa agttcttcaa gagcacacaa 2040

aaccacagtc cgtttgacgt ggttgcctgg cacggcaact atgtatgctt tcattccctt 2100

tccggcatgc ttccaagcaa tagtatacta attcatcata caggtgccct acaagtacga 2160

catgaccaag ttcgtcaacg tcggttctat ttccgttgat cacatcgatc cctctatctt 2220

ctgtgttctt acagccaagt cccgggatcc cacggctcct ttggccgatc tactcatctt 2280

ctctcctcgc tgggatgttg catcgcgtac gttccgcccg cagactgaag ttgcctgact 2340

cttgctaaca acaacaatga tagacaccta ccgacctcca tactaccacc gcaacgccgc 2400

ctccgaactg atgggtctca tctacggcga ctacggcggt cgttccgacg ccttccagcc 2460

tggaagtgtc tccttcgaat gtggaagtaa gcatcacccc taccccccga ttccaaattt 2520

aaaaaagcca cacagctaac ctactgatca accagtggtc cctcacggtg tagcctacga 2580

agaattcaaa gccgccacgg aagtcgaccc gccggccatg cggatctccg aggcctcaat 2640

ggcattcatg ttcgagtcga gtcgcccatt caccatcaca gactacgcct ggacaagcaa 2700

caagcgacac gagcacgaac ctaagatgtg ggataacctc gtcgacaact tctccaagca 2760

cgagaaagaa gtcgaagagt tattggccaa gaaggcccag gggatgaaga tctaactttt 2820

gtatatatag cagatagtat gttgctattc ttgctgtgtt ttgtttcccc gcccttgagc 2880

ttagccattc ctccttttct gtgtgcatgt tcttggtctt gttgttcatg tttcatgcct 2940

gtgattgtta tgattgtatt actaggtgtc tttgtctgta catatttgag acactagtca 3000

tatatgccaa cattattctt gtgctttgtg aagaagcgtg acgtgtgcat gacgtatagt 3060

atattcaggg accatcattt gtagaatgcc gacgtctaga gatttcaaac ttttccttgt 3120

attcttgagg gaaacagaaa gaagacttgg gaagctcatt cgacttgtta agagaggggc 3180

aacaatgcag gacatatcca ccacatgctc gaatatgcct gaaaacacga cagattggca 3240

caaaaggatg aacaattacg gttggtctgt cacattgggc tcaaagccca caatgcaagt 3300

ggtcgtgtat gtctacctga ggcgcatacc ggttttcagg cagcgacaca gggcagcacc 3360

ccaccaagtg gcaaaacaaa aacacaatgt atgtgttatg acagtacaga ataaacagca 3420

agtctcggta cgtcttttat tttcatgaat tggtatcaac acggtcagaa atacggacat 3480

acaattattt aggccagacc gttttggttg tgtctttccg ttgccaaacg tcccaggagc 3540

cctttcgaag ggtcggagaa cctcaacttg ccagaggtga gccggttatg tctatcacaa 3600

aaatcaaatc acaggaagta gtcgggtgtg cgcctcgaga ccatgggttc tcctgcgcgc 3660

gggcagggat cgaattgtaa gctgtagatt gtgtcagtaa tttcttctcc agtttgcaat 3720

gatggggcca accacataca aggtatattt cagatgctcg tcaatttcca taattgcggc 3780

ttggttacc 3789

<210> 17
<211> 24
<212> DNA
<213> Artificial Sequence

<220>
<223> Synthetic oligonucleotide

<400> 17
cgaactactt tcggcatctt ctag 24

<210> 18
<211> 49
<212> DNA
<213> Artificial Sequence

<220>
<223> Synthetic oligonucleotide

<400> 18
gcacttacct tcgcattttc tggtatattc gtaaatccag agacgtgcg 49

<210> 19
<211> 49
<212> DNA
<213> Artificial Sequence

<220>
<223> Synthetic oligonucleotide

<400> 19
cctgtgttgg ttctcaggaa ctgcgaatat ggatacggaa gcggaaacg 49

<210> 20
<211> 20
<212> DNA
<213> Artificial Sequence

<220>
<223> Synthetic oligonucleotide

<400> 20
ccgccatgtt agatcttggc 20

<210> 21
<211> 21

<212>  DNA
<213>  Artificial Sequence

<220>
<223>  Synthetic oligonucleotide

<400>  21
cgcaagttca ttgacatcgg c                                                21


<210>  22
<211>  23
<212>  DNA
<213>  Artificial Sequence

<220>
<223>  Synthetic oligonucleotide

<400>  22
ggaagaaatg ttcacaccag tcg                                              23


<210>  23
<211>  22
<212>  DNA
<213>  Artificial Sequence

<220>
<223>  Synthetic oligonucleotide

<400>  23
gcacagcagg tatcagagag ag                                               22


<210>  24
<211>  52
<212>  DNA
<213>  Artificial Sequence

<220>
<223>  Synthetic oligonucleotide

<400>  24
gcacttacct tcgcattttc tggtatattt ccaaaggttc tgacaaagag gt              52


<210>  25
<211>  54
<212>  DNA
<213>  Artificial Sequence

<220>
<223>  Synthetic oligonucleotide

<400>  25
cctgtgttgg ttctcaggaa ctgcgaatat cgttccttct tcaacatgat tggg            54


<210>  26
<211>  20
<212>  DNA
<213>  Artificial Sequence

<220>
<223>  Synthetic oligonucleotide

<400>  26
gcttctagcc aacaccatgc                                                    20


<210>  27
<211>  23
<212>  DNA
<213>  Artificial Sequence

<220>
<223>  Synthetic oligonucleotide

<400>  27
cgaaacatga ttgatcctgc aga                                                23


<210>  28
<211>  24
<212>  DNA
<213>  Artificial Sequence

<220>
<223>  Synthetic oligonucleotide

<400>  28
ggctagattc agtagtaaac gtcg                                               24


<210>  29
<211>  22
<212>  DNA
<213>  Artificial Sequence

<220>
<223>  Synthetic oligonucleotide

<400>  29
gcacagcagg tatcagagag ag                                                 22


<210>  30
<211>  52
<212>  DNA
<213>  Artificial Sequence

<220>
<223>  Synthetic oligonucleotide

<400>  30
gcacttacct tcgcattttc tggtatattt ccaaaggttc tgacaaagag gt                52


<210>  31
<211>  54
<212>  DNA
<213>  Artificial Sequence

<220>
<223>  Synthetic oligonucleotide

<400>  31
cctgtgttgg ttctcaggaa ctgcgaatat cgttccttct tcaacatgat tggg          54


<210>  32
<211>  20
<212>  DNA
<213>  Artificial Sequence

<220>
<223>  Synthetic oligonucleotide

<400>  32
gcttctagcc aacaccatgc                                                20


<210>  33
<211>  20
<212>  DNA
<213>  Artificial Sequence

<220>
<223>  Synthetic oligonucleotide

<400>  33
gcgaggacat catgttacca                                                20


<210>  34
<211>  20
<212>  DNA
<213>  Artificial Sequence

<220>
<223>  Synthetic oligonucleotide

<400>  34
caaatcatgg ttgcgcactg                                                20


<210>  35
<211>  21
<212>  DNA
<213>  Artificial Sequence

<220>
<223>  Synthetic oligonucleotide

<400>  35
cctgctattc ttgtttgacg g                                              21


<210>  36
<211>  52
<212>  DNA
<213>  Artificial Sequence

<220>
<223>  Synthetic oligonucleotide

<400>  36
gcacttacct tcgcattttc tggtatattg catgatgggt gataggtctc ag            52

<210>  37
<211>  52
<212>  DNA
<213>  Artificial Sequence

<220>
<223>  Synthetic oligonucleotide

<400>  37
cctgtgttgg ttctcaggaa ctgcgaatat ccttgagctt agccattcct cc          52


<210>  38
<211>  22
<212>  DNA
<213>  Artificial Sequence

<220>
<223>  Synthetic oligonucleotide

<400>  38
ggtaaccaag ccgcaattat gg                                           22


<210>  39
<211>  19
<212>  DNA
<213>  Artificial Sequence

<220>
<223>  Synthetic oligonucleotide

<400>  39
gccgacccca aagaatacc                                               19


<210>  40
<211>  21
<212>  DNA
<213>  Artificial Sequence

<220>
<223>  Synthetic oligonucleotide

<400>  40
tctcaaggat gtaaccacga g                                            21


<210>  41
<211>  501
<212>  PRT
<213>  Aspergillus niger

<400>  41

Met Ala Ser Pro Gln Ile Gln His Glu Gly Leu Arg Ala Ile Phe Thr
1               5                   10                  15


Gly Ile Glu Arg Val Ser Glu Gly Val Thr Val Asn Glu Phe Arg Gly
            20                  25                  30

Ile Lys Tyr Ala Ser Val Pro Ala Arg Phe Glu Arg Ala Gln Pro Val
        35                  40                  45

Asp Gly Phe Gly Asp Ala Val Val Asp Ala Ser Ser Tyr Gly Pro Arg
    50                  55                  60

Cys Pro Gln Val Asp Val Asp Val Arg His Leu Leu Arg Ile Pro Glu
65                  70                  75                  80

Asp Phe Pro Ile Glu Arg Glu Pro Glu Asp Glu Phe Glu Cys Leu Asn
                85                  90                  95

Leu Asp Ile Thr Cys Pro Pro Ala Ser Ser Ala Lys Gly Pro Leu Pro
            100                 105                 110

Val Leu Leu Trp Ile Tyr Gly Gly Ser Gln Val Val Thr Phe Cys Ser
        115                 120                 125

Ala Ala Ser Lys Ile Cys Asp Pro Val Lys Ile Val Ala Asp Ser Ile
    130                 135                 140

Lys Ser Gly Gln Pro Ile Ile Phe Val Ser Ile Asn Tyr Arg Leu Asn
145                 150                 155                 160

Ile Phe Ser Phe Gly Asp Gly Lys Glu Lys Asn Leu Ala Ile Lys Asp
                165                 170                 175

Gln Lys Leu Gly Ile Glu Trp Val Arg Asn Asn Ile Ala Ala Phe Gly
            180                 185                 190

Gly Asp Pro Asn Asn Ile Thr Leu Ala Gly Glu Ser Ala Gly Ala Val
        195                 200                 205

Tyr Thr His Ala His Leu Val Thr Gly Thr Pro Val Lys Arg Ala Val
    210                 215                 220

Leu Ala Ser Gly Ser Leu Tyr Leu Ser Ser Pro Leu Pro Ile Glu Lys
225                 230                 235                 240

Gly Gln Gly Leu Ile Asn Ala Leu Glu Ala Lys Val Gln Glu Leu Gly
                245                 250                 255

Glu Pro Ser Leu Arg Leu Ala Ser Val Pro Ala Leu Leu Gln Ala Leu
            260                 265                 270

Arg Glu Cys Asn Val Asn Thr Met Trp Ile Gln Glu Asp Glu Asp Phe
        275                 280                 285

Gln Gly Trp Glu Thr Lys Ser Glu Leu Val Asp Glu Leu Met Ile Gly
    290                 295                 300

Asp Thr Glu Tyr Glu Ser Val Ile Trp Arg Asn Gly Ile Glu Thr Phe
305                 310                 315                 320

Asp Gly Glu Ala Ile Thr Ala Ala Phe Glu Gln Asn Glu Lys Trp Gly
                325                 330                 335

Pro Lys Leu Arg Lys Met Tyr Gln Val Val Ala Asp Arg Pro Thr Ala
            340                 345                 350

Cys Lys Leu Gly Ala Leu Asp Leu Val Asn Asp Val Arg Tyr Thr Leu
        355                 360                 365

Pro Val Glu Val Val Ala Glu Lys Leu Gln Ala Ala Gly Lys Arg Val
    370                 375                 380

Tyr Lys Tyr Val Val Asp Gln Ala Asn Pro Trp Gln Ala Ser Ser Arg
385                 390                 395                 400

Ala His His Ala Val Asp Leu Leu Phe Leu Phe Asp Gly Ala Asp Leu
                405                 410                 415

Ser Phe Asn Pro Ala Ala Gln Ala Val Gly Gln Glu Met Arg Ser Arg
            420                 425                 430

Trp Val Gln Phe Val Asn Gly Gly Ser Pro Trp Ser Thr Glu Gln Arg
        435                 440                 445

Phe Ala Tyr Gly Pro Leu Gly Ser Cys Gly Glu Ile Ser Glu Ala Gln
    450                 455                 460

Phe Ala Ser Arg Arg Arg Val Glu His Val Lys Val Leu Arg Glu Ala
465                 470                 475                 480

Gly Met Gly Val Tyr Met Pro Ile Val Phe Ala Leu Thr Ala Gly Lys
                485                 490                 495

Ile Ser Leu Leu Asn
            500

<210>  42
<211>  1503
<212>  DNA
<213>  Artificial Sequence

<220>
<223>  Synthetic oligonucleotide

<400>  42
atggcatctc cgcagataca gcatgagggc cttcgtgcca ttttcacggg gattgagagg      60

gtgtccgagg gggtcacggt gaatgagttt aggggtatca agtacgcgtc tgttcccgcg     120

cggtttgagc gggcgcagcc ggtggatggc tttggggatg ctgtggttga tgcttcttca     180

tacgggccga ggtgtccgca ggtcgatgtc gacgttcgcc atttgctgcg cattccagag     240

gactttccaa tcgaaaggga gcctgaggat gagtttgaat gtctgaattt ggacatcacc     300

tgtcctccgg cgtcttcggc taagggacct cttcctgtgc tcttgtggat ttatggagga     360

tcgcaggtgg tgaccttttg ctcagctgca tcgaagatct gcgatcccgt caagatcgtc     420

gcggactcta tcaagtccgg ccagcctatc atcttcgtct caatcaacta tcgactcaac     480

atcttcagct ttggcgacgg caaggagaag aaccttgcta tcaaggatca gaaactgggt     540

attgagtggg tgcggaacaa catcgctgcc tttggaggcg atccgaacaa catcacgttg     600

gccggagaaa gtgccggggc agtttatacg catgcccatc tcgtcactgg tacaccagtc     660

aagagggccg tcctggcatc tggatccctc tatctctctt ctcccttgcc aatagagaag     720

ggccagggac taatcaatgc gctggaagcc aaggtgcagg aactgggcga gccgtcgctt     780

cggttagcgt cagtgccggc tctgttgcag gcattacgag agtgcaatgt gaacactatg     840

tggatacagg aggatgagga ctttcaagga tgggaaacca agtccgagct ggtggacgaa     900

ctcatgattg gtgatactga atatgagtcg gttatctgga ggaacggtat cgagacattc     960

gatggagaag cgattactgc agccttcgag caaaatgaga aatggggtcc gaagctacgc    1020

aagatgtacc aggttgtcgc ggatcgaccc actgcttgca agttgggtgc actggacctt    1080

gtgaatgatg tccgttatac gctgccagta gaagttgttg ccgagaaact acaggcagct    1140

gggaaacgag tctacaagta tgtggttgac caggccaacc cgtggcaggc atccagccga    1200

gcccatcatg ctgttgacct gctattcttg tttgacggcg ccgacctgtc cttcaaccct    1260

gctgcacaag cggttggtca ggagatgcgg agccgttggg tgcagttcgt caacggtggt    1320

agtccgtggt ctaccgagca gagattcgcg tacggacctc tggggagctg cggagagatt    1380

tctgaagcac agtttgcatc tagacggcgg gttgagcatg ttaaagttct tagggaggct    1440

ggaatgggtg tctatatgcc tattgtattt gccttgacgg caggcaagat tagcttgttg    1500

aat                                                                  1503

<210>  43
<211>  1503
<212>  DNA
<213>  Artificial Sequence

<220>

<223>  Synthetic oligonucleotide codon optimised

<400>  43
atggcgtctc cacaaataca gcatgaaggg ttgagagcaa tttttacggg tattgaaaga      60

gtttctgaag gtgttactgt aaacgaattt agaggcatta agtatgccag cgtgccagct     120

agattcgaac gtgcacaacc agtcgatggc tttggcgatg ctgttgttga cgcctcatcc     180

tacggcccac gttgtccaca agttgatgtg gatgtgagac atctacttag aattccagag     240

gattttccga ttgagagaga accagaggac gagttcgaat gcttaaactt ggacataact     300

tgcccccctg cctcttcagc taaaggtccg ttgccagtgt tgttgtggat atatggtggt     360

tctcaagtcg ttacgttttg ttcagctgct tcaaagattt gcgatcccgt taaaattgtt     420

gcagattcaa ttaagtcagg acaaccaata attttcgtgt ctattaacta tagactaaat     480

attttctctt ttggagacgg taaagagaaa aatctagcta ttaaagatca aaaactaggt     540

atcgagtggg ttaggaataa catagcagca ttcggtggtg atcccaataa cattaccctg     600

gctggcgaga gtgctggcgc tgtgtatacc catgcccatc ttgttacagg tactccagtt     660

aaacgtgctg tgttagcttc aggtagcttg tacttatcat ctccgcttcc tattgagaag     720

gggcaaggac ttattaatgc attggaagct aaggttcaag aattagggga accctcctta     780

agattggcaa gtgttcctgc attgttgcaa gcattgaggg aatgtaatgt gaacactatg     840

tggattcagg aagacgaaga ttttcaaggc tgggaaacga aaagcgaact agttgacgaa     900

ctaatgattg gtgatacaga atatgaatct gtcatttgga gaaacggaat tgagacgttc     960

gatggtgaag ccattaccgc cgctttcgaa cagaatgaaa agtgggggcc aaaattgagg    1020

aaaatgtatc aagtcgtggc cgaccgtcca actgcctgca aattaggagc attggatttg    1080

gttaatgatg tgagatacac tcttcctgtg gaagttgttg cagaaaagct gcaagccgcc    1140

ggtaaaaggg tatataaata tgtggtcgat caagcaaatc cttggcaagc atcttcaaga    1200

gctcatcatg cagtagatct actgtttcta tttgatggcg cggatctttc tttcaatccc    1260

gctgcgcagg cggttggtca ggaaatgcgt agcagatggg tccaattcgt gaatggaggt    1320

tctccatggt ctactgaaca aagatttgca tatggtcctc tggggagttg cggtgaaata    1380

tcagaagcac agttcgcatc cagaaggaga gttgagcatg taaaggtttt aagagaagca    1440

ggaatgggtg tttatatgcc aatcgtgttt gctcttaccg ctggtaagat atcactactt    1500

aat                                                                  1503

<210>  44
<211>  20
<212>  DNA
<213>  Artificial Sequence

<220>
<223>  Synthetic oligonucleotide

<400>  44
gtggaggaaa aggaggcacg                                                   20


<210>  45
<211>  49
<212>  DNA
<213>  Artificial Sequence

<220>
<223>  Synthetic oligonucleotide

<400>  45
gcacttacct tcgcattttc tggtatattt gctgtatctg cggagatgc                    49


<210>  46
<211>  54
<212>  DNA
<213>  Artificial Sequence

<220>
<223>  Synthetic oligonucleotide

<400>  46
cctgtgttgg ttctcaggaa ctgcgaatat gccacactga atacaggaat ctcc             54


<210>  47
<211>  20
<212>  DNA
<213>  Artificial Sequence

<220>
<223>  Synthetic oligonucleotide

<400>  47
catcagccag ttgaatcgct                                                   20


<210>  48
<211>  19
<212>  DNA
<213>  Artificial Sequence

<220>
<223>  Synthetic oligonucleotide

<400>  48
catctccgca gatacagca                                                    19


<210>  49
<211>  17
<212>  DNA
<213>  Artificial Sequence

<220>
<223>  Synthetic oligonucleotide

<400>  49
gacggccctc ttgactg                                                      17

<210> 50
<211> 1797
<212> DNA
<213> Aspergillus niger

<400> 50
atggcatctc cgcagataca gcatgagggc cttcgtgcca ttttcacggg gattgagagg 60

gtgtccgagg gggtcacggt gaatgagttt aggggtatca agtacgcgtc tgttcccgcg 120

cggtttgagc gggcgcagcc ggtggatggc tttggggatg ctgtggttga tgcttcttca 180

tacgggtact gtttgcaatc gtgtaacgct ggcagcatgg ataagtgctg atttggattt 240

gatcctgctt aggccgaggt gtccgcaggt cgatgtcgac gttcgccatt tgctgcgcat 300

tccagaggac tttccaatcg aaagggagcc tgaggatgag tttgaatgtc tgaatttgga 360

catcacctgt cctccggcgt cttcggctaa gggacctctt cctgtgctct tgtggattta 420

tggtatgtca tgggcatacc ctcaatttac actgtcaatg gaccaatggc taatcatgtc 480

atacaggagg atcgcaggtg gtgacctttt gctcagctgc atcgaagatc tgcggtaagt 540

taggccaagg gaatatcgtc cttggagact gacaacagta tcaatagatc ccgtcaagat 600

cgtcgcggac tctatcaagt ccggccagcc tatcatcttc gtctcaatca actatcgact 660

caacatcttc agctttggcg acggcaagga gaagaacctt gctatcaagg atcagaaact 720

gggtattgag tgggtgcgga acaacatcgc tgcctttgga ggcgatccgg tatgtactaa 780

ctcgcgcggt tatttactaa atctaaccaa ccttcttaca gaacaacatc acgttggccg 840

gagaaagtgc cggggcagtt tatacgcatg cccatctcgt cactggtaca ccagtcaaga 900

gggccgtcct ggcatctgga tccctctatc tctcttctcc cttgccaata gagaagggcc 960

agggactaat caatgcgctg gaagccaagg tgcaggaact gggcgagccg tcgcttcggt 1020

tagcgtcagt gccggctctg ttgcaggcat tacgagagtg caatgtgaac actatgtgga 1080

tacaggagga tgaggacttt caaggatggg aaaccaagtc cgagctggtg gacgaactca 1140

tgattggtga tactgaatat gaggtgggtg accagtccac acaatttatc aagataacta 1200

ctaataataa tcatacagtc ggttatctgg aggaacggta tcgagacatt cgatggagaa 1260

gcgattactg cagccttcga gcaaaatgag aaatggggtc cgaagctacg caagatgtac 1320

caggttgtcg cggatcgacc cactgcttgc aagttgggtg cactggacct tgtgaatgat 1380

gtccgttata cgctgccagt agaagttgtt gccgagaaac tacaggcagc tgggaaacga 1440

gtctacaagt atgtggttga ccaggccaac ccgtggcagg catccagccg agcccatcat 1500

gctgttgacc tgctattctt gtttgacggc gccgacctgt ccttcaaccc tgctgcacaa 1560

gcggttggtc aggagatgcg gagccgttgg gtgcagttcg tcaacggtgg tagtccgtgg 1620

tctaccgagc agagattcgc gtacggacct ctggggagct gcggagagat ttctgaagca 1680

cagtttgcat ctagacggcg ggttgagcat gttaaagttc ttagggaggc tggaatgggt 1740

gtctatatgc ctattgtatt tgccttgacg gcaggcaaga ttagcttgtt gaattag 1797